(19) Europäisches Patentamt
European Patent Office
Office européen des brevets

(11) Publication number: **0 503 838 A2**

# (12) EUROPEAN PATENT APPLICATION

(21) Application number: **92301884.0**

(22) Date of filing: **05.03.92**

(51) Int. Cl.[5]: **C07D 471/04,** C07D 487/04, C07D 473/00, C07D 473/02, C07D 473/26, C07D 473/40, C07F 9/547, A61K 31/435, A61K 31/495, A61K 31/53, A61K 31/675, // (C07D471/04, 235:00, 221:00)

(30) Priority: **08.03.91 US 666534**

(43) Date of publication of application: **16.09.92 Bulletin 92/38**

(84) Designated Contracting States: **AT BE CH DE DK ES FR GB GR IT LI LU NL PT SE**

(71) Applicant: **MERCK & CO. INC.**
**126, East Lincoln Avenue P.O. Box 2000**
**Rahway New Jersey 07065-0900 (US)**

(72) Inventor: **Chakravarty, Prasun K.**
**16 Churchill Road**
**Edison, NJ 08820 (US)**

Inventor: **Patchett, Arthur A.**
**1090 Minisink Way**
**Westfield, NJ 07090 (US)**
Inventor: **Mantlo, Nathan B.**
**402 Mountain Avenue**
**Westfield, NJ 07090 (US)**
Inventor: **Rivero, Ralph A.**
**49 Columbia Drive**
**Tinton Falls, NJ 07724 (US)**
Inventor: **Greenlee, William J.**
**115 Herrick Avenue**
**Teaneck, NJ 07666 (US)**
Inventor: **Kim, Dooseop**
**24 Country Club Lane**
**Scotch Plains, NJ 07076 (US)**

(74) Representative: **Thompson, John Dr. et al**
**Merck & Co., Inc. European Patent Department Terlings Park Eastwick Road**
**Harlow, Essex CM20 2QR (GB)**

## (54) Heterocyclic compounds bearing acidic functional groups as angiotensin II antagonists.

(57) Heterocyclic compounds of structural formula :

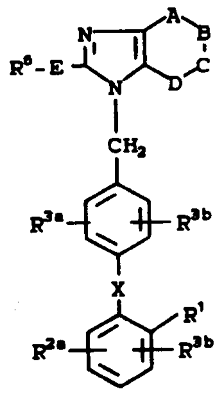

(I)

wherein A, B, C, and D are independently carbon atoms or nitrogen atoms are angiotensin II antagonists useful in the treatment of hypertension and congestive heart failure.



Jouve, 18, rue Saint-Denis, 75001 PARIS

SUMMARY OF THE INVENTION

This invention relates to novel compounds of structural formula I which are angiotensin II antagonists useful in the treatment of hypertension, congestive heart failure, and elevated intraocular pressure.

It also relates to processes for preparing the novel compounds; pharmaceutical formulations comprising one or more of the compounds as active ingredient; and, a method of treatment of hypertension, congestive heart failure, and elevated intraocular pressure.

BACKGROUND OF THE INVENTION

Renin-angiotensin system (RAS) plays a central role in the regulation of normal blood pressure and seems to be critically involved in hypertension development and maintenance as well as congestive heart failure. Angiotensin II (AII), an octapeptide hormone is produced mainly in the blood during the cleavage of angiotensin I by angiotensin converting enzyme (ACE) localized on the endothelium of blood vessels of lung, kidney, and many other organs, and is the end product of the RAS.AII is a powerful arterial vasoconstricter that exerts its action by interacting with specific receptors present on cell membranes. One of the possible modes of controlling the RAS is angiotensin II receptor antagonism. Several peptide analogs of A II are known to inhibit the effect of this hormone by competitively blocking the receptors, but their experimental and clinical applications have been limited by the partial agonist activity and lack of oral absorption [M. Antonaccio. Clin. Exp. Hypertens. A4, 27-46 (1982); D. H. P. Streeten and G. H. Anderson, Jr. - Handbook of Hypertension, Clinical Pharmacology of Antihypertensive Drugs, ed. A. E. Doyle, Vol. 5, pp. 246-271, Elsevier Science Publisher, Amsterdam, The Netherlands, 1984].

Recently, several non-peptide compounds have been described as A II antagonists. Illustrative of such compounds are those disclosed in U.S. Patents 4,207,324; 4,340,598; 4,576,958; 4,582,847; and 4,880,804; in European Patent Applications 028,834; 245,637; 253,310; and 291,969; and in articles by A.T. Chiu, et al. [Eur. J. Pharm. Exp. Therap, 157, 13-21 (1988)] and by P.C. Wong, et al. [J. Pharm. Exp. Therap, 247, 1-7(1988)]. All of the U.S. Patents, European Patent Applications 028,834, 253,310, 324,377, 403,158 and 403,159 and the two articles disclose substituted imidazole compounds which are generally bonded through a lower alkyl bridge to a substituted phenyl. European Patent Application 245,637 discloses derivatives of 4,5,6,7-tetrahydro-2H-imidazo[4,5-c]-pyridine-6-carboxylic acid and analogs thereof as antihypertensive agents. U.S. Patent No. 4,880,804 and European Patent Applications 392,317, 399,732 and 400,835 disclose derivatives of benzimidazole attached via a bridge to a biphenyl moiety as antihypertensive agents. European Patent Applications 399,732 and 400,974 disclose derivatives of imidazopyridines attached via a bridge to a biphenyl moiety as antihypertensive agents.

DETAILED DESCRIPTION OF THE INVENTION

This invention relates to substituted imidazo-fused 6-membered ring heterocycles of the formula I shown below which are angiotensin II antagonists and are useful in the treatment of hypertension, congestive heart failure, and elevated intraocular pressure.

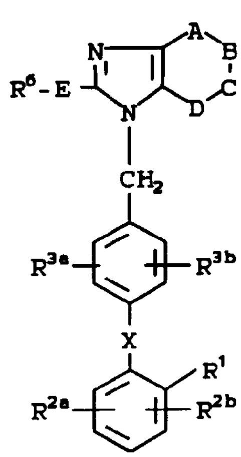

(I)

wherein:

$R^1$ is

(a) $-SO_2N(R^{24})-OR^{24}$,
(b) $-SO_2NHSO_2R^{23}$,
(c)

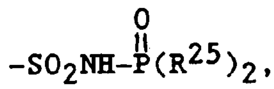

(d)

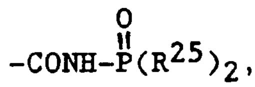

(e) $-SO_2NHCN$,
(f) $-SO_2NHCO_2R^{23}$,
(g)

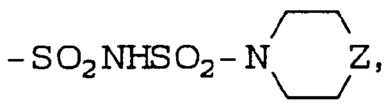

(h) $-NHSO_2NHSO_2R^{23}$,
(i)

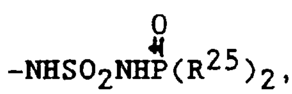

(j) 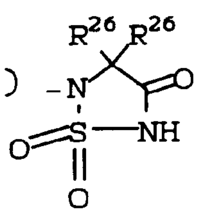 ,

(k) 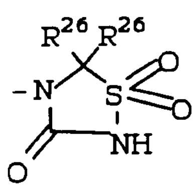 ,

(l) 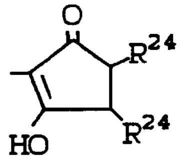 ,

(m) 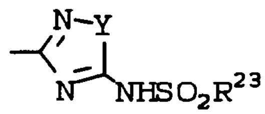 ,

(n) 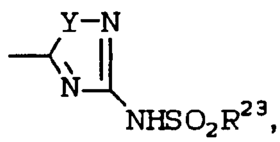

(o) 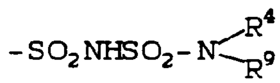 ,

(p) 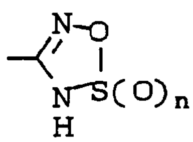 ,

(q) 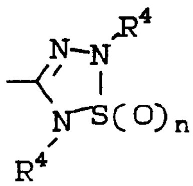 ,

(r) 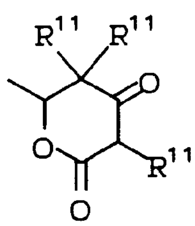 ,

(s) 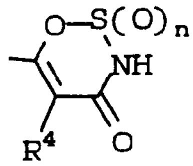 ,

(t) 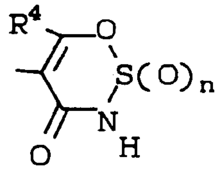 ,

(u) 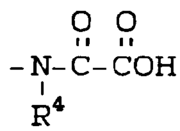 , or

(v) $-NHSO_2R^{23}$;

wherein Y is O or S;

$R^{2a}$ and $R^{2b}$ are independently H, Cl, Br, I, F, $-NO_2$, $-NH_2$, $C_1$-$C_4$-alkylamino, di($C_1$-$C_4$ alkyl)-amino, $-SO_2NHR^9$, $CF_3$, $C_1$-$C_6$-alkyl, or $C_1$-$C_6$-alkoxy, $CH_2$-$C_1$-$C_6$-alkoxy, $CH_2$-S-$C_1$-$C_6$-alkyl, $CH_2NR^9R^9$, $CH_2$-aryl, or aryl;

$R^{3a}$ is

(a) H,
(b) Cl, Br, I, or F,
(c) $C_1$-$C_6$-alkyl,
(d) $C_1$-$C_6$-alkoxy, or
(e) $C_1$-$C_6$-alkoxyalkyl;

$R^{3b}$ is

(a) H,
(b) Cl, Br, I, or F,
(c) $NO_2$,
(d) $C_1$-$C_6$-alkyl,
(e) $C_1$-$C_6$-acyloxy,
(f) $C_1$-$C_6$-cycloalkyl
(g) $C_1$-$C_6$-alkoxy,
(h) $-NHSO_2R^4$,
(i) hydroxy $C_1$-$C_4$-alkyl,
(j) aryl $C_1$-$C_4$-alkyl,
(k) $C_1$-$C_4$-alkylthio,
(l) $C_1$-$C_4$-alkyl sulfinyl,
(m) $C_1$-$C_4$-alkyl sulfonyl,
(n) $NH_2$,
(o) $C_1$-$C_4$-alkylamino,
(p) $C_1$-$C_4$-dialkylamino,
(q) fluoro $C_1$-$C_4$-alkyl,
(r) $-SO_2$-$NHR^9$,
(s) aryl, or
(t) furyl;

wherein aryl is phenyl or naphthyl or substituted phenyl or naphthyl with one or two substituents selected from the group consisting of Cl, Br, I, F, $C_1$-$C_4$-alkyl, $C_1$-$C_4$-alkoxy, $NO_2$, $CF_3$, $C_1$-$C_4$-alkylthio, OH, $NH_2$, NH($C_1$-$C_4$-alkyl), N($C_1$-$C_4$-alkyl)$_2$, $CO_2H$, and $CO_2$-$C_1$-$C_4$-alkyl;

$R^4$ is H, $C_1$-$C_6$ alkyl, aryl, $-CH_2$-aryl, -CO-$C_1$-$C_6$-alkyl, -CO-$C_3$-$C_6$-cycloalkyl, -CO-aryl, $-CO_2$-$C_1$-$C_6$-alkyl,

$-CO_2-C_3-C_6$-cycloalkyl, $-CO_2$-aryl, $-CONH-C_1-C_6$-alkyl, $-SO_2$-aryl, or $-SO_2-C_1-C_6$-alkyl;

$R^{4a}$ is $C_1-C_6$-alkyl, aryl or $-CH_2$-aryl;

$R^5$ is

H, 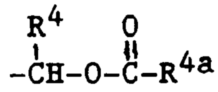;

E is a single bond $-NR^{13}(CH_2)_s-$,$-S(O)_n-$, $(CH_2)_s-$ where n is 0 to 2 and s is 0 to 5, -CH(OH)-, -O-, or -CO-;

$R^6$ is

(a) aryl or substituted aryl with 1 or 2 substituents selected from the group consisting of Cl, Br, I, F $-O-C_1-C_4$-alkyl, $C_1-C_4$-alkyl, $-NO_2$, $-CF_3$, $-SO_2NR^9R^{10}$, $-S-C_1-C_4$-alkyl, -OH, $-NH_2$, $C_3-C_7$-cycloalkyl, $C_3-C_{10}$-alkenyl;

(b) $C_1-C_9$-alkyl, $C_2-C_6$-alkenyl or $C_2-C_6$-alkynyl, or substituted $C_1-C_9$ alkyl, $C_2-C_6$ alkenyl or $C_2-C_6$ alkynyl with a substituent selected from the group consisting of aryl as defined above, $C_3-C_7$-cycloalkyl, Cl, Br, I, F, -OH, $-NH_2$, $-NH(C_1-C_4$-alkyl), $-CF_2CF_3$, $-N(C_1-C_4$-alkyl$)_2$, $-NH-SO_2R^4$, $-COOR^4$, $-CF_3$, $-CF_2CH_3$, $-SO_2NHR^9$; or

(c) an unsubstituted, monosubstituted or disubstituted aromatic 5 or 6 membered cyclic ring which contains one or two members selected from the group consisting of N, O, S, and wherein the substituents are members selected from the group consisting of -OH, -SH, $C_1-C_4$-alkyl, $C_1-C_4$-alkyloxy,$-CF_3$, Cl, Br, I, F, or $NO_2$, or

(d) perfluoro-$C_1-C_4$-alkyl, or

(e) $C_3-C_7$-cycloalkyl or mono- or disubstituted $C_3-C_7$ cycloalkyl with a $C_1-C_4$-alkyl or $-CF_3$ substituent;

$R^9$ is H, $C_1-C_5$-alkyl, aryl or $-CH_2$-aryl;

$R^{10}$ is H, $C_1-C_4$-alkyl;

$R^{11}$ is H, $C_1-C_6$-alkyl, $C_2-C_4$-alkenyl, $C_1-C_4$-alkoxy-$C_1-C_4$-alkyl, or

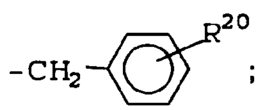;

$R^{12}$ is -CN, $-NO_2$ or $-CO_2R^4$;

$R^{13}$ is H, $-CO(C_1-C_4$-alkyl), $C_1-C_6$-alkyl, allyl, $C_3-C_6$-cycloalkyl, phenyl or benzyl;

$R^{14}$ is H, $C_1-C_8$-alkyl, $C_1-C_8$-perfluoroalkyl, $C_3-C_6$-cycloalkyl, phenyl or benzyl;

$R^{15}$ is H, $C_1-C_6$-alkyl;

$R^{16}$ is H, $C_1-C_6$-alkyl, $C_3-C_6$-cycloalkyl, phenyl or benzyl;

$R^{17}$ is $-NR^9R^{10}$, $-OR^{10}$, $-NHCONH_2$, $-NHCSNH_2$,

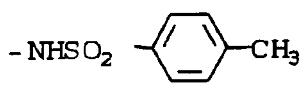 or 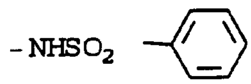;

$R^{18}$ and $R^{19}$ are independently $C_1-C_4$-alkyl or taken together are $-(CH_2)_q-$ where q is 2 or 3;

$R^{20}$ is H, $-NO_2$, $-NH_2$, -OH or $-OCH_3$;

$R^{23}$ is

(a) aryl,

(b) heteroaryl, wherein heteroaryl is an unsubstituted, monosubstituted or disubstituted five- or six-membered aromatic ring which contains 1 to 3 heteroatoms selected from the group consisting of O, N or S and wherein the substituents are members selected from the group consisting of -OH, -SH, $-C_1-C_4$-alkyl, $-C_1-C_4$-alkoxy, Cl, Br, F, I, $-NO_2$, $-CO_2H$, $-CO_2-C_1-C_4$-alkyl, $-NH_2$, $-NH(C_1-C_4$-alkyl) and $-N(C_1-C_4$-alkyl$)_2$;

(c) $C_3-C_4$-cycloalkyl,

(d) $C_1-C_4$-alkyl or substituted $C_1-C_4$ alkyl with a substituent that is a member selected from the group consisting of aryl, heteroaryl, -OH, -SH, $-C_1-C_4$-alkyl, $-C_3-C_7$- cycloalkyl, $-O(C_1-C_4$-alkyl), $-S(O)_n(C_1-C_4$-alkyl), $-CF_3$, Cl, Br, F, I, $-NO_2$, $-CO_2H$, $-CO_2-C_1-C_4$- alkyl, $-NH_2$, $-NH(C_1-C_4$-alkyl), $-NHCOR^{4a}$, $-N(C_1-C_4$-alkyl$)_2$, $-PO(OH)(C_1-C_4$-alkyl), -PO(OH)- (aryl), or $-PO(OH)(O-C_1-C_4$-alkyl); where n is 0 to 2, or

(e) perfluoro-$C_1-C_4$-alkyl;

$R^{24}$ is

(a) H,
(b) aryl as defined above, or
(c) $C_1$-$C_6$-alkyl optionally substituted with aryl, F, Cl, Br, -OH, $-NH_2$, -NH($C_1$-$C_4$-alkyl), -N($C_1$-$C_4$-alkyl)$_2$, $CF_3$, O-$C_1$-$C_4$-alkyl, or $O(CH_2)_{n+1}$-O-$C_1$-$C_4$-alkyl, or
(d) $C_3$-$C_7$-cycloalkyl;

$R^{25}$ is
(a) aryl and substituted aryl as defined above,
(b) $C_1$-$C_6$-alkyl optionally substituted with aryl, F, Cl, Br, -OH, $-NH_2$, -NH($C_1$-$C_4$-alkyl), -N($C_1$-$C_4$-alkyl)$_2$, $CF_3$, $-COOR^4$, or CN,
(c) $-CH(R^4)$-O-CO-$R^{4a}$, or
(d) -OH, -O-$C_1$-$C_6$-alkyl wherein alkyl is as defined in (b);

$R^{26}$ is
(a) H,
(b) $C_1$-$C_6$-alkyl optionally substituted with aryl, F, Cl, Br, -OH, $-NH_2$, -NH($C_1$-$C_4$-alkyl), -N($C_1$-$C_4$-alkyl)$_2$, $CF_3$, $-COOR^4$, or CN,
(c) F, Cl, Br, or
(d) -O-$C_1$-$C_4$-alkyl wherein alkyl is defined as in (b);

X is
(a) a carbon-carbon single bond,
(b) -CO-,
(c) -O-,
(d) -S-,
(e)

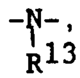

(f)

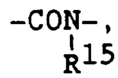

(g)

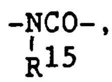

(h) $-OCH_2-$,
(i) $-CH_2O-$
(j) $-SCH_2-$,
(k) $-CH_2S-$,
(l) $-NHC(R^9)(R^{10})$,
(m) $-NR^9SO_2-$,
(n) $-SO_2NR^9-$,
(o) $-C(R^9)(R^{10})NH-$,
(p) -CH=CH-,
(q) -CF=CF-,
(r) -CH=CF-,
(s) -CF=CH-,
(t) $-CH_2CH_2-$,
(u) $-CF_2CF_2-$,

(v) 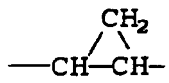 and 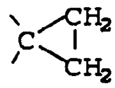 ,

(w) 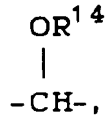

(x) 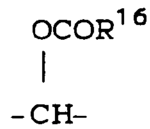

(y) 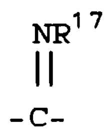 , or

(z) 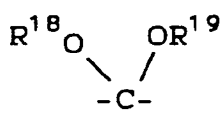 ;

Z is $CH_2$, O, $NR^{13}$ or S;

-A-B-C-D- represents the constituent atoms of a 6-member saturated or unsaturated heterocyclic ring with the imidazole to which they are attached containing 1 to 3 nitrogen atoms and includes the following:

1) 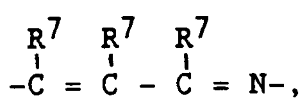

2) 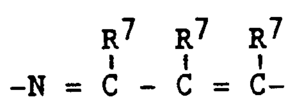

3) 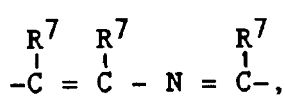

4) 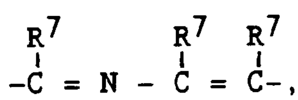

5) 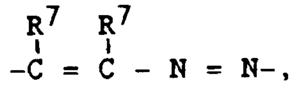

6) 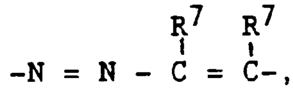

7) 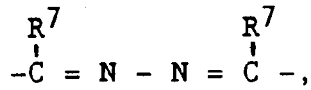

8) 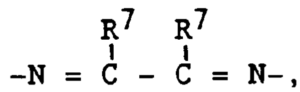

9) 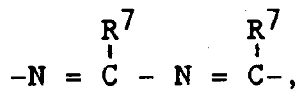

10) 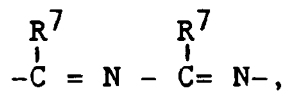

11) 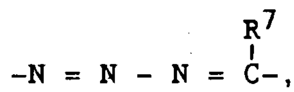

12) 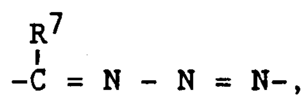

13) 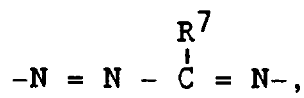

14) 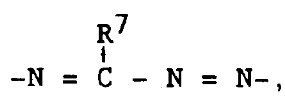

15) 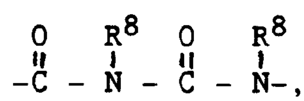

16) 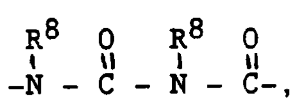

17) 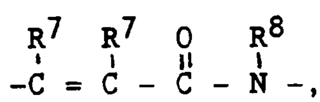

18) 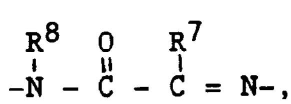

19) 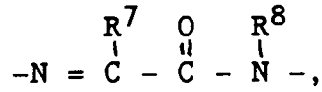

20) 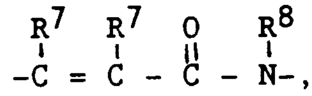

21) 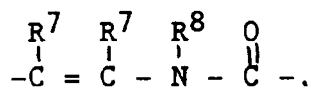

22) 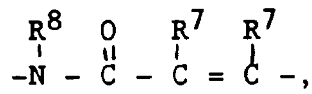

23) 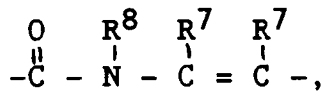

24) 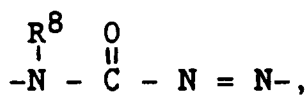

25) 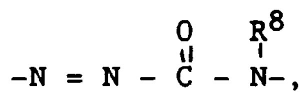

26) 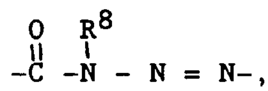

27) 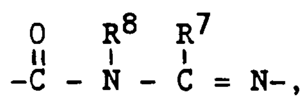

28) 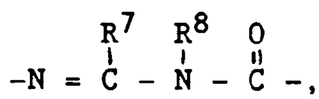

29) 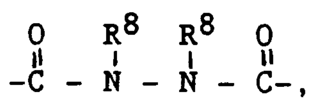

30) 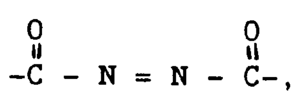

31) 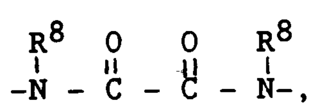

32) 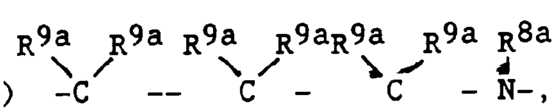

33) 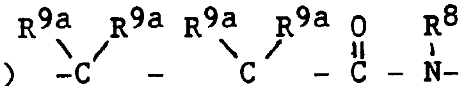,

34) 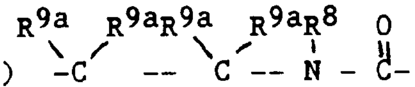,

35) 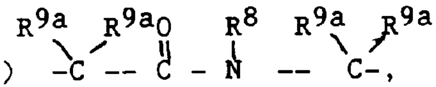,

36) 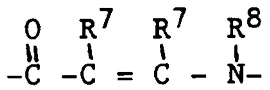,

37) 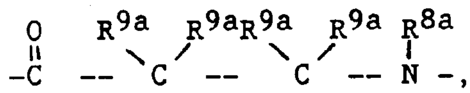, and

38) 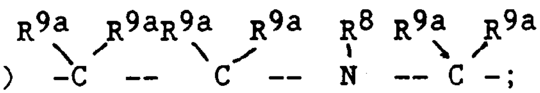;

$R^7$ groups can be the same or different and represent:

a) hydrogen,

b) $C_1$-$C_6$ alkyl, or $C_2$-$C_6$ alkenyl, or $C_2$-$C_6$ alkynyl each of which is unsubstituted or substituted with:

i) -OH

ii) $C_1$-$C_4$-alkoxy,

iii) $-CO_2R^4$ or $-CO_2R^5$,

iv) $-OCOR^4$,

v)

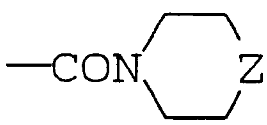,

vi) $-CON(R^4)_2$

vii)

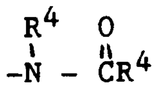

viii) $-N(R^4)_2$,

ix) aryl,

x) heteroaryl as defined in (o) below,

xi) $-S(O)_nR^{23}$,

xii) tetrazol-5-yl,

xiii) $-CONHSO_2R^{23}$,

xiv) $-SO_2NHR^{23}$,

xv) $-SO_2NHCOR^{23}$,

xvi)

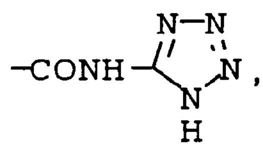

xvii)

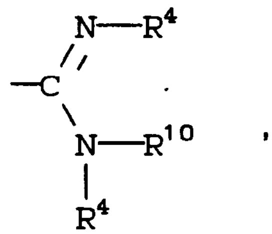

xviii)

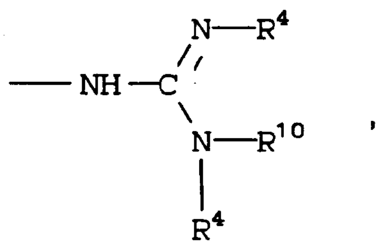

xix) $-PO(OR^4)_2$, or
xx) $-PO(OR^4)R^9$,
c) fluoro, chloro, bromo or iodo,
d) perfluoro-$C_1$-$C_4$-alkyl,
e) -OH,
f) $-NH_2$,
g)

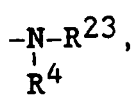

h)

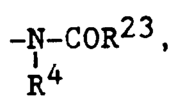

i) $-OR^{23}$,
j) $-CO_2R^4$ or $-CO_2R^5$,
k) $-CON(R^4)_2$,
1) -NH-$C_3$-$C_7$-cycloalkyl,
m) $C_3$-$C_7$-cycloalkyl,
n) aryl,
o) heteroaryl which is a five- or six- membered saturated or unsaturated ring containing up to three heteroatoms selected from the group consisting of O, N or S wherein S may in the form of sulfoxide or sulfone and which may be substituted with one or two substituents which are members selected from the group consisting of Cl, Br, F, I, $C_1$-$C_4$-alkyl, $C_1$-$C_4$-alkoxy, $C_1$-$C_4$-$S(O)_n$-, $CF_3$, $NO_2$, OH, $CO_2H$, $CO_2$-$C_1$-$C_4$-alkyl, $NH_2$, $NH(C_1$-$C_4$-alkyl), or $-N(R^4)_2$;
p) -CN,

q)

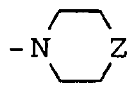,

r) $-SO_2N(R^4)_2$,
s) tetrazol-5-yl,
t) $-CONHSO_2R^{23}$,
u) $-PO(OR^4)_2$,
v) $-SO_2NHR^{23}$,
w) $-SO_2NHCOR^{23}$,
x) $-S(O)_n-R^{23}$,
y)

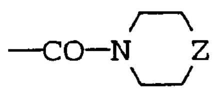,

z) $-PO(OR^4)R^9$ or $-PO(OR^5)R^9$,
aa) $-SO_2NHCON(R^{23})_2$,
bb) $-NHSO_2NHR^{23}$,
cc) $-NHSO_2NHCOR^{23}$,
dd) $-NHCONHSO_2R^{23}$,
ee) $-N(R^4)CO_2R^{23}$,
ff)

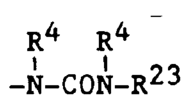,

gg) -CO-aryl,
hh)

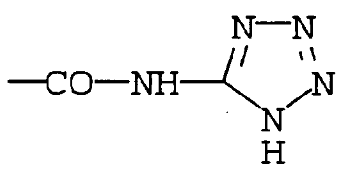,

ii) $-CO-C_1-C_4$-alkyl,
jj) $-SO_2NH-CN$,
kk) $-NHSO_2R^{23}$,

11)

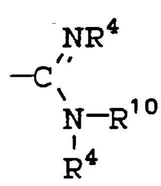, or

mm)

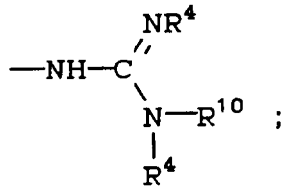

$R^8$ groups can be the same or different and represent:

a) hydrogen,

b) $C_1$-$C_6$-alkyl- or $C_2$-$C_6$ alkenyl either unsubstituted or substituted with hydroxy, $C_1$-$C_4$-alkoxy, -$N(R^4)_2$, -$CO_2R^4$, or $C_3$-$C_5$-cycloalkyl, or

c) $C_3$-$C_5$-cycloalkyl;

$R^{8a}$ is $R^8$ or $C_1$-$C_4$-acyl;

$R^{9a}$ groups can be the same or different and represent:

a) hydrogen, or

b) $C_1$-$C_6$-alkyl either unsubstituted or substituted with

i) hydroxy,

ii) -$CO_2R^4$,

iii) -$CONHR^4$, or

iv) -$CON(R^4)_2$; and,

the pharmaceutically acceptable salts thereof.

The terms "alkyl," "alkenyl," "alkynyl," and the like include both the straight chain and branched chain species of these generic terms wherein the number of carbon atoms in the species permit. Unless otherwise noted, the specific names for these generic terms shall mean the straight chain species. For example, the term "butyl" shall mean the normal butyl substituent, n-butyl.

One embodiment of the novel compounds of this invention is the class compounds of Formula I wherein:

$R^1$ is:

(a) -$SO_2N(R^{24})$-$OR^{24}$,

(b) -$SO_2NHSO_2R^{23}$,

(c)

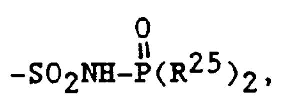

(d) -$SO_2NHCN$,

(e) -$SO_2NHCO_2R^{23}$,

(f) 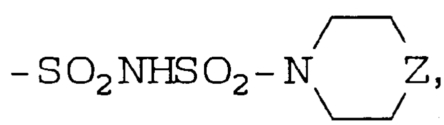

(g) -$SO_2NHSO_2$-$N(R^4)(R^9)$,

(h) -$NHSO_2NHSO_2R^{23}$, or

(i)

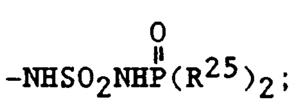

(j) 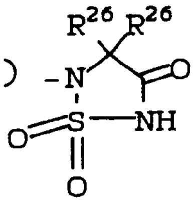 ,

(k) 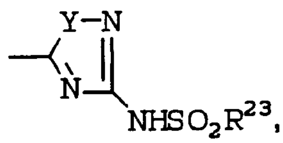 ,

(l) 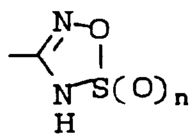 ,

(m) 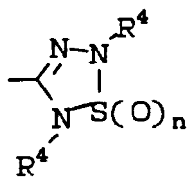 ,

(n) 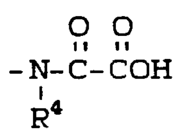 , or

(o) $-NHSO_2R^{23}$;

X is a single bond;

$R^{2a}$ and $R^{2b}$ are independently:

a) $C_1$-$C_6$-alkyl,
b) halogen,
c) hydrogen,
d) $CH_2$-$C_1$-$C_6$-alkoxy, or
e) $C_1$-$C_6$-alkoxy;

$R^{3a}$ and $R^{3b}$ are independently:

a) $C_1$-$C_6$-alkyl,
b) halogen,
c) $C_1$-$C_6$-alkoxy, or
d) hydrogen;

$R^4$ is H, or $C_1$-$C_4$-alkyl;

E is a single bond or -S-;

$R^6$ is $C_1$-$C_6$-alkyl, $C_3$-$C_7$-cycloalkyl, $C_2$-$C_6$-alkenyl or $C_2$-$C_6$- alkynyl each of which is either unsubstituted or substituted with $C_1$-$C_4$- alkylthio, $C_1$-$C_4$-alkoxy, $CF_3$, $CF_2CF_3$ or $-CF_2CH_3$;

and A-B-C-D- represents:

1) 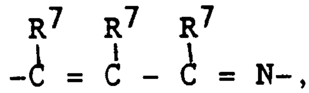

2) 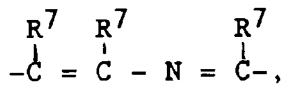

3) 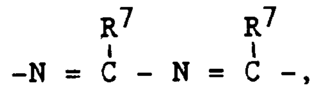

4) 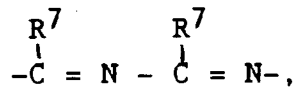

5) 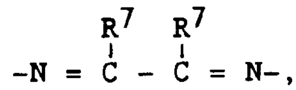

6) 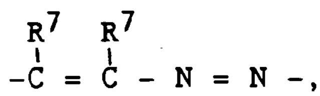

7) 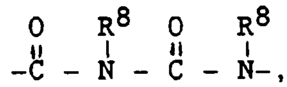

8) 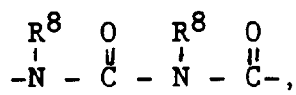

9) 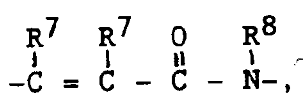

10) 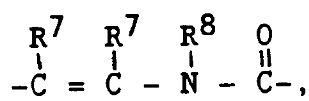

11) 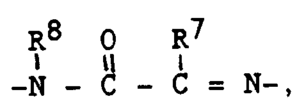

12) 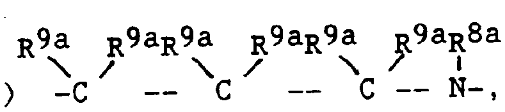

13) 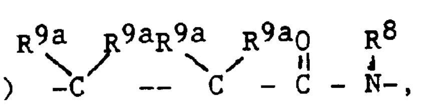 or

14) 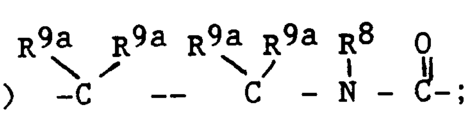

$R^7$ groups are the same or different and represent:
a) hydrogen,
b) $C_1$-$C_4$-alkyl, either unsubstituted or substituted with:
i) -OH,
ii) $-CO_2R^4$ or $-CO_2R^5$,
iii) $-NH_2$,
iv) ($C_1$-$C_4$ alkyl)amino,
v) di($C_1$-$C_4$-alkyl)amino,
c) -F, Cl-, -Br, or -I,
d) $-CF_3$,
e) -OH,
f) $-N(R^4)_2$,
g) -$C_1$-$C_4$-alkoxy,
h) $-CO_2R^4$ or $-CO_2R^5$,
i) $-CONH_2$,
j) -$C_3$-$C_7$-cycloalkyl,
k) aryl,
l) heteroaryl,
m) $-CF_3$,
n)tetrazol-5-yl,
o) $-CONHSO_2R^{23}$ or
p) $-NHSO_2R^{23}$;
$R^8$ groups are the same or different and represent,
a) hydrogen, or
b) $C_1$-$C_4$-alkyl either unsubstituted or substituted with -OH or $-CO_2R^4$;
$R^{8a}$ represents
a) hydrogen, or
b) $C_1$-$C_4$ alkyl, or
c) ($C_1$-$C_4$-alkyl)CO-; and
$R^{9a}$ groups are the same or different and represent:
a) hydrogen, or
b) $C_1$-$C_4$-alkyl.
A class of this embodiment include those compounds of Formula I wherein:
$R^1$ is
(a) $-SO_2N(R^{24})-OR^{24}$,
(b) $-SO_2NHSO_2R^{23}$,
(c)

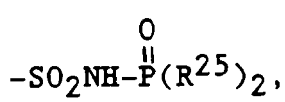

(d) $-SO_2NHCN$,
(e) $-SO_2NHCO_2R^{23}$,
(f)

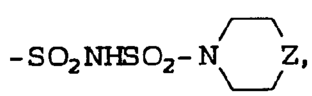

(g)

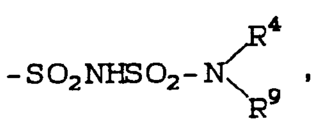

(h) $-NHSO_2NHSO_2R^{23}$, or

(i)

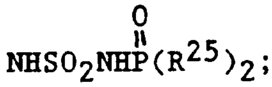

(j)

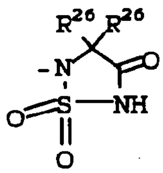

,

(k)

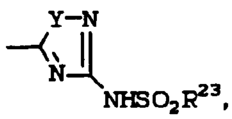

(l)

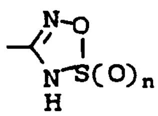

,

(m)

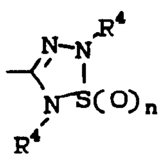

,

(n)

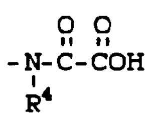

, or

(o) $-NHSO_2R^{23}$;

E is a single bond;

A-B-C-D represents:

1) 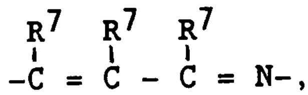

2) 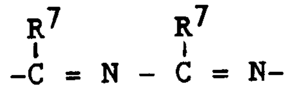 or

3) 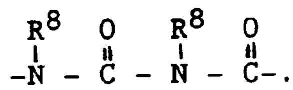

A particular subclass of this embodiment includes the compounds of Formula (II).

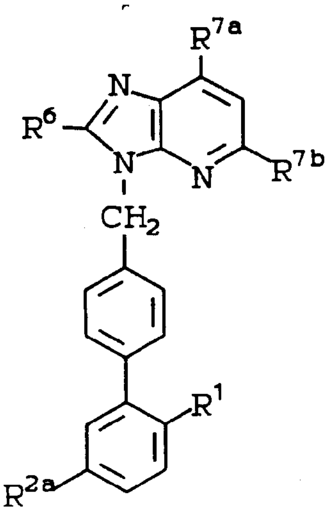

(II)

wherein:

$R^1$ is:

(a) $-SO_2N(R^{24})-OR^{24}$,

(b) $-SO_2NHSO_2R^{23}$,

(c)

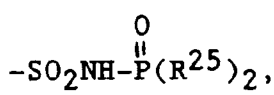

(d) $-SO_2NHCN$,

(e) $-SO_2NHCO_2R^{23}$,

(f)

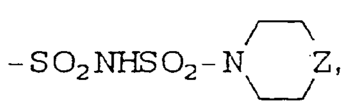

(g) 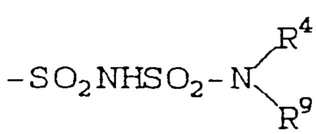 ,

(h) $-NHSO_2NHSO_2R^{23}$, or

(i)

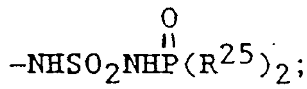

(j) 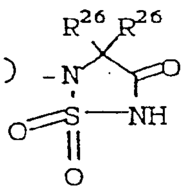 ,

(k)

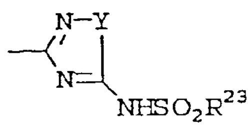 ,

(l)

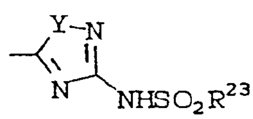 ,

(m) 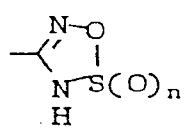 ,

(n) 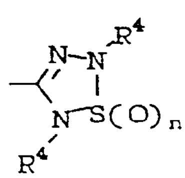 ,

(o) $-NHSO_2R^{23}$;

$R^6$ is $C_1$-$C_6$-alkyl or $C_2$-$C_6$ alkenyl or $C_3$-$C_7$cycloalkyl; and

$R^{7a}$ and $R^{7b}$ independently are:

(a) hydrogen,

(b) $C_1$-$C_6$ alkyl or $C_2$-$C_6$ alkenyl,

(c) $-CO_2R^4$ or $-CO_2R^5$, or

(d) $-CON(R^4)_2$.

Illustrating this particular subclass are the compounds of Formula (II) wherein

$R^1$ is:

(a) $-SO_2N(R^{24})-OR^{24}$,

(b) $-SO_2NHSO_2R^{23}$,

(c)

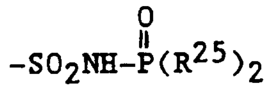,

(d) $-SO_2NHCN$,
(e) $-SO_2NHCO_2R^{23}$,

(f) 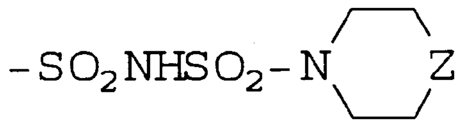,

(g) 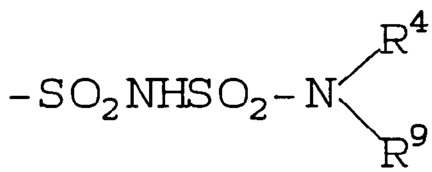;

(h) $-NHSO_2NHSO_2R^{23}$, or
(i)

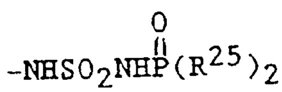;

(j) 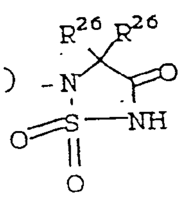,

(k) 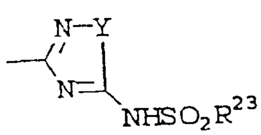,

(l) 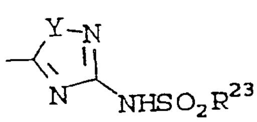,

(m) 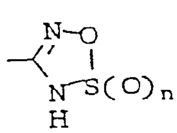,

(n) 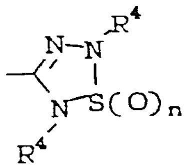 ,

(o) $-NHSO_2R^{23}$;

$R^{2a}$ is:

(a) $C_1$-$C_6$-alkyl, or

(b) $CH_2$-$C_1$-$C_6$-alkoxy;

$R^6$ is $C_1$-$C_6$alkyl; and

$R^{7a}$ and $R^{7b}$ independently are hydrogen, a branched or straight $C_1$-$C_6$ alkyl or $CO_2R^4$.

Exemplifying this subclass are the following compounds of the Formula II shown in Table A:

## TABLE A

| Compound No. | $R^1$ | $R^6$ | $R^{7a}$ | $R^{7b}$ |
|---|---|---|---|---|
| A1 | $-SO_2NHOH$ | Et | Me | Me |
| A2 | $-SO_2NHSO_2Ph$ | Et | Me | Me |
| A3 | $-SO_2NHSO_2Me$ | Et | Me | Me |
| A4 | 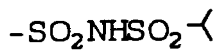 | Pr | $CO_2H$ | Me |
| A5 | 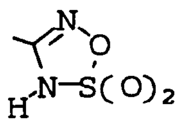 | Et | Me | Me |
| A6 | 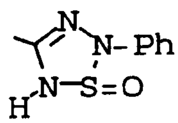 | Et | Me | Me |
| A7 | 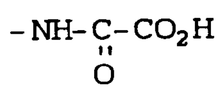 | Et | $CO_2H$ | Me |
| A8 | 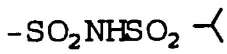 | Et | Me | Me |
| A9 | 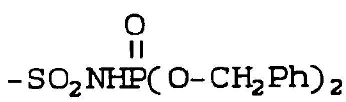 | Et | Me | Me |
| A10 | 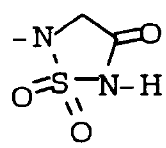 | Et | Me | Me |
| A11 | 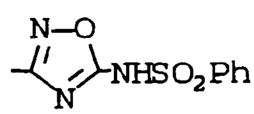 | Et | Me | Me |
| A12 | 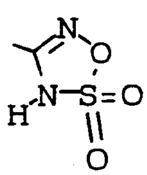 | Et | Me | Me |

| Compound No. | $R^1$ | $R^6$ | $R^{7a}$ | $R^{7b}$ |
|---|---|---|---|---|
| A13 | $-SO_2NHCOOC_2H_5$ | Et | Me | Me |
| A14 | $-SO_2NHCOOCH_2Ph$ | Et | Me | Me |
| A15 | $-SO_2NHCOOBu$ | Et | Me | Me |
| A16 | $-SO_2NHCOOCH_2c-Pr$ | Et | Me | Me |
| A17 | $-SO_2NHOH$ | Pr | Me | $CONH_2$ |
| A18 | $-SO_2NHCOO-tBu$ | Et | Me | COOH |
| A19 | $-SO_2NHCOO-tBu$ | Et | Me | Me |
| A20 | $-SO_2NHCOO-Et$ | Et | Me | $NMe_2$ |
| A21 | $-SO_2NHCOO-Bu$ | Et | Me | COOH |
| A22 | $-SO_2NHOH$ | Et | Me | $NMe_2$ |

Another subclass of this embodiment includes the compounds of structural formula:

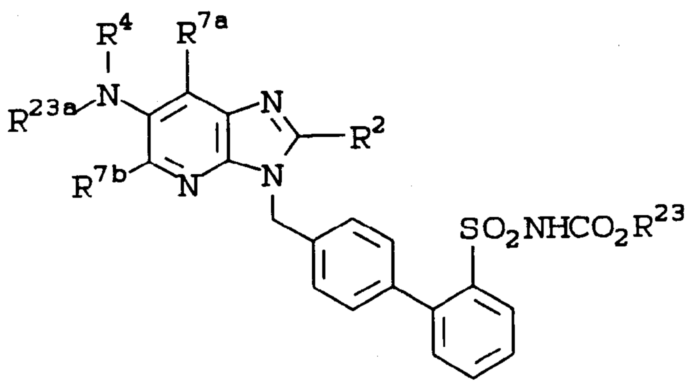

wherein:

| $R^2$ | $R^{23a}$ | $R^4$ | $R^{7a}$ | $R^{7b}$ | $R^{23}$ |
|---|---|---|---|---|---|
| Bu | C(O)Bu | H | H | H | -Bu |
| Bu | C(O)Bu | H | H | H | -Pentyl |
| Bu | C(O)Bu | H | H | H | -Hexyl |

| | | | | | |
|---|---|---|---|---|---|
| Bu | C(O)Bu | H | H | H | $-CH_2CH(CH_3)_2$ |
| Bu | C(O)Bu | H | H | H | $-CH_2CH_2CH(CH_3)_2$ |
| Bu | C(O)Bu | H | H | H | $-CH_2CH(CH_3)CH(CH_3)_2$ |
| Bu | C(O)Bu | H | H | H | $-CH_2(C_5H_9)$ |
| Bu | C(O)Bu | H | H | H | $-CH_2CH_2C_5H_9$ |
| Bu | C(O)Bu | H | H | H | $-CH_2(C_6H_{11})$ |
| Bu | C(O)Bu | H | H | H | $-CH_2CH_2C_6H_{11}$ |
| Bu | C(O)Bu | H | H | H | $-CH_2(C_6H_5)$ |
| Bu | C(O)Bu | H | H | H | $-CH_2CH_2C_6H_5$ |
| Bu | C(O)Bu | H | H | H | $-CH(CH_3)CH_2CH_3$ |
| Bu | C(O)Bu | H | H | H | $-CH(CH_3)CH_2CH_2CH_3$ |
| Bu | C(O)Bu | H | H | H | $-CH(CH_3)_2CH_2CH_3$ |
| Bu | C(O)Bu | H | H | H | $-CH(CH_3)_2CH_2CH_2CH_3$ |
| Bu | C(O)Bu | H | H | H | $-CHC(CH_3)_2$ |
| Bu | C(O)Bu | H | H | H | $-CH_2CH_2OCH_3$ |
| Bu | C(O)Bu | H | H | H | $-CH_2CH_2OCH_2CH_3$ |
| Bu | C(O)Bu | H | H | H | $-CH_2CH_2OCH(CH_3)_2$ |
| Bu | C(O)Bu | H | H | H | -cyclopropane |
| Bu | C(O)Bu | H | H | H | $-C_5H_9$ |
| Bu | C(O)Bu | H | H | H | $-C_6H_{11}$ |
| Bu | C(O)Bu | H | H | H | $-C_6H_5$ |
| Bu | C(O)NHPr | H | H | H | -Bu |
| Bu | C(O)NHPr | H | H | H | -Pentyl |
| Bu | C(O)NHPr | H | H | H | -Hexyl |
| Bu | C(O)NHPr | H | H | H | $-CH_2CH(CH_3)_2$ |
| Bu | C(O)NHPr | H | H | H | $-CH_2CH_2CH(CH_3)_2$ |
| Bu | C(O)NHPr | H | H | H | $-CH_2CH(CH_3)CH(CH_3)_2$ |
| Bu | C(O)NHPr | H | H | H | $-CH_2(C_5H_9)$ |
| Bu | C(O)NHPr | H | H | H | $-CH_2CH_2C_5H_9$ |
| Bu | C(O)NHPr | H | H | H | $-CH_2(C_6H_{11})$ |
| Bu | C(O)NHPr | H | H | H | $-CH_2CH_2C_6H_{11}$ |

| | | | | | |
|---|---|---|---|---|---|
| Bu | C(O)NHPr | H | H | H | $-CH_2(C_6H_5)$ |
| Bu | C(O)NHPr | H | H | H | $-CH_2CH_2C_6H_5$ |
| Bu | C(O)NHPr | H | H | H | $-CH(CH_3)CH_2CH_3$ |
| Bu | C(O)NHPr | H | H | Me | -Bu |
| Bu | C(O)NHPr | H | H | Me | -Pentyl |
| Bu | C(O)NHPr | H | H | Me | -Hexyl |
| Bu | C(O)NHPr | H | H | Me | $-CH_2CH(CH_3)_2$ |
| Bu | C(O)NHPr | H | H | Me | $-CH_2CH_2CH(CH_3)_2$ |
| Bu | C(O)NHPr | H | Me | H | -Bu |
| Bu | C(O)NHPr | H | Me | H | -Pentyl |
| Bu | C(O)NHPr | H | Me | H | -Hexyl |
| Bu | C(O)NHPr | H | Me | H | $-CH_2CH(CH_3)_2$ |
| Bu | C(O)NHPr | H | Me | H | $-CH_2CH_2CH(CH_3)_2$ |
| Bu | C(O)NHPr | H | Me | Me | -Bu |

A second subclass of this embodiment includes the compounds of Formula (III).

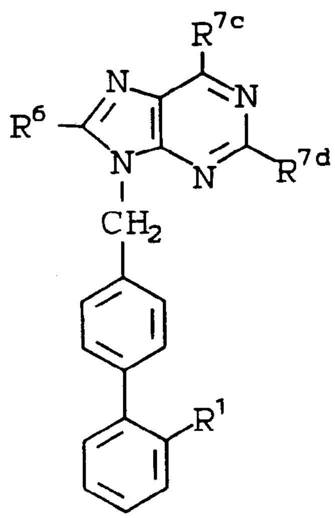

(III)

wherein:

$R^1$ is:

(a) $-SO_2NR^{24}OR^{24}$,

(b) $-SO_2NHSO_2R^{23}$,

(c)

$$-SO_2NH\overset{O}{\overset{\|}{P}}(R^{25})_2,$$

(d) $-SO_2NHCN$,

(e) $-SO_2NHCO_2R^{23}$,

(f) 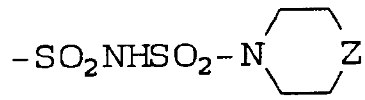,

(g) 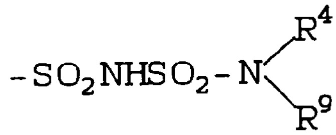,

(h) $-NHSO_2NHSO_2R^{23}$, or

(i)

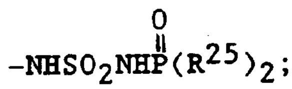

(j)

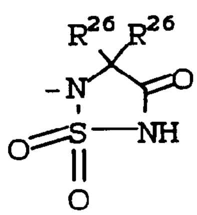,

(k)

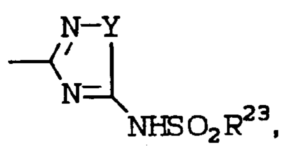,

(l)

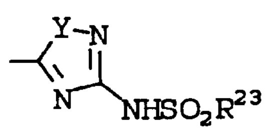,

(m)

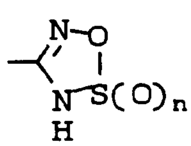,

(n)

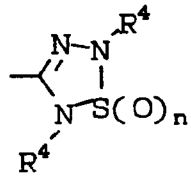
,

(o) $-NHSO_2R^{23}$;

$R^6$ is a $C_1$-$C_6$-alkyl or $C_2$-$C_6$ alkenyl or $C_3$-$C_7$-cycloalkyl;

and

$R^{7c}$ and $R^{7d}$ independently are:

(a) hydrogen,

(b) $C_1$-$C_6$ alkyl or $C_2$-$C_6$ alkenyl,

(c) $-CO_2R^4$,

(d) $-CON(R^4)_2$,

(e) $-NH_2$,

(f)

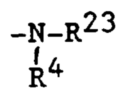
or

(g) 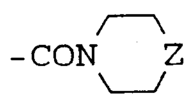 .

wherein Z is $CH_2$, O, or S.

Illustrating this second subclass are the compounds of Formula (III) wherein

$R^1$ is:

(a) $-SO_2N(R^{24})-OR^{24}$,

(b) $-SO_2NHSO_2R^{23}$,

(c)

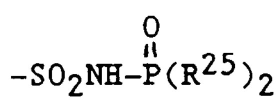

(d) $-SO_2NHCN$,

(e) $-SO_2NHCO_2R^{23}$,

(f)

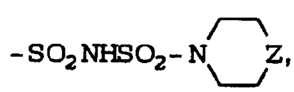

(g)

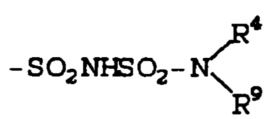
,

(h)

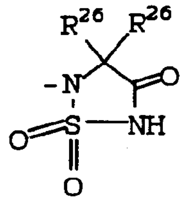

,

(i)

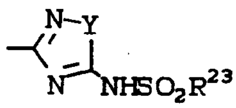

,

(j)

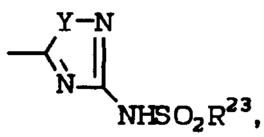

,

(k)

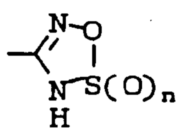

,

(l)

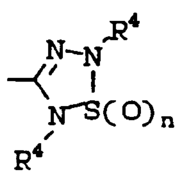

, or

(m) $-NHSO_2R^{23}$;

$R^6$ is $C_1$-$C_6$-alkyl; and

$R^{7c}$ and $R^{7d}$ independently are hydrogen, $C_1$-$C_6$ alkyl, $CO_2R^4$ or $N(R^4)_2$.

Exemplifying this second subclass are the following compounds of the Formula (III) shown in Table B;

## TABLE B

| Compound No. | $R^1$ | $R^6$ | $R^{7c}$ | $R^{7d}$ |
|---|---|---|---|---|
| B1 | $-SO_2NHOH$ | Pr | Me | $-NHCH_3$ |
| B2 | $-SO_2NHOH$ | Pr | Me | 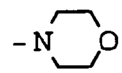 |
| B3 | 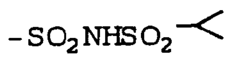 | Pr | Me | 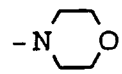 |
| B4 | $-SO_2NH-SO_2Ph$ | Et | Me | $-NHCH_3$ |
| B5 | 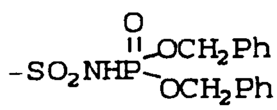 | Pr | Me | 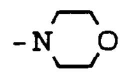 |
| B6 | 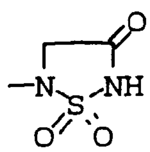 | Et | Me | 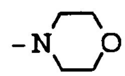 |
| B7 | " | Pr | Me | $-NHCH_3$ |
| B8 | 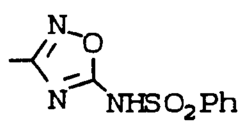 | Pr | Me | 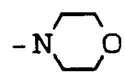 |
| B9 | 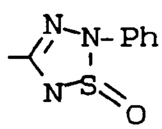 | Pr | Me | $-NHCH_3$ |
| B10 | 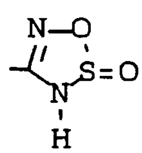 | Et | Me | 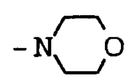 |
| B11 | 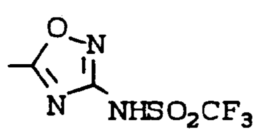 | Pr | Me | 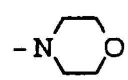 |

A third subclass of this embodiment includes the compounds of Formula (IV)

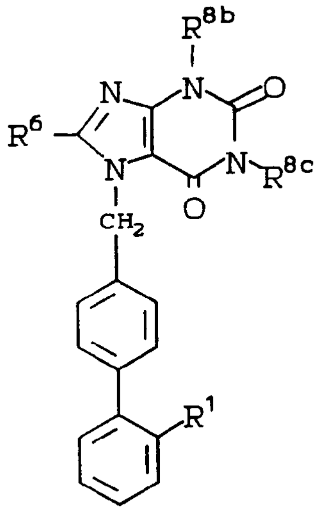

(IV)

wherein:

$R^1$ is:

(a) $-SO_2NR^{24}OR^{24}$,

(b) $-SO_2NHSO_2R^{23}$,

(c)

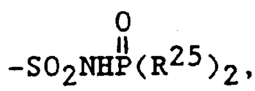

(d) $-SO_2NHCN$,

(e) $-SO_2NHCO_2R^{23}$,

(f)

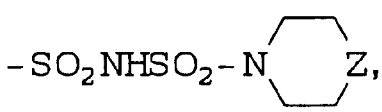

(g)

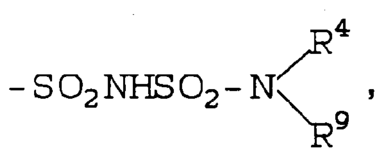

(h) $-NHSO_2NHSO_2R^{23}$,

(i)

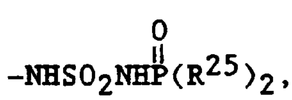

(j)

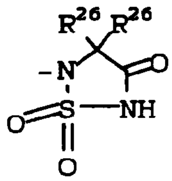

,

(k)

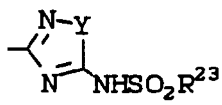

, or

(l) $-NHSO_2R^{23}$;

$R^6$ is $C_1$-$C_6$-alkyl or $C_2$-$C_6$alkenyl or $C_3$-$C_7$-cycloalkyl; and

$R8^b$ and $R^{8c}$ independently are:

(a) hydrogen,

(b) $C_1$-$C_6$ alkyl or $C_2$-$C_6$ alkenyl.

Illustrating this third subclass are the compounds of Formula (IV) wherein

$R^1$ is:

(a) $-SO_2NR^{24}OR^{24}$,

(b) $-SO_2NHSO_2R^{23}$,

(c)

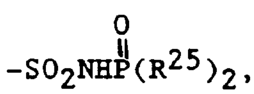

(d) $-SO_2NHCN$,

(e) $-SO_2NHCO_2R^{23}$,

(f)

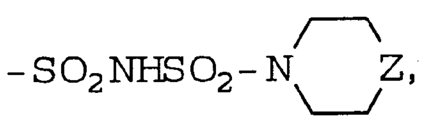

(g)

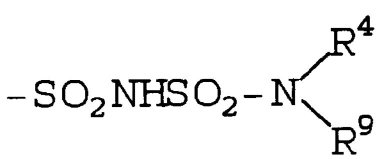

,

(j)

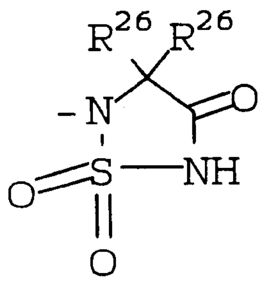

,

(k)

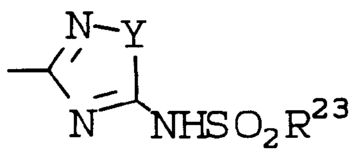

, or

(l)

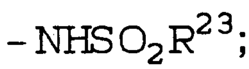

$R^6$ is $C_1$-$C_6$-alkyl; and

$R^{8b}$ and $R^{8c}$ independently are hydrogen, or $C_1$-$C_6$ alkyl.

Exemplifying this third subclass are the following compounds of the Formula (IV) shown in Table C.

## TABLE C

| Compound No. | $R^1$ | $R^6$ | $R^{8b}$ | $R^{8c}$ |
|---|---|---|---|---|
| C1 | $-SO_2NHOH$ | nPr | Me | Me |
| C2 | $-SO_2NHOH$ | nBu | Me | Me |
| C3 | $-SO_2NHSO_2Ph$ | nPr | Me | Me |
| C4 | 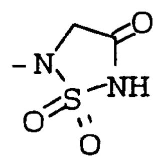 | nPr | Me | Me |
| C5 | 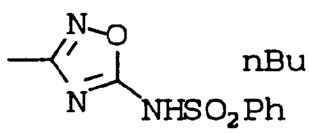 | nBu | Me | Me |

The compounds of Formula (I) can be synthesized using the reactions and techniques described herein below. The reactions are performed in a solvent appropriate to the reagents and materials employed and suitable for the transformation being effected. It is understood by those skilled in the art of organic synthesis that the functionality present on the heterocycle and in the reactants being employed should be consistent with the chemical transformations being conducted. Depending upon the reactions and techniques employed, optimal yields may require changing the order of synthetic steps or use of protecting groups followed by deprotection.

ABBREVIATIONS USED IN REACTION SCHEMES

Reagents

| | |
|---|---|
| NBS | N-bromosuccinimide |
| AIBN | Azo(bis)isobutyronitrile |
| DDQ | Dichlorodicyanoquinone |
| $Ac_2O$ | acetic anhydride |
| TEA | triethylamine |
| DMAP | 4-dimethylaminopyridine |
| $PPh_3$ | triphenylphosphine |
| TFA | trifluoroacetic acid |
| TMS-Cl | trimethylsilyl chloride |
| Im | imidazole |
| AcSK | potassium thioacetate |
| p-TsOH | p-toluenesulfonic acid |

Solvents:

| | |
|---|---|
| $Et_2O$ | diethyl ether |
| DMF | dimethylformamide |
| HOAc (AcOH) | acetic acid |

| | |
|---|---|
| EtOAc (EtAc) | ethyl acetate |
| Hex | hexane |
| THF | tetrahydrofuran |
| DMSO | dimethylsulfoxide |
| MeOH | methanol |
| iPrOH | isopropanol |
| DBU | 1,8-diazabicyclo-[5.4.0] undec-7-ene |
| $Me_3SnCl$ | trimethylstannyl chloride |

Others:

| | |
|---|---|
| rt | room temperature |
| TBDMS | t-butyldimethylsilyl |
| OTf | $OSO_2CF_3$ |
| OTs | $OSO_2$-(4-methyl)phenyl |
| OMs | $OSO_2CH_3$ |
| Ph | phenyl |
| FAB-MS (FABMS) | Fast atom bombardment mass spectroscopy |
| NOE | Nuclear Overhauser Effect |
| $SiO_2$ | silica gel |
| trityl | triphenylmethyl |

As shown in Scheme 1, compounds of Formula (I) can be prepared by carrying out direct alkylation of alkali-metal salts of heterocycles (1) (preparations of heterocycles are illustrated in Schemes 3-6) using appropriately protected benzyl halide, tosylate (OTs) or mesylate (OMs) derivatives (2). The salt is prepared preferably using MH (where M is lithium, sodium or potassium) in anhydrous dimethylformamide (DMF), or by treating it with a metal alkoxide such as sodium or potassium methoxide, ethoxide or t-butoxide in an appropriate alcohol such as methanol, ethanol or t-butanol as the solvent. The alkylation is generally carried out by dissolving the metal salt of the heterocycle in a dipolar aprotic solvent such as DMF or dimethylsulfoxide (DMSO) and reacting it with the alkylating agent at 20°C to reflux temperature of the solvent for 1-24 hours.

## SCHEME 1

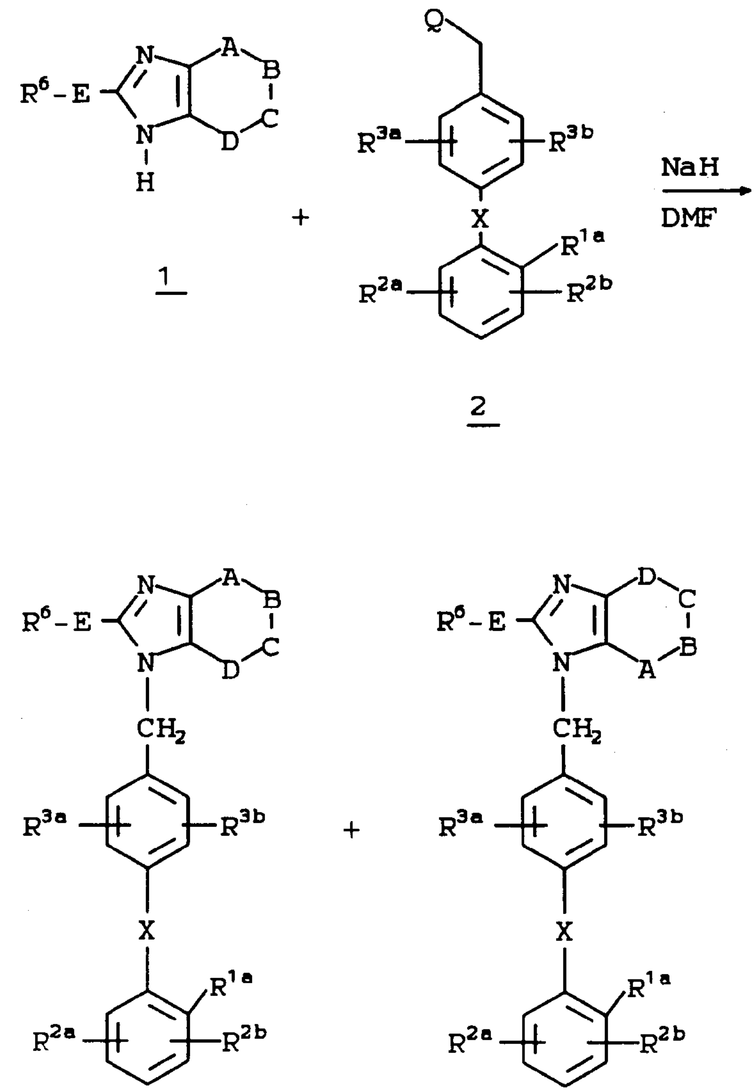


1

2

(I)

(Ia)

where Q = I, Br,Cl , -O-tosyl, -O-mesyl
and $R^{1a}$ = $-NO_2$, -CN, $-COOC(CH_3)_3$, $-SO_2NHC(CH_3)_3$

If substituents and/or the hetero atom positions in the six-membered ring are not symetrically disposed, the alkylation on the imidazole nitrogen(s) generally produces a mixture of two regioisomers as products arising from $N^1$ and $N^3$ alkylation. These regioisomers I and Ia possess distinct physiochemical and biological properties and in most cases can be separated and purified by using conventional separation techniques such as chromatography (flash column chromatography, medium-pressure liquid chromatography, high performance liquid chromatography) and/or crystallization. In those cases where separation of regioisomers is difficult by conventional techniques, the mixture can be transformed into suitable derivatives that can be separated by the above separation methods. The structural assignments of the isomers can be made using Nuclear Overhauser Effect (NOE), $^{1}H$-$^{13}C$ coupled NMR experiments or X-ray crystallography.

When there is potential for alkylation in the 6-membered heterocyclic ring, this can be avoided by the use of suitable protecting groups.

The substituted benzyl halides (2) including the more preferred alkylating agents (8a and 8b and 8c,

Scheme 2) can be prepared as described in European Patent Applications 253,310 and 291,969 and the references cited therein. In addition a preferred method to prepare the biphenyl precursors 7a, 7b using Ni(0) or Pd(0) catalyzed cross-coupling reaction [E. Negishi, T. Takahashi, and A. O. King, Org. Synthesis, 66, 67 (1987)] is outlined in Scheme 2. As shown in Scheme 2, treatment of 4-bromotoluene (3) with t-BuLi, followed by the addition of a solution of $ZnCl_2$, produces the organo-zinc compound (5). Compound (5) is then coupled with (6a) or (6b) in the presence of $Ni(PPh_3)Cl_2$ catalyst to produce the desired biphenyl compound (7a) or (7b). Similarly, 1-bromo-2-nitrobenzene (6c) is coupled with organo-zinc compound (5) in the presence of $Pd(PPh_3)_4$ catalyst [prepared by treating $Cl_2Pd(PPh_3)_2$ with $(i\text{-}Bu)_2A1H$ (2 equiv.)] to give the biphenyl compound (7c). These precursors, (7a), (7b) and (7c), are then transformed into halomethylbiphenyl derivatives (8a), (8b) and (8c), respectively, according to procedures described in European Patent Applications 253,310 and 291,969.

When there is additional substitution on the second phenyl ring ($R^2$ not hydrogen) the preferred method to prepare the biphenyl precursors (7d) and (7e), using the Pd(0) catalyzed cross-coupling reaction [J. K. Stille, Angew. Chem. Int. Ed. Engl., 25, 508 (1986)], is outlined in reaction Scheme 2a. As shown in Scheme 2a, p-tolyltrimethyltin (5a) is coupled with (6d) or (6e) in refluxing toluene in the presence of 5 mole% of $Pd(PPh_3)_4$ to produce the desired biphenyl compounds (7d) and (7e). Table I illustrates the synthetic utility of this protocol. Compounds (7d) ($R^2 = NO_2$) and (7e) ($R^2 = NO_2$) could be converted to their respective chlorides by catalytic hydrogenation, diazotization and treatment with copper (I) chloride. The biphenyl fluorides which could not be obtained by direct coupling to a fluoro arylbromide were prepared from (7d) ($R^2 = NO_2$) and (7e) ($R^2 = NO_2$) via reduction, formation of the diazonium tetrafluoroborate salt and thermal decomposition. These precursors (7d) ($R^2 = NO_2$ or F or Cl) and (7e) ($R^2 = NO_2$ or F or Cl) are then transformed into the halomethyl biphenyl derivatives (8d) and (8e), respectively according to the procedures described in European Patent Applications 253,310 and 292,969.

SCHEME 2

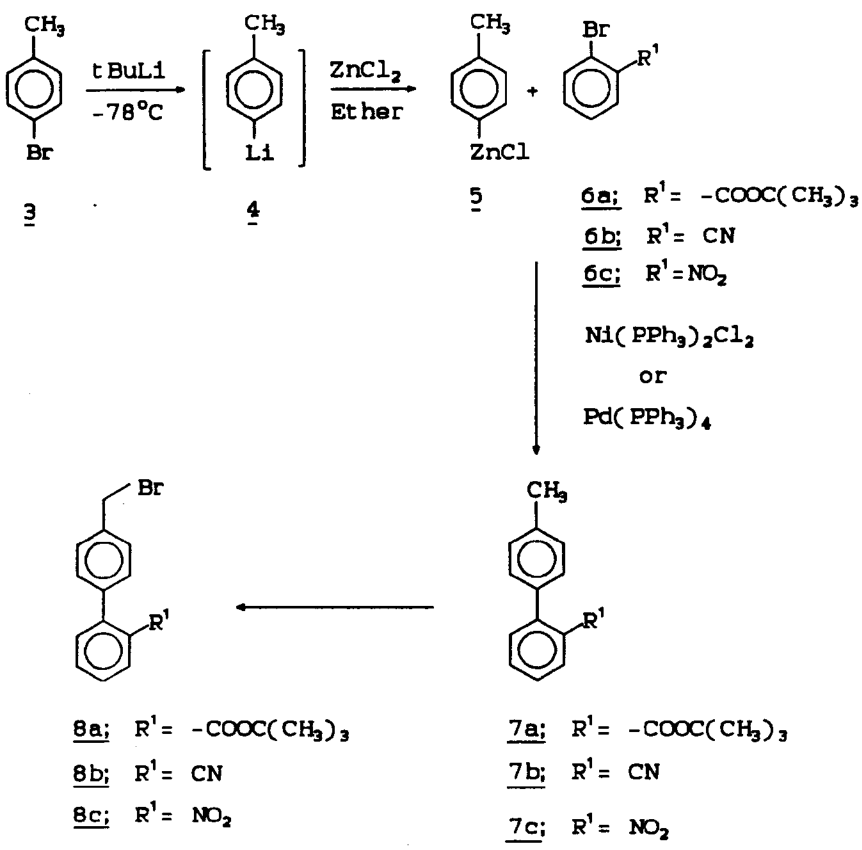


3 4 5

6a; $R^1$ = $-COOC(CH_3)_3$
6b; $R^1$ = CN
6c; $R^1$ = $NO_2$

8a; $R^1$ = $-COOC(CH_3)_3$
8b; $R^1$ = CN
8c; $R^1$ = $NO_2$

7a; $R^1$ = $-COOC(CH_3)_3$
7b; $R^1$ = CN
7c; $R^1$ = $NO_2$

SCHEME 2a

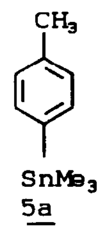

5a

+

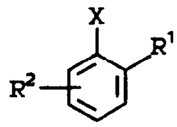

6d: X=Br $R^1$ = CN or $CO_2Me$
$R^2$ = $NO_2$ or F

6e: X=Cl $R^1$ = CN or $CO_2Me$
$R^2$ = $NO_2$ or F

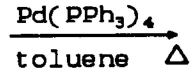


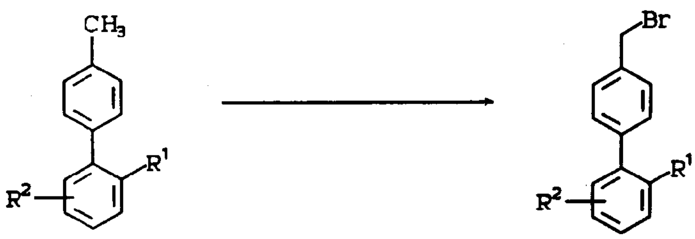

7d: $R^1$ = $CO_2Me$
$R^2$ = $NO_2$ or F

7e: $R^1$ = CN
$R^2$ = $NO_2$ or F

8d: $R^1$ = $CO_2Me$
$R^2$ = $NO_2$ or F or Cl

8e: $R^1$ = $CN_4$-$CPh_3$
$R^2$ = $NO_2$ or F or Cl

## Biphenyl Synthesis Table I

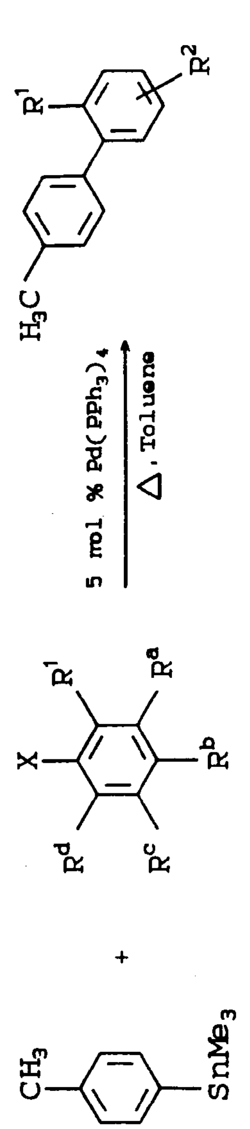


(6d, 6e)

(7d, 7e)

| $\underline{X}$ | $\underline{R^1}$ | $\underline{R^a}$ | $\underline{R^b}$ | $\underline{R^c}$ | $\underline{R^d}$ | Product ($R^2$) | Rf (solvent) | Yield |
|---|---|---|---|---|---|---|---|---|
| Br | $CO_2Me$ | $NO_2$ | H | H | H | 7d (3'-nitro) | 0.35(15:1 Hex/EtOAc) | 71% |
| Br | CN | H | $NO_2$ | H | H | 7e (4'-nitro) | 0.62(2x 6:1 Hex/EtOAc) | 74% |
| Br | $CO_2Me$ | H | F | H | H | 7d (4'-fluoro) | 0.43(15:1 Hex/EtOAc) | 83% |
| Cl | $CO_2Me$ | H | H | $NO_2$ | H | 7d (5'-nitro) | 0.22(15:1 Hex/EtOAc) | 70% |
| Br | $CO_2Me$ | H | H | H | $NO_2$ | 7d (6'-nitro) | 0.24(15:1 Hex/EtOAc) | 79% |
| Br | CN | H | F | H | H | 7e (4'-fluoro) | 0.44(15:1 Hex/EtOAc) | 64% |
| Cl | CN | H | H | F | H | 7e (5'-fluoro) | 0.40(15:1 Hex/EtOAc) | 62% |

The heterocycles of type (1) can be prepared by any of the standard procedures described in the literature [J.A. Montgomery and J.A. Secrist III in "Comprehensive Heterocyclic Chemistry," Vol. 5, A.R. Katritsky and C.W. Rees eds., Pergamon Press 1984; pp 567-597 and 631-656 and references cited therein]. As shown in Scheme 3, the most widely used starting materials are six member heterocyclic vicinal diamines (9). Fused imidazoles (10) can be prepared by condensation of (9) with an appropriate carboxylic acid, nitrile, imidate ester, or orthoesters, either neat, or in a solvent appropriate and compatible with the starting materials and reagents, such as polyphosphoric acid, ethanol, methanol, hydrocarbon solvents, and with a catalytic amount of acid if required. Oxidation of an imine formed by reaction of diamine (9) with an appropriate aldehyde using oxidants such as Cu (II), nitrobenzene, or 2,3-dichloro-5,6-dicyano-1,4-benzoquinone (DDQ) also affords heterocycles

(10). Aminoamides (11, W = H) or diamides (11, W = $R^6CO$) can be converted to fused imidazoles (10) by heating neat, or at an elevated temperature in a solvent such as xylene under acidic or neutral conditions.

## SCHEME 3

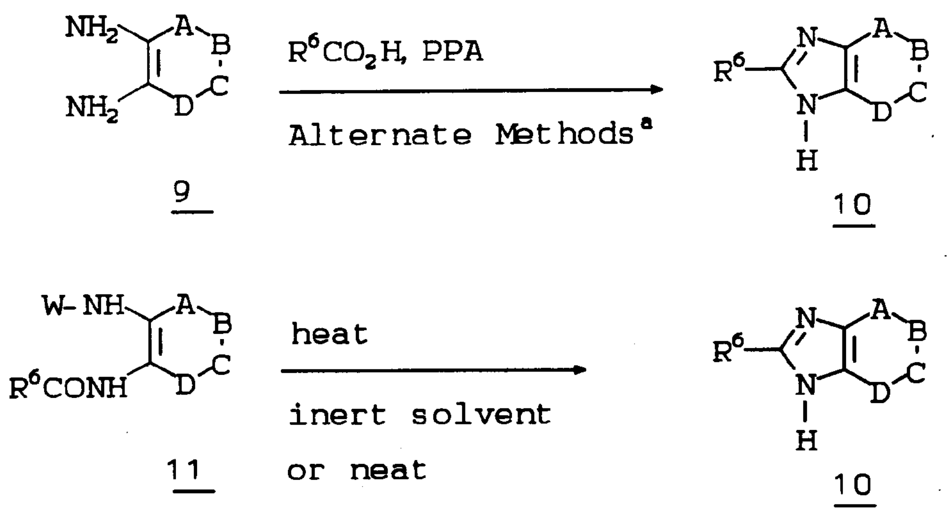


9 10 11 10

W = H or $R^6CO$

[a] Alternate reagents and reaction conditions:

$R^6C$=N, PPA;
$OC_2H_5$
$R^6$-C=NH·HCl, $C_2H_5OH$, Δ;
$R^6C(OCH_3)_3$, toluene, $H^+$, Δ; and
$R^6CHO$, $C_2H_5OH$, $Cu(OCOCH_3)_2$.

As shown in Scheme 4, methods of preparing heterocycles of types (12 and 13) involve treatment of diamines (9) with reagents such as urea, phosgene, potassium cyanate, alkyl chloroformates, dialkylcarbonate, or carbon disulfide in the presence of bases such as potassium hydroxide or potassium carbonate. Amino acids (14) or (15) can be converted to (13) via Curtius or Hoffman rearrangement on suitable derivatives such as acyl azides, hydroxy-amides, or N-haloamides. Bicyclic compounds of type (16, E = sulfur or oxygen) are formed from (12) by reaction under neutral or basic conditions with alkyl halides, alkylmesylates, alkyltosylates, trialkyloxonium salts, or with an appropriate diazoalkane. Compounds of type (16; E = oxygen or sulfur) are prepared by displacement reactions using alkoxides or alkyl mecaptides with chloro intermediates as indicated.

Diamines of type (9) can be prepared by a wide variety of methods such as hydrolysis of bis-amides or amino amides, reduction of dinitro or aminonitro or hydrazino or azido groups, displacement of heteroaromatic halides or alkoxy or thio or alkylthio or hydroxy or alkyl sulfonyl groups with ammonia or amines, or rearrangement of acyl azides or amides or acids (Curtius, Hoffman, or Schmidt rearrangements). [A.S. Tomcufcik, L.N. Starker in "Heterocyclic Compounds, Pyridine and it's Derivatives" Pt 3, E. Klingsberg Ed. Wiley Interscience, 1962, pp 59-62, and references cited there in; T. Nakagome in "Heterocyclic Compounds, Pyridazines" Vol. 28, R.N. Castle, Ed., Wiley Interscience, 1973, pp 597-601, and references cited therein; "Heterocyclic Compounds, The Pyrimidines" Vol. 16, D.J. Brown ed., Wiley Interscience 1985, pp 299-325; E. Schipper, and A.R. Day J. Am. Chem. Soc. (1952) 74, 350; "Comprehensive Heterocyclic Chemistry," Vol. 5, A.R.Katritsky and C.W. Rees Eds., Pergamon Press 1984; pp 567-597 and 631-656 and references cited therein].

SCHEME 4

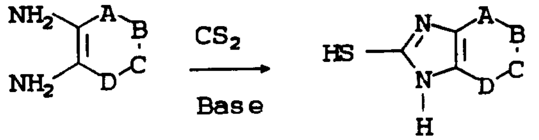


9 12

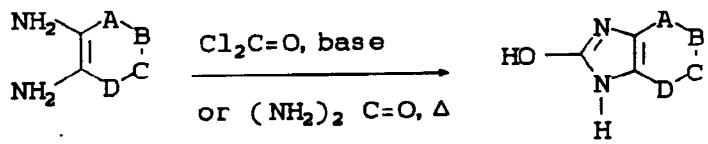


9 13

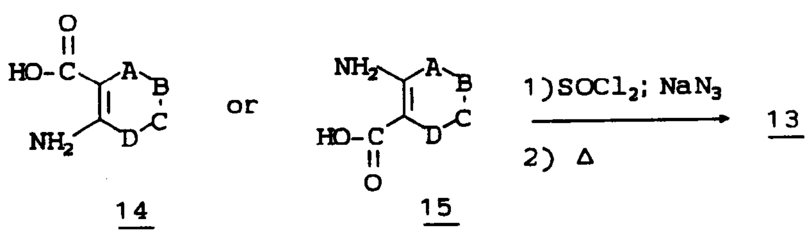


14 15

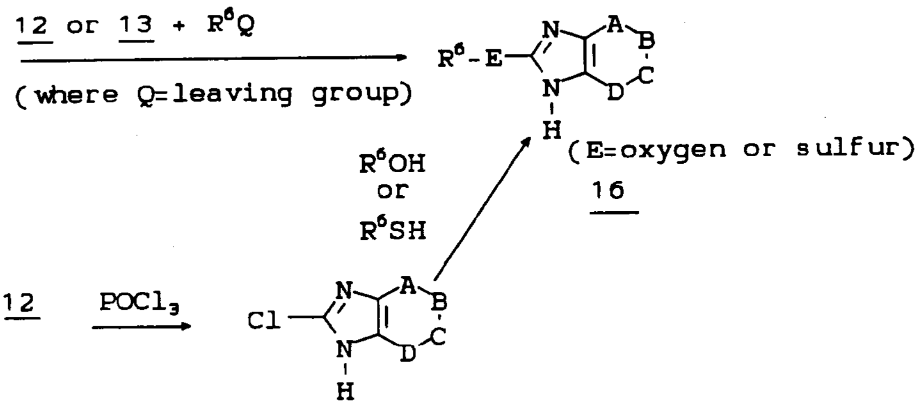


12a

In cases wherein heterocycles of type (10) or (16) are not easily prepared from their corresponding diamines, or when these diamines cannot be prepared then alternative routes, involving fusion of the six member heterocycle onto an appropriately substituted imidazole, are used. Two of these routes are illustrated in Scheme 5. For example, imidazo[4,5-d][1,2,3]triazines (18) are preferentially prepared by treatment of amino carboxamido imidazoles (17) with sodium nitrite in aqueous acid. Precursor imidazoles (17) are prepared by degradation of an appropriately substituted xanthine or by condensation of an appropriate imidate ester with aminocyanoacetamide. Imidazo[4,5-b]pyridazines (20) can be prepared from imidazodicarboxylate esters (19) by treatment with hydrazine. Oxidation of (20) gives pyridazindiones (21). The oxygen(s) in (20) or (21) can be converted to other functionalities such as halides or thiones, which are themselves precursors for the synthesis of more elaborate systems ["Comprehensive Heterocyclic Chemistry," Vol. 5, A.R.Katritsky and C.W. Rees,

eds., Pergamon Press 1984; pp 567-597 and 631-656 and references cited therein].

SCHEME 5

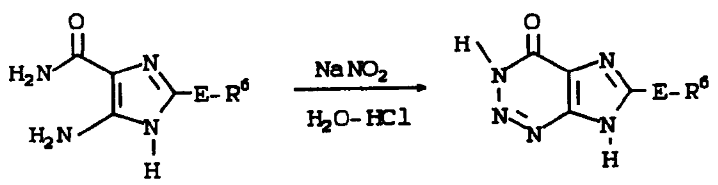


17 18

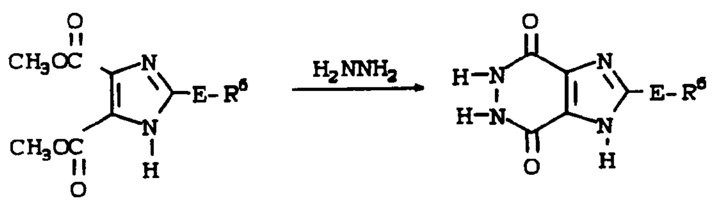


19 20

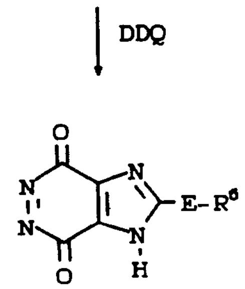


21

Moreover, as shown in Scheme 6, amino imidazole esters and amides are versatile intermediates for the preparation of purines. This scheme also illustrates the synthesis of the 6-membered heterocyclic ring after the alkylating agent (2) has been reacted with a suitably substituted imidazole to afford (22) or (24).

SCHEME 6

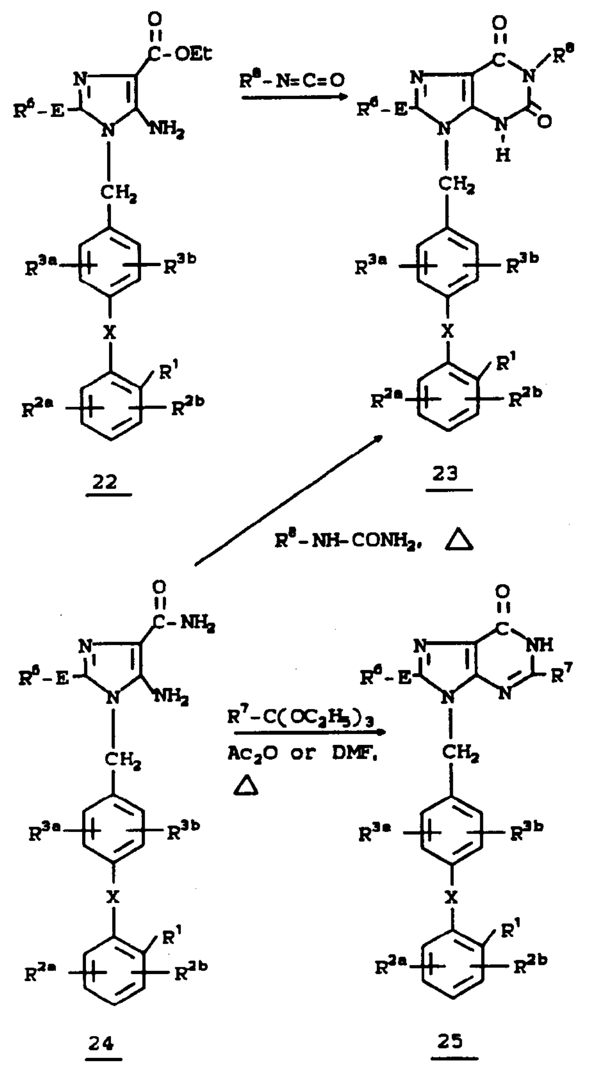


22 23 24 25

The preparation of reduced forms of hetero-cycles can be achieved by catalytic reduction, or by synthesis from a suitable imidazole precursor. For example, histidine and derivatives thereof react with formaldehyde to afford partially saturated imidazo(4,5-c) pyridines [cf. Neuberger, A. Biochem J., (1944), 38, 309].

Halogenation of the imidazo[4,5-b]pyridine ring at the 6-position can be accomplished using $Br_2$, or N-bromosuccinimide. Halogenation of the 7-position can be accomplished by reaction of the corresponding imidazopyridine-4-oxide (prepared by reaction of the imidazopyridine with peracids such as m-chloroperbenzoic acid) with $POCl_3$. When the 7-position is substituted with other than hydrogen, halogenation at the 5-position of the 4(N)-oxide precursor occurs upon treatment with $POCl_3$. Chlorides may be substistuted by bromides or iodides by treatment with either HBn or HI, respectively, in a solvent such as HOAc.

2-Alkyl-imidazo[4,5-b]pyridines can be substituted at the 5, 6, or 7 positions by displacement of a halogen at that position by nucleophiles such as cyanide, amines, copper alkoxides, trialkylphosphites, and thiolates. Also, substitution of the halogens, in particular bromides or iodides, can be accomplished by reaction with a coupling partner such as alkylzinc or arylzinc halides, or monoalkylarylphosphonites in the presence of an appropriate metal catalyst such as nickle, palladium, ruthenium, or platinum. In cases where the reaction is sluggish or complicated due to an acidic proton, the imidazopyridine may be protected at the 1, 3, or 4 positions by benzyl or other arylmethyl groups.

Compounds of formula I where $R^1$ is

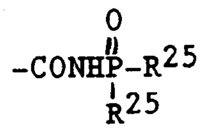

may be prepared from the corresponding carboxylic acid derivatives (I) as outlined in Scheme 7. The carboxylic acid (I), obtained as described in Scheme 1, can be converted into the corresponding amide by treatment with carbonyldiimidazole and then with ammonia. The resulting amide then can be treated with sodium hydride or n-butyllithium in THF at -20°C followed by an appropriately substituted phosphonyl or phosphinyl halide to form the desired compounds (26).

## SCHEME 7

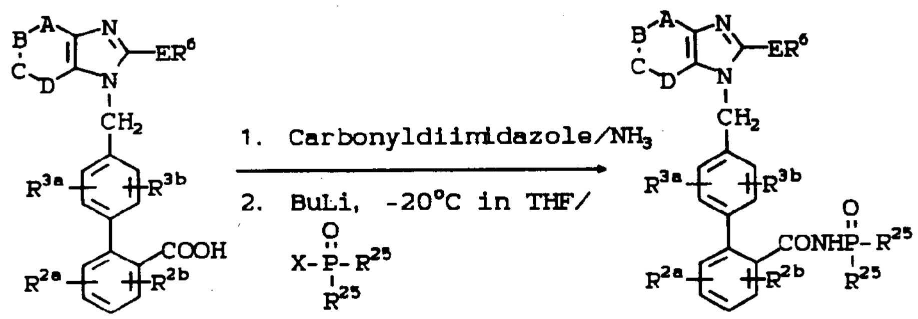


I

26

The biaryl sulfonamides (32) and (37), precursors for the alkylating agent 33, can be prepared from appropriate aryl-organotin precursors using palladium (0) catalyzed cross-coupling reactions [J. K. Stille, Pure Appl. Chem., 57, 1771 (1985); T. R. Baiely, Tetra Lett., 27, 4407 (1986); D. A. Widdowson and Y. Z. Zhang, Tetrahedron, 42, 2111 (1986)], as outlined in Schemes 8 and 9. The organotin compound (29) [S. M. Moerlein, J. Organometallic Chem., 319, 29 (1987)], obtained from the aromatic precursors (27 or 28), may be coupled with aryl sulfonamide (31) using $Pd(PPh_3)_4$ or $(PPh_3)_2PdCl_2$ as catalysts to give biaryl sulfonamide 32. Similarly, the biphenylmethyl bromide (33) may be alternatively prepared from the appropriate organotin precursor (36) using the Pd(0) catalyzed cross-coupling reaction as outlined in Scheme 9.

SCHEME 8

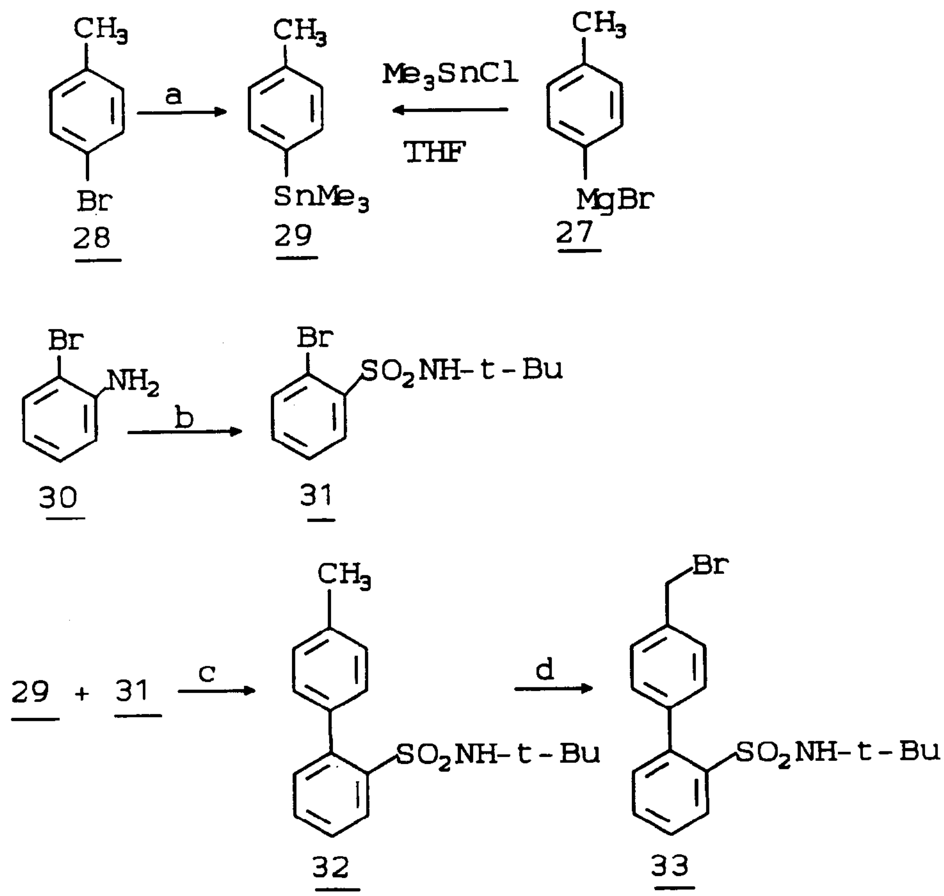


28 29 27

30 31

29 + 31 32 33

a. i) t-BuLi/ether, -78°C ii) $Me_3SnCl$

b. i) $NaNO_2/HCl$ ii) $SO_2$, $CuCl_2$ (iii) t-butylamine

c. $Pd(PPh_3)_4$, Toluene or $(PPh_3)_2PdCl_2$, DMF, Heat

d. $NBS/CCl_4$, AIBN, Reflux

## SCHEME 9

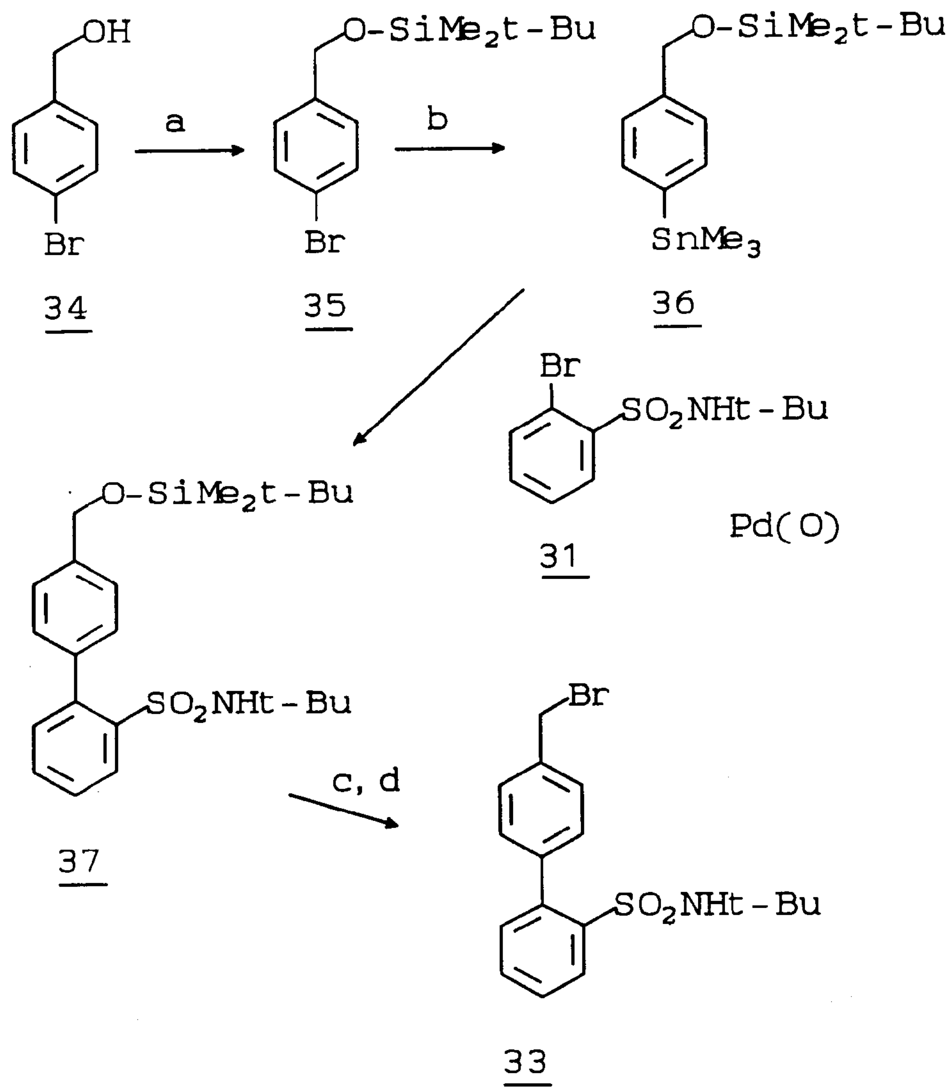


34 35 36

31

37

33

a. t-$BuMe_2Si$-Cl/Imidazole, DMF

b. t-BuLi, -78°C, $Me_3SnCl$

c. Tetrabutylammonium fluoride

d. $CBr_4/Ph_3P$.

Compounds of formula I where $R^1$ is $-SO_2NHSO_2R^{23}$ may be prepared from the key sulfonamide intermediate 38 as outlined in Scheme 10. The intermediate 38 may be prepared by the alkylation of appropriate heterocycles with the alkylating agent 33 as outlined in Scheme 1. Treatment of 38 with trifluoroacetic acid followed by acylation of the resulting sulfonamide 39 with appropriate sulfonyl chlorides may produce the desired compounds (40).

SCHEME 10

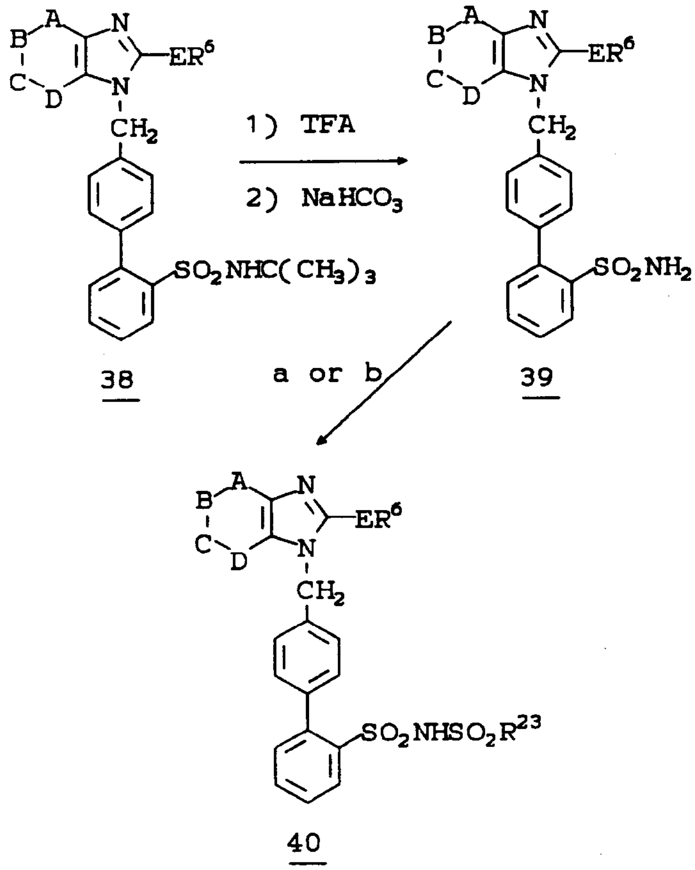


a. i) NaH/THF or DMF (ii) $R^{23}SO_2Cl$
b. $R^{23}SO_2Cl$, DBU, THF

Compounds of Formula (I) wherein $R^1$ is $-SO_2NHCO_2R^{24}$ may be prepared by reacting an appropriate chloroformate with the sulfonamide (39) in pyridine or in the presence of DBU in THF to afford the desired compound (41), as outlined in Scheme 11.

SCHEME 11

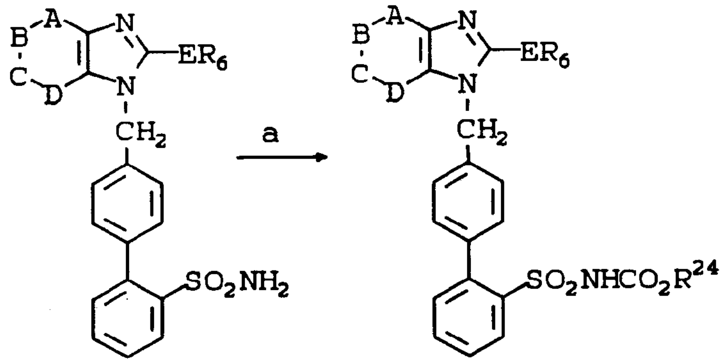


39 41

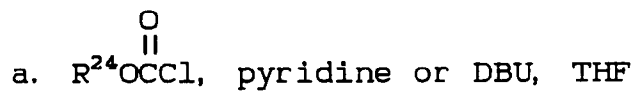


Compounds of Formula (I) wherein $R^1$ is

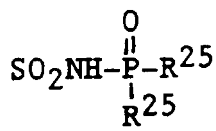

may be prepared by treating sulfonamide (39) with n-butyllithium in THF followed by the treatment of the resulting anion with an appropriately substituted phosphonyl or phosphinyl halide to form the desired compounds (42). (Scheme 12)

SCHEME 12

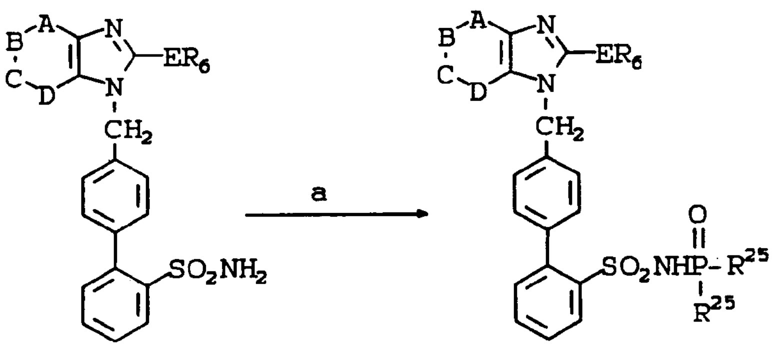


39 42

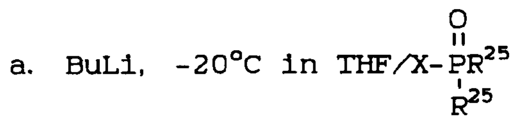


Compounds of Formula (I) wherein $R^1$ is $SO_2NHSO_2N(R^{25})_2$ or

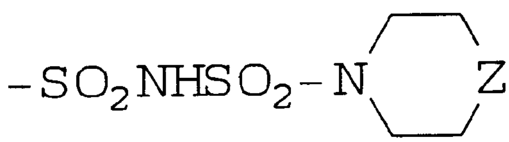

may also be prepared from sulfonamide (39) as outlined in,Scheme 13. Treatment of 39 with n-butyllithium in THF at -25°C and then with an appropriate sulfamoyl halide may produce the desired product (43) or (44).

## SCHEME 13

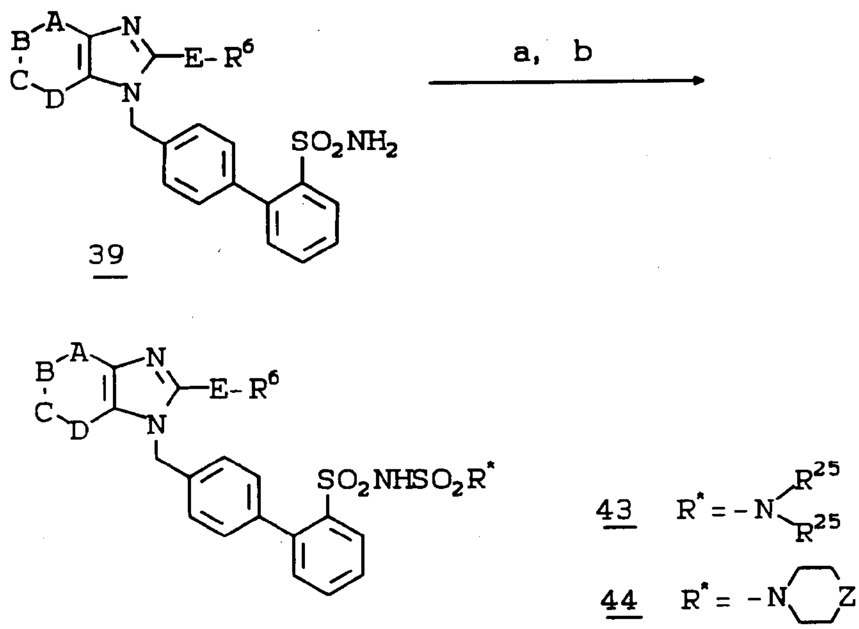


a. nBuLi, -25°C in THF

b. R* $SO_2Cl$

Compounds of Formula (I) wherein $R^1$ is $-NHSO_2NHSO_2R^{23}$ or

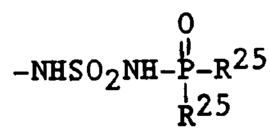

may be prepared from arylamine (46) as outlined in Scheme 14. The arylamine (46) obtained from the corresponding nitro compound 45 can be treated with t-butylsulfamoyl chloride to afford the protected amino sulfonamide (47). The amino sulfonamide (48) obtained after removal of the t-butyl protecting group may then be reacted with an appropriate acylating agent in the presence of a base such as pyridine or DBU in an organic solvent such as THF or DMF to form the desired products (49a) or (49b).

Compounds of the Formula (I) wherein $R^1$ is $-NHSO_2R^{23}$ may be prepared by the reaction of an appropriate sulfonyl halide ($R^{23}SO_2Cl$) or sulfonyl imidazole derivative with the aryl amine 46 in the presence of an appropriate base such as pyridine, triethylamine or DBU.

## SCHEME 14

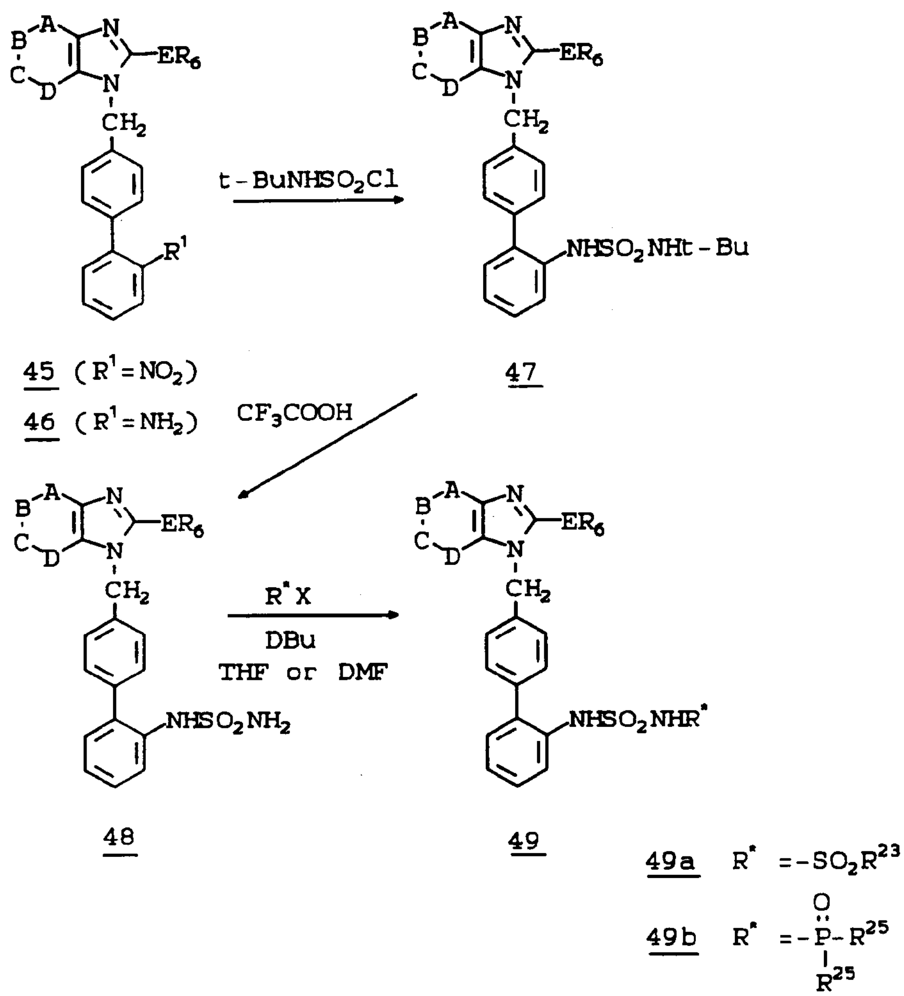


45 ($R^1 = NO_2$)

46 ($R^1 = NH_2$)

47

48

49

49a $R^* = -SO_2R^{23}$

49b $R^*$ =

Compounds of Formula (I) and the benzyl halides of the formula (55) wherein $R^1$ is 1,2,3,5-oxathiadiazole-2-oxide may be prepared from the corresponding cyano derivative (50) or cyano precursor (7b) as outlined in Schemes 15 and 16, respectively utilizing procedures described in U.S. Patent 4,910,019. The cyano derivatives (50), obtained as described in Scheme 1, can be converted into the corresponding amidoxime (51) by treatment with hydroxylamine hydrochloride and sodium methoxide in an organic solvent, such as methanol or DMSO. The amidoxime (51) then can be treated with base and thionyl chloride in an aprotic solvent to form the desired 1,2,3,5-oxathiadiazole-2-oxide (52). Similarly, the oxathiadiazole-2,2-dioxide 52a can be prepared by treatment of amidoxime 51 with a base and sulfuryl chloride. As shown in Scheme 16, the cyano precursor (7b) may be converted into the desired 1,2,3,5-oxathiadiazole (54) which is then protected with the trityl group prior to the formation of the desired benzyl halide (55). The protecting group is removed subsequent to the alkylation of heterocycle (1) to give the desired product (52).

SCHEME 15

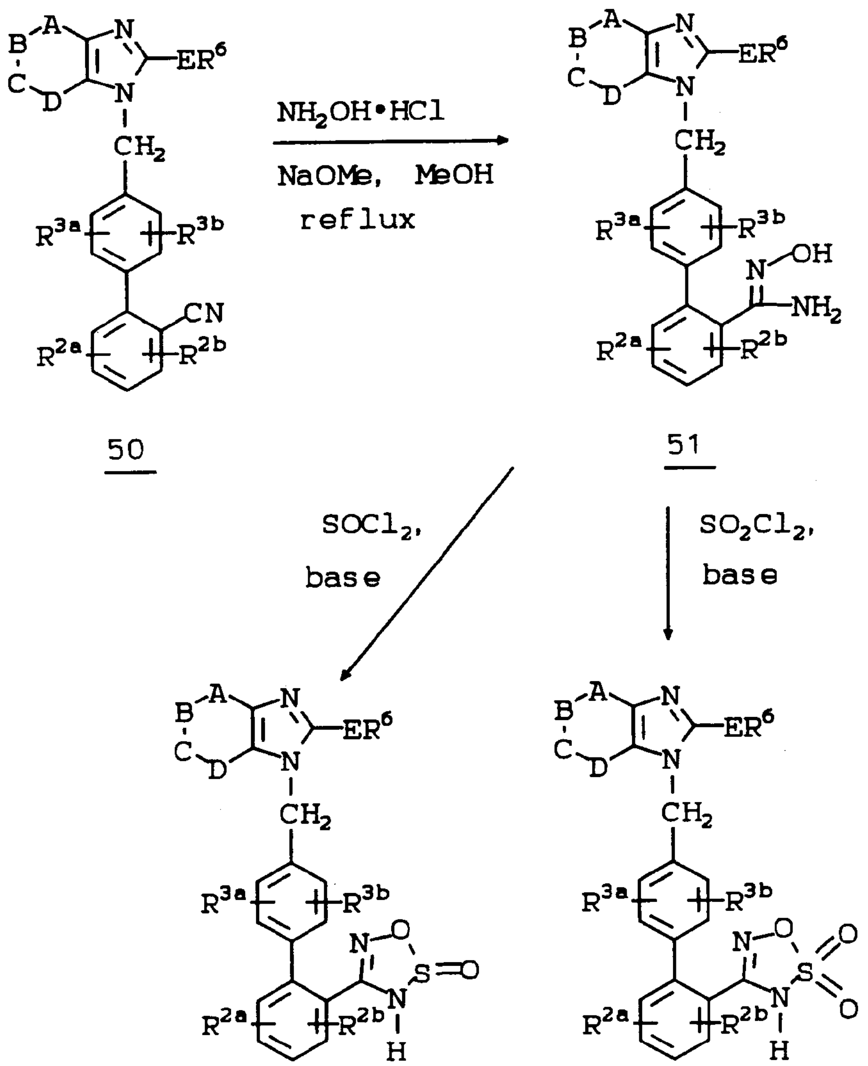


50

51

52

52a

SCHEME 16

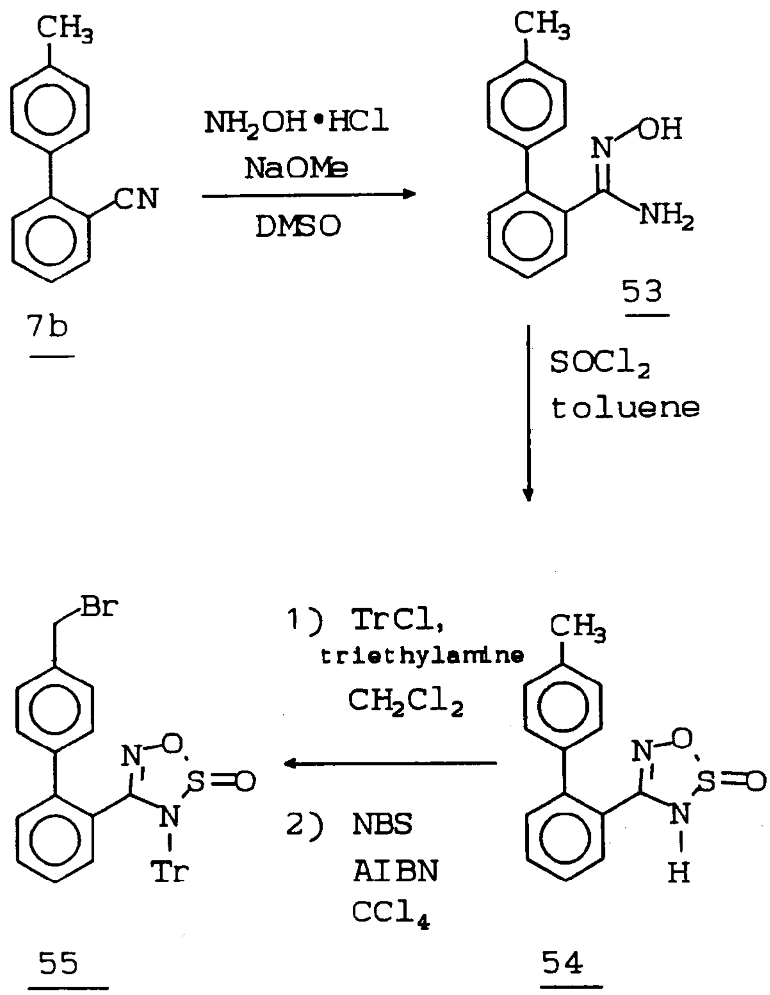


Compounds of Formula (I) and the benzyl halides of the formula (2) wherein $R^1$ is 1,2,3,5-thiatriazole-1-oxide may be prepared from the corresponding carboxamido precursor or cyano precursor as outlined in Scheme 17 and 18, respectively. Intermediates (57) and (61) can be treated with $SOCl_2$ (see procedures in: Ber. Deutsh. Chem. Ges. 1971, 104 pp 639) to give intermediates, (58) and (62), each of which may be protected with a trityl group and subsequently brominated to give (60) and (64), respectively. Alternatively, (60) and (64) may be prepared as shown in Scheme 19 and 20. Treatment of (65) with $SOCl_2$ (see procedures in: Ber. Deutsch. Chem. Ges. 1971, 104 pp 639) provides (66), which under mild hydrolytic conditions provides (58). The conversion of (58) to (60) is as described for Scheme 17. Alkylation of the trityl protected analog (67) by treatment with a base such as NaH and an alkyl halide would provide (59), which then may be converted to (64) as previously described.

Compounds of Formula (I) wherein $R^1$ is 1,2,3,5-thiatriazole-1-oxide may also be prepared as exemplified in Example 14.

SCHEME 17

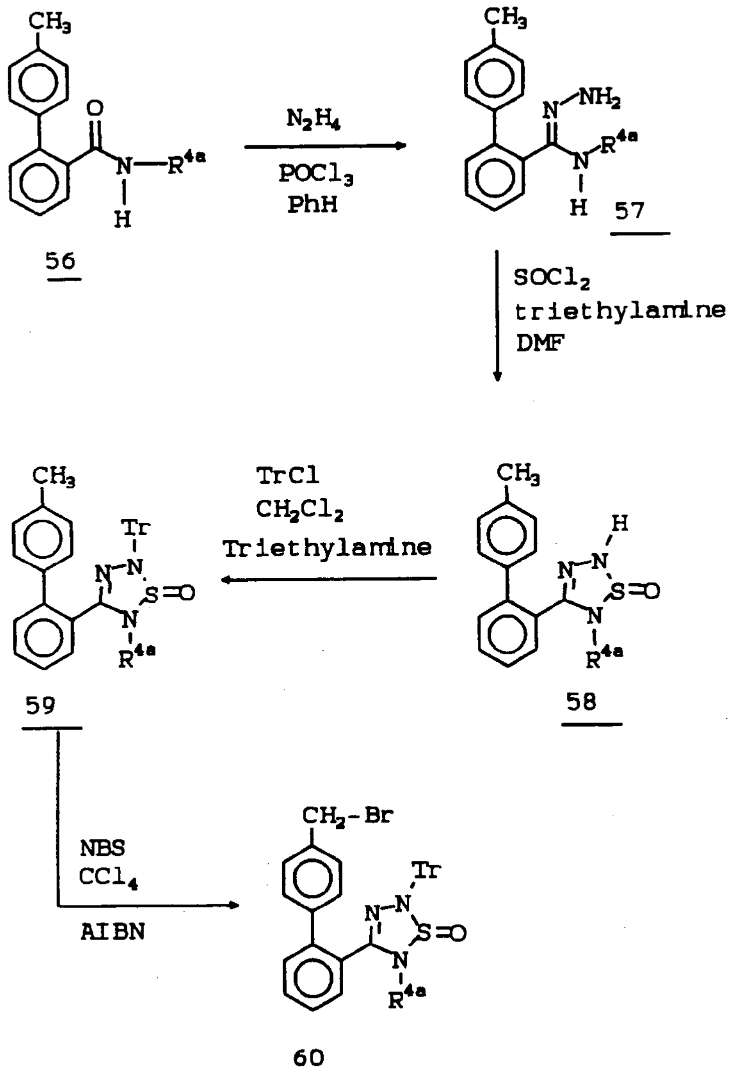


56

57

58

59

60

## SCHEME 18

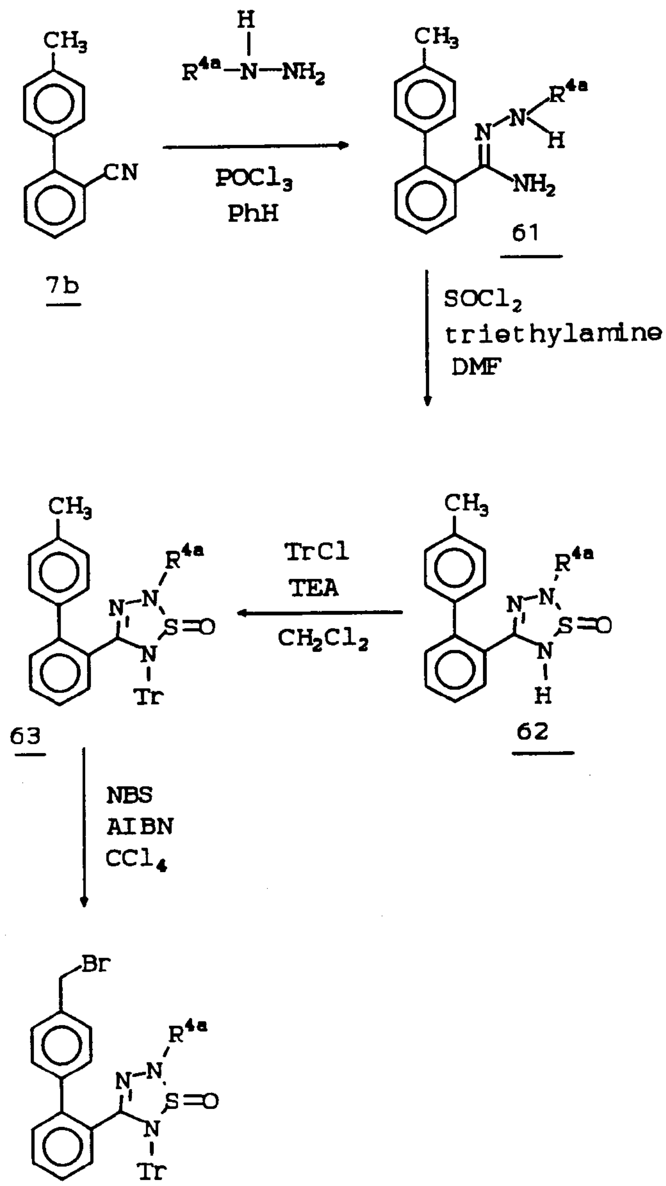


7b, 61, 62, 63, 64

SCHEME 18a

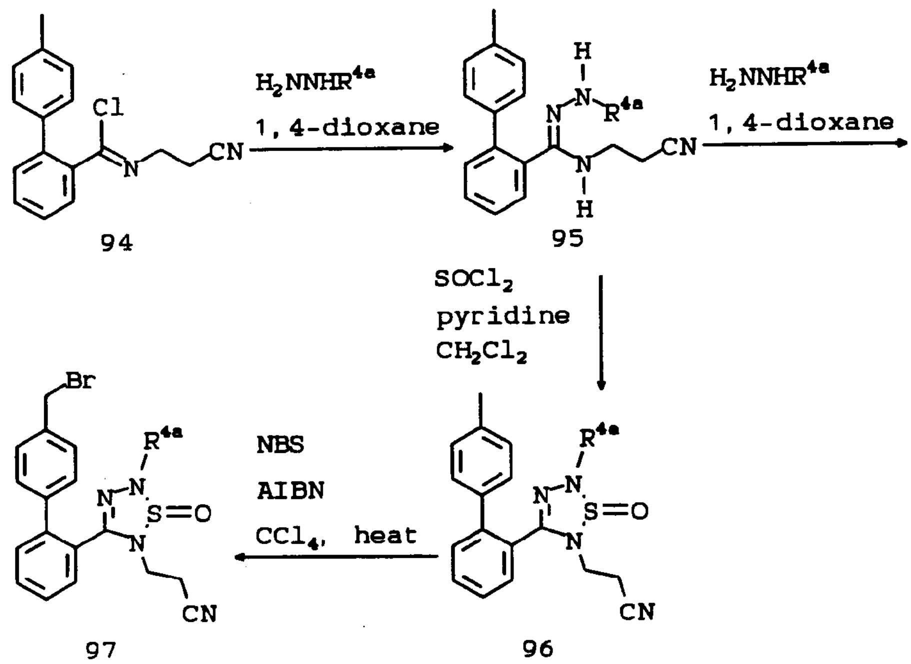

94
95
96
97
$H_2NNHR^{4a}$
1,4-dioxane
$H_2NNHR^{4a}$
1,4-dioxane
$SOCl_2$
pyridine
$CH_2Cl_2$
NBS
AIBN
$CCl_4$, heat

SCHEME 19

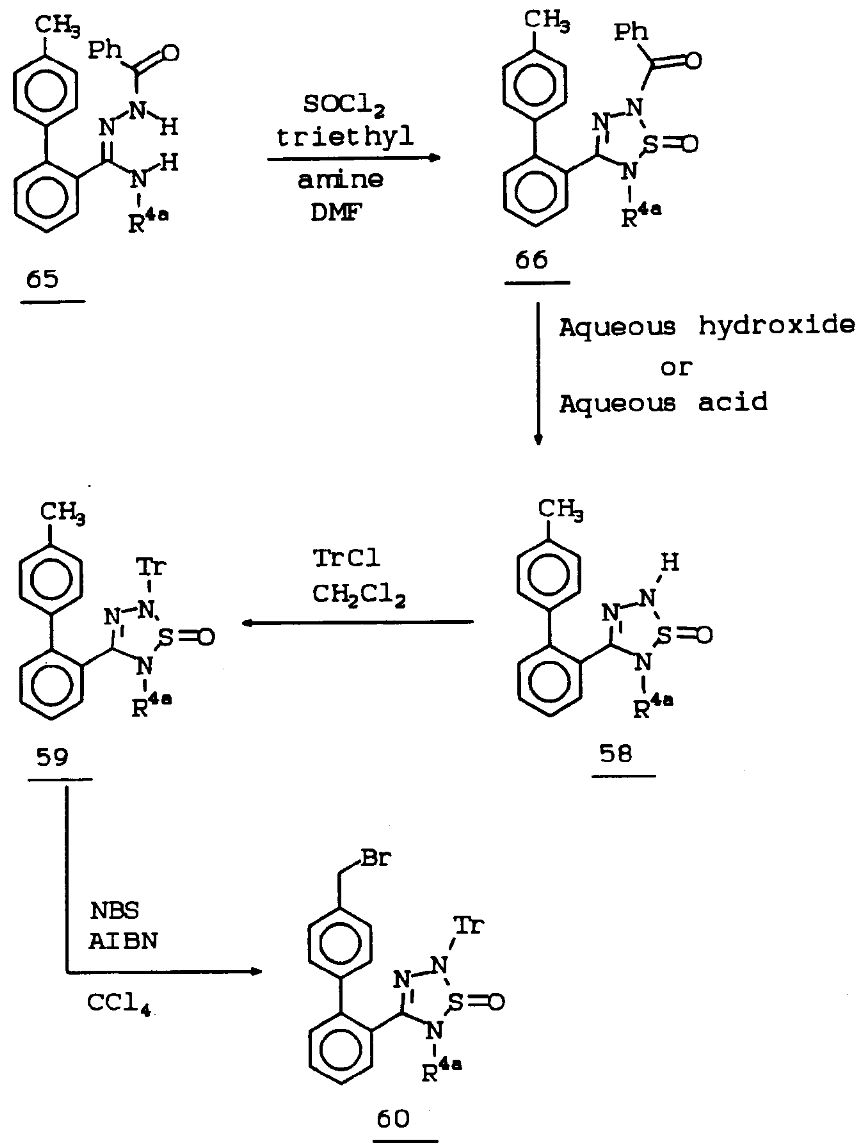


65

66

59

58

60

## SCHEME 20

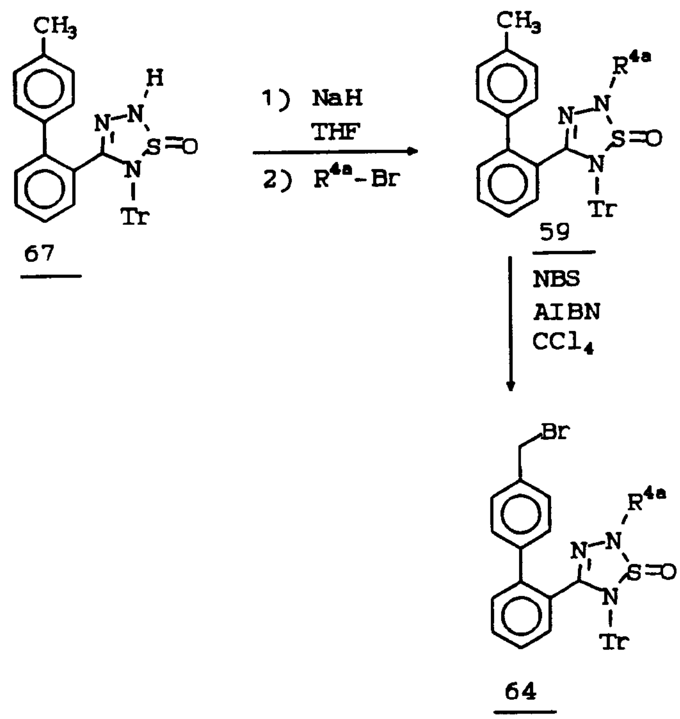


Compounds of Formula (I) and the benzyl halides of formula (2) wherein $R^1$ is 1,2,3,5-thiatriazole-1,1-dioxide-4-yl- may be prepared using procedures described in Monatsh. Chem., 1985, 116, pp 1321 and described herein. Sequential treatment of intermediates such as (61) or (57) with n-BuLi and $SO_2F_2$ will provide the 1,2,3,5-thiatriazol-1,1-dioxide analogs of (58) and (62). Further elaboration of the afore mentioned analogs by the methods described for the conversion of (58) to (60) in Scheme 17 and the methods described for the conversion of (62) to (64) in Scheme 18 would give the benzyl halides of formula (2) wherein $R^1$ is 2-triphenylmethyl-1,2,3,5-thiatriazole-1,1-dioxide-4-yl- and 5-triphenylmethyl-1,2,3,5-thiatriazole-1,1-dioxide-4-yl, respectively.

Compound of Formula (I) wherein $R^1$ is 3-oxo-1,2,4-thiadiazolidine-1,1-dioxide may be prepared from the nitro derivative (7c) as outlined in Scheme 21. The amino compound 68 obtained from 7c may be reacted with t-butyl sulfamoylchloride to form the intermediate 69, which then can be alkylated with an appropriate bromoacetic acid derivative to give 70. Treatment of 70 with trifluoroacetic acid followed by the treatment with an appropriate base such as sodium or potassium alkoxide may produce the desired compound 71, which can be elaborated further to give the key alkylating agent 73 as outline in the scheme. Alkylation of an appropriate heterocyclic compound with 73 may then furnish the desired antagonist.

SCHEME 21

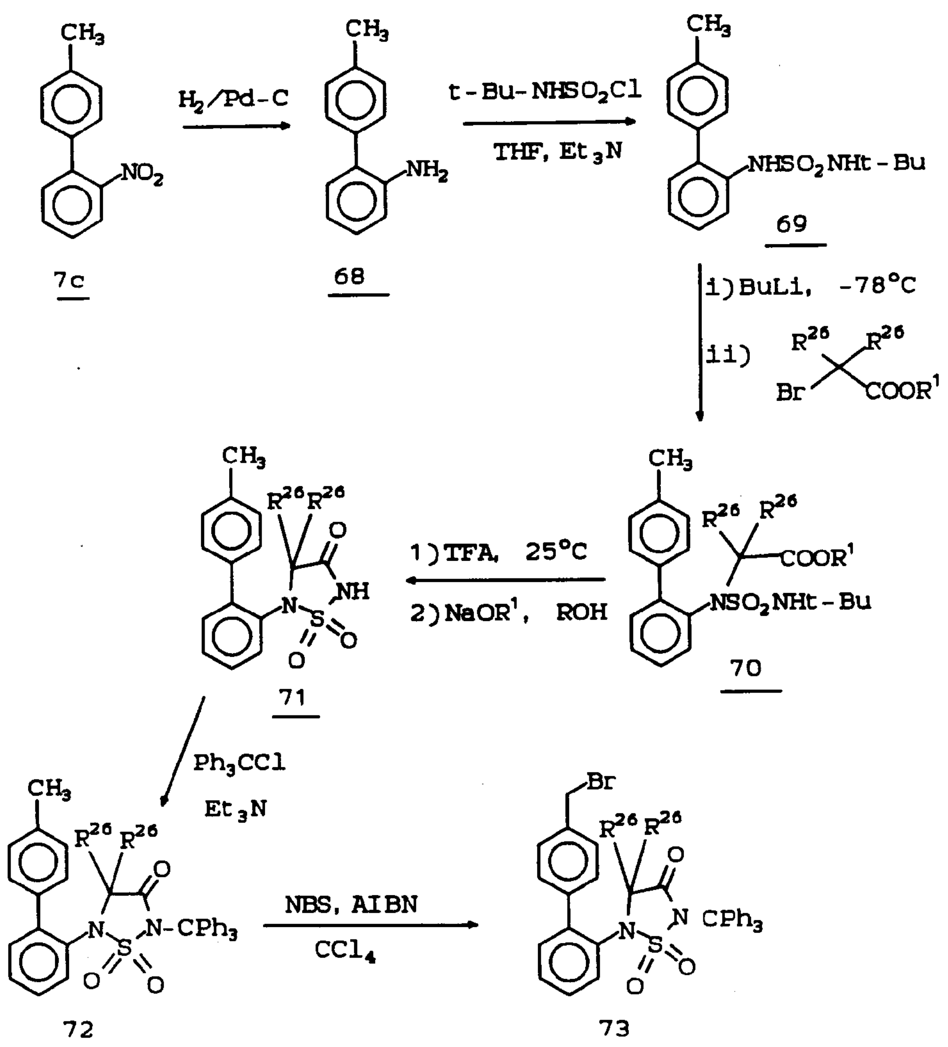


Compound of Formula (I) wherein $R^1$ is 5-aminosulfonyl-1,2,4-oxadiazole may be prepared using the bromomethyl biphenyl derivative 77 and an appropriate heterocyclic compound. The synthesis of 77 can be accomplished as outlined in Scheme 22. The amidoxime 53 may be reacted with S-methylisothiourea to form the 5-amino-1,2,4-oxadiazole 74, which can be then treated with an appropriate sulfonylchloride to give the corresponding 5-aminosulfonyl-1,2,4-oxadiazole 75. The appropriately protected derivative 76 then can be brominated to form the desired alkylating agent 77.

SCHEME 22

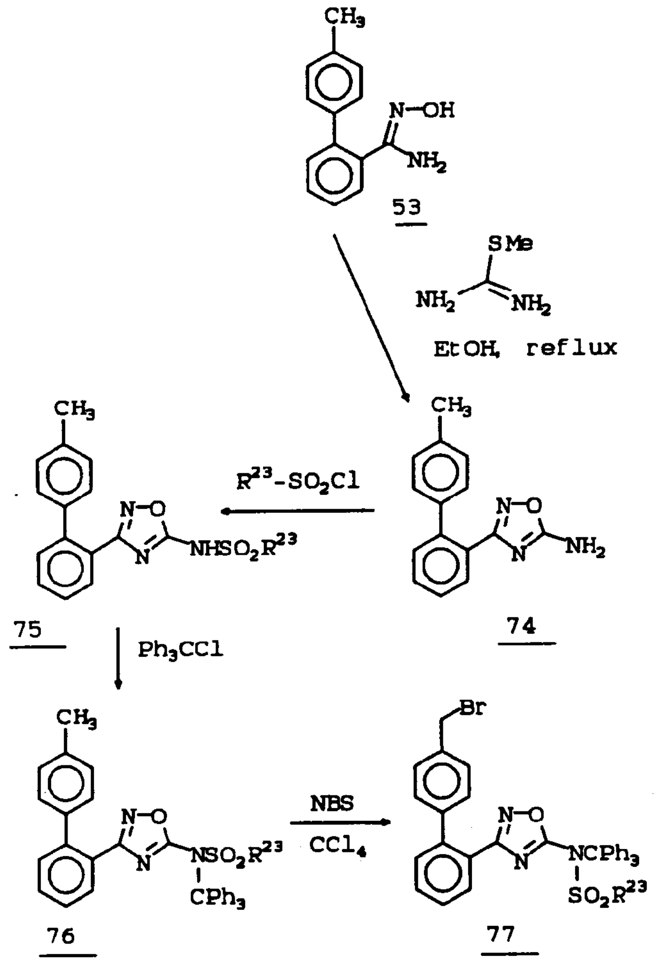


53, 74, 75, 76, 77

Compounds of Formula (I) wherein $R^1$ is 3-aminosulfonyl-1,2,4-oxadiazole can be prepared starting from the carboxylate derivative (7a) as outlined in Scheme 23. The ester derivative 78 obtained from 7a is treated with N-hydroxy guanidine sulfate in the presence of an alkoxide base to form the 3-amino-1,2,4-oxadiazole derivative 79, which may be reacted with an appropriate sulfonyl chloride to give the 3-aminosulfonyl-1,2,4-oxadiazole compound 80. The compound 81 can be prepared from 80 as outlined in Scheme 23.

SCHEME 23

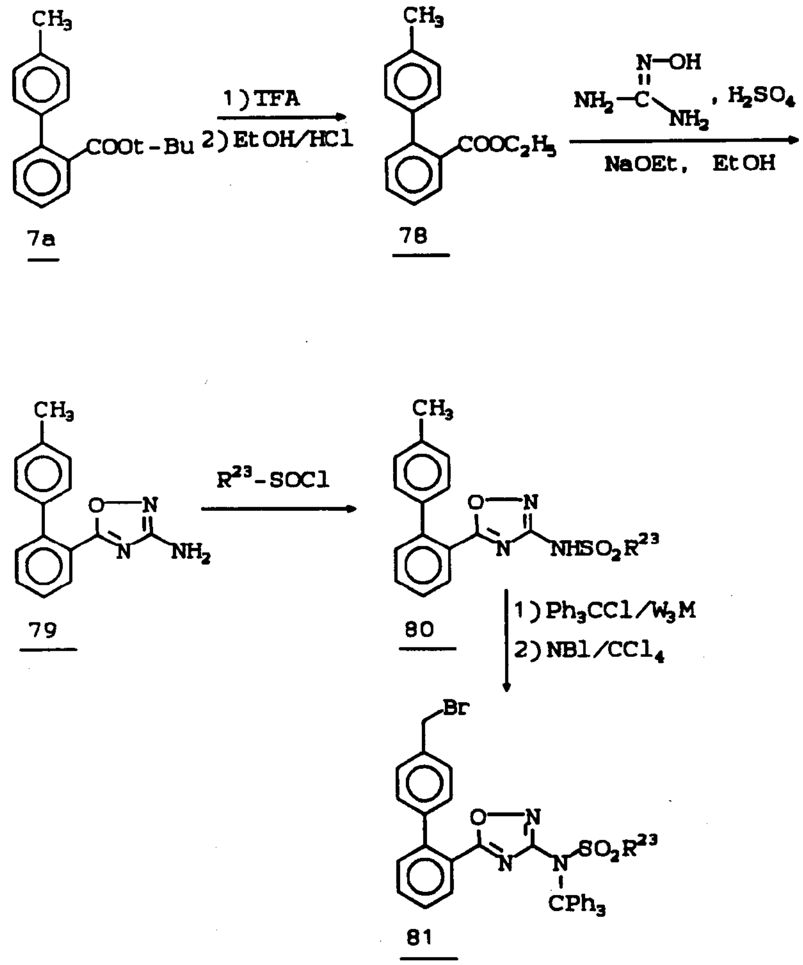


7a 78 79 80 81

Compounds of Formula (I) and the benzyl halides of formula (2) wherein $R^1$ is 1,2,3-oxathiazin-4(3H)-one-2,2-dioxide-6-yl- may be prepared as outlined in Scheme 24. As shown and according to procedures in Angew. Chem. Int. Edn., (1973), 12, pp 869, the betaketoester (82) is treated with fluorosulphonyl isocyante, heated to extrude $CO_2$ and iso-butene, then treated with base such as KOH to form the oxathiazolinone dioxide intermediate (83). Treatment of (83) with triphenylmethyl chloride and triethylamine in $CH_2Cl_2$ gives (84) which in turn is converted to benzyl halide (85) by treatment with N-bromosuccinimide, AIBN, in $CCl_4$ at reflux.

SCHEME 24

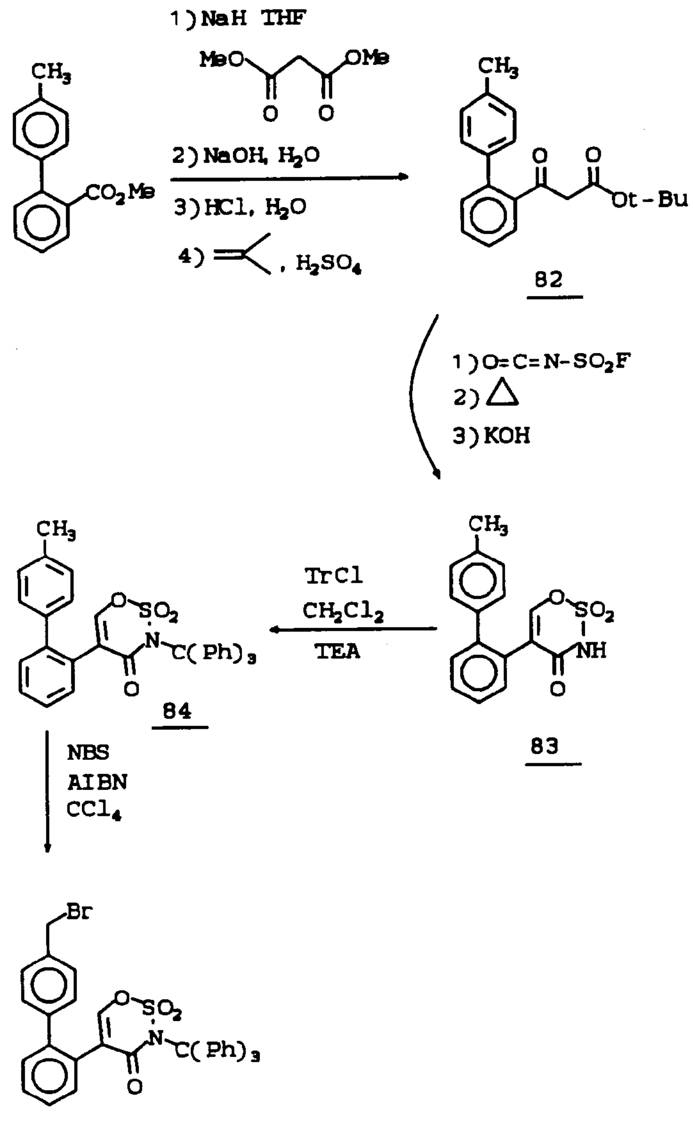


82

83

84

85

Compounds of Formula (I) wherein R[1] is oxamic acid may be prepared utilizing procedures described in J. Med. Chem., 1981, 24, pp 742-748 and as outlined in Scheme 25. The amine (46) is reacted with ethyl oxalyl chloride in the presence of a base such as pyridine or triethylamine and a solvent such as $CH_2Cl_2$ to form the intermediate oxalyl ester which is subsequently saponified with hydroxide to form oxamic acid (86).

SCHEME 25

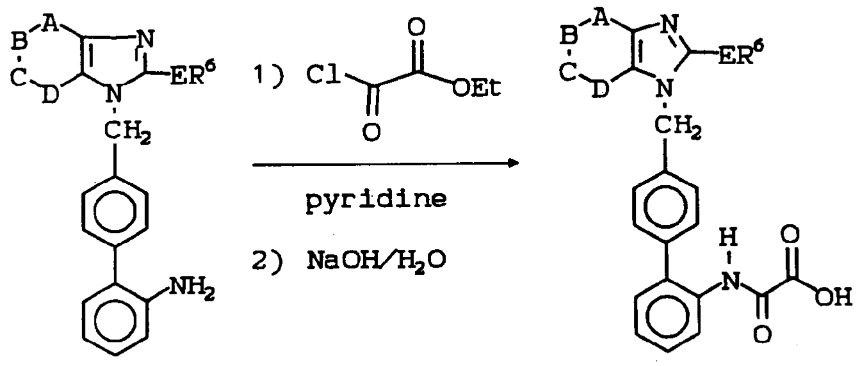


46 86

Compounds of Formula (I) wherein $R^1$ is $-SO_2NR^{24}OR^{24}$ may be prepared as outlined in Scheme 26. The key intermediate 89 is prepared by the reaction of an appropriate heterocyclic compound (1), preferably as an alkali metal salt, with the alkylating agent 87 (prepared from 36). The compound 91, prepared from the sulfonyl chloride 90 and O-t-butylhydroxylamine, is then reacted with 89 in the presence of a Pd(0) catalyst to give 92. Removal of the t-butyl protecting group produces the desired N-hydroxy sulfonamide 93.

SCHEME 26

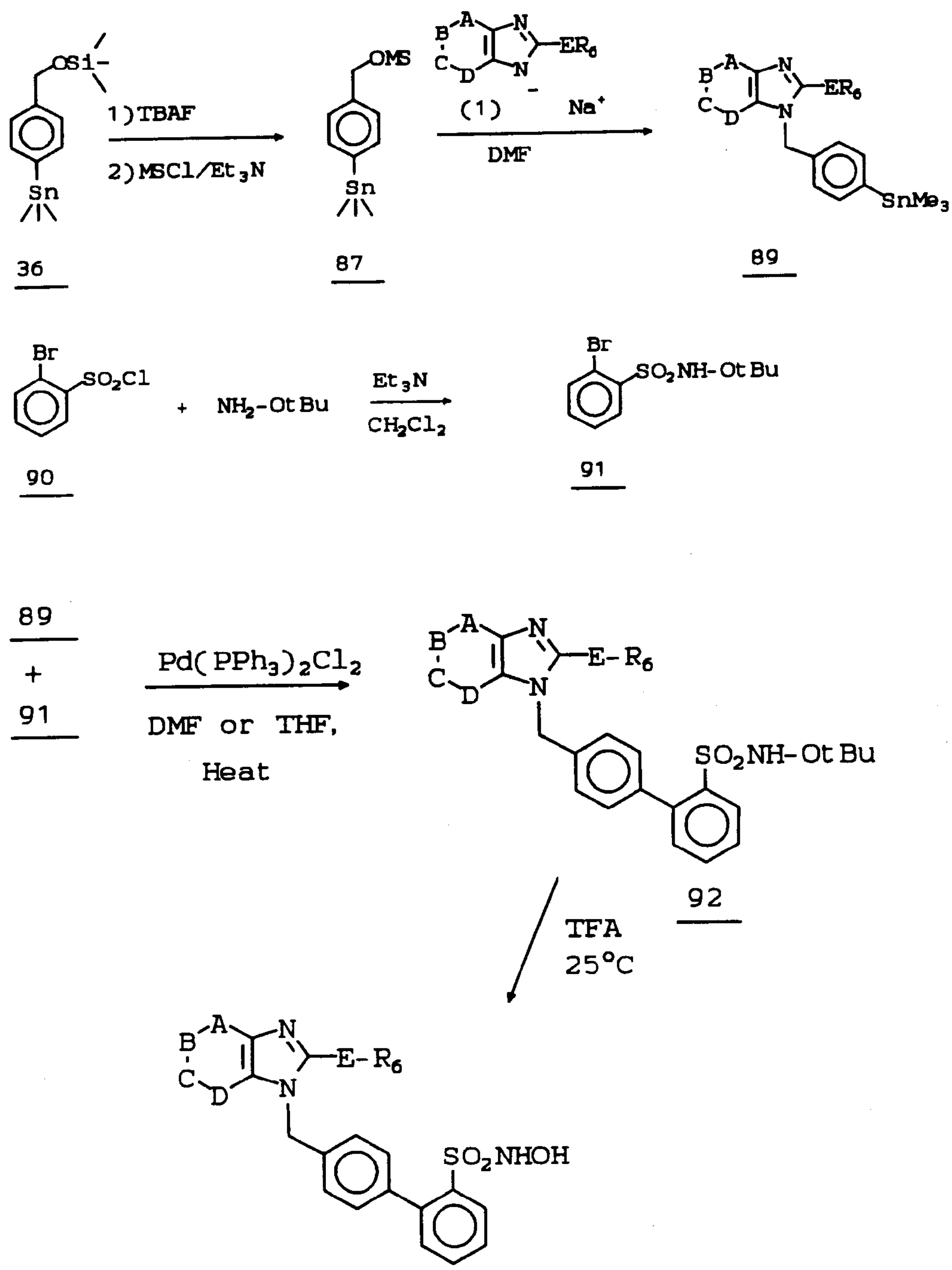


36 87 89

90 91

89 + 91

92

93

It will be appreciated by those skilled in the art that functional group transformations can be conducted on aryl and heterocyclic rings to afford desired analogs. For example, esters may be converted to amides by heating them with amines and an amide nitrogen if present in the heterocycle may be alkylated using bases such as sodium hydride in DMF with the appropriate alkyl halide. Functional group protection throughout these syntheses will be chosen to be compatible with subsequent reaction conditions. Ultimately such protecting groups will be removed to generate the desired optimally active compounds of Formula 1. For example, $R^1$ as carboxyl is often protected as its t-butyl ester which in the last step is removed by treatment with trifluoroacetic acid.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases; e.g., dicyclohexylamine salts, N-methyl-D-glucamine, salts with amino acids like arginine, lysine, and the like. Also, salts with organic and inorganic acids may be prepared; e.g., HCl, HBr, $H_2SO_4$, $H_3PO_4$, methane-sulfonic, toluenesulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Angiotensin II (AII) is a powerful arterial vasoconstrictor, and it exerts its action by interacting with specific receptors present on cell membranes. The compounds described in the present invention act as competitive antagonists of AII at the receptors. In order to identify AII antagonists and determine their efficacy in vitro, the following two ligand-receptor binding assays were established.

Receptor binding assay using rabbit aortae membrane preparation:

Three frozen rabbit aortae (obtained from Pel-Freeze Biologicals) were suspended in 5mM Tris-0.25M Sucrose, pH 7.4 buffer (50 ml) homogenized, and then centrifuged. The mixture was filtered through a cheesecloth and the supernatant was centrifuged for 30 minutes at 20,000 rpm at 4°C. The pellet thus obtained was resuspended in 30 ml of 50mM Tris-5 mM $MgCl_2$ buffer containing 0.2% Bovine Serum Albumin and 0.2 mg/ml Bacitration and the suspension was used for 100 assay tubes. Samples tested for screening were done in duplicate. To the membrane preparation (0.25 ml) there was added $^{125}$ISar$^1$Ile$^8$-angiotensin II [obtained from New England Nuclear] (10ul; 20,000 cpm) with or without the test sample and the mixture was incubated at 37°C for 90 minutes. The mixture was then diluted with ice-cold 50mM Tris-0.9% NaCl, pH 7.4 (4ml) and filtered through a glass fiber filter (GF/B Whatman 2.4" diameter). The filter was soaked in scintillation cocktail (10 ml) and counted for radioactivity using Packard 2660 Tricarb liquid scintillation counter. The inhibitory concentration ($IC_{50}$) of potential AII antagonist which gives 50% displacement of the total specifically bound $^{125}$I-Sar$^1$Ile$^8$-angiotensin II was presented as a measure of the efficacy of such compounds as AII antagonists.

Receptor assay using Bovine adrenal cortex preparation

Bovine adrenal cortex was selected as the source of AII receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added 3H-angiotensin II (50mM) (10ul) with or without the test sample and the mixture was incubated at 37°C for 1 hour. The mixture was then diluted with Tris buffer (4 ml) and filtered through a glass fiber filter (GF/B Whatman 2.4" diameter). The filter was soaked in scintillation cocktail (10ml) and counted for radioactivity using Packard 2660 Tricarb liquid scintillation counter. The inhibitory concentration ($IC_{50}$) of potential AII antagonist which gives 50% displacement of the total specifically bound $^3$H-angiotensin II was presented as a measure of the efficacy of such compounds as AII antagonists.

Using the methodology described above, representative compounds of this invention were evaluated and were found to exhibit an activity of at least $IC_{50}$<50 μM, thereby demonstrating and confirming the utility of the compounds of the invention as effective A II antagonists.

The antihypertensive effects of the compounds described in the present invention may be evaluated using the methodology described below:

Male Charles River Sprague-Dawley rats (300-375 gm) were anesthetized with methohexital (Brevital; 50 mg/kg i.p.) and the trachea was cannulated with PE 205 tubing. A stainless steel pithing rod (1.5 mm thick, 150 mm long) was inserted into the orbit of the right eye and down th spinal column. The rats were immediately placed on a Harvard Rodent Ventilator (rate - 60 strokes per minute, volumn -1.1 cc per 100 grams body weight). The right carotid artery was ligated, both left and right vagal nerves were cut, and the left carotid artery was cannulated with PE 50 tubing for drug administration, and body temperature was maintained at 37°C by a thermostatically controlled heating pad which received input from a rectal temperature probe. Atropine (1 mg/kg i.v.) was then administered, and 15 minutes later propranolol (1 mg/kg i.v.). Thirty minutes later antagonists of formula I were administered intravenously or orally. Angiotensin II was then typically given at 5, 10, 15, 30, 45

and 60 minute intervals and every half-hour thereafter for as long as the test compound showed activity. The change in the mean arterial blood pressure was recorded for each angiotensin II challenge and the precent inhibition of the angiotensin II response was calculated.

The compounds of the invention are useful in treating hypertension. They are also of value in the management of acute and chronic congestive heart failure. These compounds may also be expected to be useful in the treatment of secondary hyperaldosteronism, primary and secondary pulmonary hyperaldosteronism, primary and secondary pulmonary hypertension, renal failure such as diabetic nephropathy, glomerulonephritis, scleroderma, glomerular sclerosis, proteinuria of primary renal disease, end stage renal disease, renal transplant therapy, and the like, renal vascular hypertension, left ventricular dysfunction, diabetic retinopathy and in the management of vascular disorders such as migraine, Raynaud's disease, luminal hyperclasia, and to minimize the atherosclerotic process. The application of the compounds of this invention for these and similar disorders will be apparent to those skilled in the art.

The compounds of this invention are also useful to treat elevated intraocular pressure and to enhance retinal blood flow and can be administered to patients in need of such treatment with typical pharmaceutical formulations such as tablets, capsules, injectables and the like as well as topical ocular formulations in the form of solutions, ointments, inserts, gels, and the like. Pharmaceutical formulations prepared to treat intraocular pressure would typically contain about 0.1% to 15% by weight, preferably 0.5% to 2% by weight, of a compound of this invention.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg. per patient per day which can be administered in single or multiple doses. Perferably, the dosage range will be about 2.5 to 250 mg. per patient per day; more preferably about 5 to 150 mg. per patient per day.

The compounds of this invention can also be administered in combination with other antihypertensives and/or diuretics and/or angiotensin converting enzyme inhibitors and/or calcium channel blockers. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazide, metolazone, metoprolol tartate, methyclothiazide, methyldopa, methyldopate hydrochloride, minoxidil, pargyline hydrochloride, polythiazide, prazosin, propranolol, rauwolfia serpentina, rescinnamine, reserpine, sodium nitroprusside, spironolactone, timolol maleate, trichlormethiazide, trimethophan camsylate benzthiazide, quinethazone, ticrynafan, triamterene acetazolamide, aminophylline, cyclothiazide, ethacrynic acid, furosemide, merethoxylline procaine, sodium ethacrynate, captopril, delapril hydrochloride, enalapril, enalaprilat, fosinopril sodium, lisinopril, pentopril, quinapril hydrochloride, ramapril, teprotide, zofenopril calcium, diflusinal, diltiazem, felodipine, nicardipine, nifedipine, niludipine, nimodipine, nisoldipine, nitrendipine, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 2.5-250 milligrams per day range can be effectively combined at levels at the 0.5-250 milligrams per day range with the following compounds at the indicated per day dose range: hydrochlorothiazide (15-200 mg) chlorothiazide (125-2000 mg), ethacrynic acid (15-200 mg), amiloride (5-20 mg), furosemide (5-80 mg), propranolol (20-480 mg), timolol maleate (5-60 mg.), methyldopa (65-2000 mg), felodipine (5-60 mg), nifedipine (5-60 mg), and nitrendipine (5-60 mg). In addition, triple drug combinations of hydrochlorothiazide (15-200 mg) plus amiloride (5-20 mg) plus angiotensin II antagonist of this invention (3-200 mg) or hydrochlorothiazide (15-200 mg) plus timolol maleate (5-60) plus an angiotensin II antagonist of this invention (0.5-250 mg) or hydrochlorothiazide (15-200 mg) and nifedipine (5-60 mg) plus an angiotensin II antagonist of this invention (0.5-250 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the unit dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The following examples further illustrate the preparation of the compounds of Formula I and their incorporation into pharmaceutical compositions and, as such, are not to be considered or construed as limiting the invention recited in the appended claims.

Example 1

Preparation of 4′bromomethylbiphenyl-2-tert-butyl-sulfonamide

Step 1: Preparation of 2-bromobenzene(tert-butyl)-sulfonamide

To a stirred solution of 2-bromobenzene-sulfonyl chloride (Lancaster Synthesis) (2.21 g, 8.65 mmol) in chloroform (40 ml) under nitrogen at room temperature was added tert-butylamine (Aldrich) (2.30 ml, 21.9 mmol). The orange solution was stirred at room temperature for 12 h, then the mixture evaporated to dryness. Flash chromatography (silica gel, 10,15% ethyl acetate-hexane) afforded 2-bromobenzene(tert-butyl)sulfonamide as a white solid; $^1H$ NMR (300 MHz, $CDCl_3$) δ 8.18 (d, J = 8.5 Hz, 1H), 7.73 (d, J = 8.5 Hz, 1H), 7.50-7.35 (m, 2H), 5.11 (s, 1H), 1.20 (s, 9H).

Step 2: Preparation of p-tolyltrimethyltin

p-Tolylmagnesium bromide solution (Aldrich) (1.0M solution in diethyl ether) (53 ml, 0.0530 mol) was added dropwise to trimethyltin chloride (6.92 g, 0.0347 mol) in tetrahydrofuran (50 ml) under nitrogen at -10 °C. The suspension was allowed to warm slowly to room temperature over 3 h then saturated ammonium chloride solution (10 ml) was added followed by sufficient water to dissolve the precipitate.The solution was extracted three times with diethyl ether-hexane (1:1). The combined organic phase was washed with brine, dried (magnesium sulfate) and the solvents removed in vacuo . Vacuum distillation of the residue afforded a colorless liquid (39-40 °C, 0.1 mm Hg) which was further purified by flash chromatography (silica gel, hexane) to give p-tolyltrimethyltin as a colorless liquid; $^1H$ NMR (300 MHz, $CDCl_3$) δ 7.40 (d, J = 7.7 Hz, 2H), 7.19 (d, J = 7.7 Hz, 2H), 2.34 (s, 3H), 0.30 (s, 9H).

Step 3: Preparation of 4′-methylbiphenyl-2-tert-butylsulfonamide

2-Bromobenzene(tert-butyl)sulfonamide (1.00 g, 3.92 mmol), p-tolyl-trimethyltin (1.95 g, 6.67 mmol), bis(triphenylphosphine)palladium(II) chloride (Aldrich) (165 mg, 0.235 mmol) and dimethylformamide (25 ml) were heated with stirring under nitrogen at 90°C for 5 h. The black suspension was cooled to room temperature, then filtered through a pad of celite which was washed with tetrahydrofuran. The colorless filtrate was evaporated to dryness then chromatographed (silica gel, 8,10% ethyl acetate-hexane) to give 4′-methylbiphenyl-2-tert-butylsulfonamide as a white solid; $^1H$ NMR (300 MHz, $CDCl_3$) δ 8.16 (d, J = 7.9 Hz, 1H), 7.60-7.37 (m, 4H), 7.36-7.24 (m, 3H), 3.57 (s, 1H), 2.42 (s, 3H), 0.99 (s, 9H).

Step 4: Preparation of 4′-bromomethylbiphenyl-2-tert-butylsulfonamide

N-Bromosuccinimide (0.387 g, 2.17 mmol), a,a′-azoisobutyronitrile (catalytic), 4′-methylbiphenyl-2-tert-butylsulfonamide (0.55 g, 1.81 mmol) and carbon tetrachloride (50 ml) were heated with stirring at reflux for 3 h. After cooling to room temperature the mixture was filtered and the filtrate evaporated to dryness. Flash chromatography (silica gel, 10,20% ethyl acetate-hexane) afforded 4′-bromomethylbiphenyl-2-tert-butylsulfonamide (77% pure (the remainder of the material was 4′-dibromomethylbiphenyl-2-tert-butylsulfonamide)) as a white solid; $^1$H NMR (300 MHz, $CDCl_3$) δ 8.17 (dd, J = 7.5, 1.6 Hz, 1H), 7.68-7.45 (m, 6H), 7.31 (dd, J = 7.5, 1.6 Hz, 1H), 4.55 (s, 2H), 3.52 (s, 1H), 1.00 (s, 9H).

Example 2

Preparation of 5,7-dimethyl-2-ethyl-3-(2′-(amino-sulfonyl)(1,1′-biphen-4-yl)methyl)-imidazo(4,5-b)-pyridine

Step 1: Preparation of 5,7-dimethyl,2-ethyl-3-(2′-((tert-butylamino)sulfonyl)(1,1′-biphen-4-yl)methyl)-imidazo(4,5-b)pyridine

5,7-dimethyl-2-ethyl-imidazo(4,5-b)pyridine [prepared by the method described in European Patent Application 400,974] (0.132 g, 0.753 mmol) was added to a stirred suspension of sodium hydride (60% dispersion) (0.03 g, 0.75 mmol) in dimethylformamide (3 ml) at room temperature under nitrogen. The mixture was heated at 50 °C for 45 min then cooled to room temperature. A solution of 4′-(bromomethyl)-biphenyl-2-tert-butylsulfonamide (77% pure) (0.413 g, 0.832 mmol) in dimethylformamide (3 ml) was added dropwise and the solution heated at 50°C for 4 h. After cooling to room temperature the solvent was removed in vacuo. Flash chromatography (silica gel, 40, 60% ethyl acetate-hexane) afforded 5,7-dimethyl-2-ethyl-3-(2′-((tert-butylamino)sulfonyl)(1,1′-biphen-4-yl)methyl)-imidazo(4,5-b)pyridine as a white solid; $^1$H NMR (300 MHz, $CDCl_3$) δ 8.16 (d, 1H), 7.58-7.41 (m, 4H), 7.30-7.16 (m, 3H), 6.91 (s, 1H), 5.52 (s, 2H), 3.48 (s, 1H), 2.83 (q, 2H), 2.64 (s, 3H), 2.59 (s, 3H), 1.36 (t, 3H), 0.94 (s, 9H); FAB-MS: 477 (M+H), 246 ($C_{13}H_{12}NO_2S$).

Step 2: Preparation of 5,7-dimethyl,2-ethyl-3-(2′-) aminosulfonyl)(1,1′-biphen-4-yl)methyl)-imidazo(4,5-b)pyridine

Anisole (6 drops) was added to a stirred solution of 5,7-dimethyl-2-ethyl-3-(2′-((tert-butylamino)sulfonyl)(1,1′-biphen-4-yl)methyl)-imidazo(4,5-b)pyridine (0.264 g, 0.554 mmol) in trifluoroacetic acid (6 ml) under nitrogen at room temperature. The solution was stirred at room temperature for 8 h then the solvent removed in vacuo. Flash chromatography (silica gel, 60, 70% ethyl acetate-hexane, 0.5% ammonia) afforded 5,7-dimethyl-2-ethyl-3-(2′-(aminosulfonyl)(1,1′-biphen-4-yl)methyl)-imidazo(4,5-b)pyridine as a white solid; $^1$H NMR (400 MHz, $CDCl_3$) δ 8.11 (dd, 1H), 7.53 (dt, 1H), 7.46 (dt, 1H), 7.38 (m, 2H), 7.25 (m, 1H), 7.18 (d, 2H), 6.88 (s, 1H), 5.51 (s, 2H), 4.32 (s, 2H), 2.82 (q, 2H), 2.61 (s, 3H), 2.54 (s, 3H), 1.32 (t, 3H); FAB-MS: 421 (M+H), 246 ($C_{13}H_{12}NO_2S$).

Example 3

Preparation of 5,7-dimethyl-2-ethyl-3-(2′-((isopropyl-sulfonylamino)sulfonyl)(1,1′-biphen-4-yl)methyl)-imidazo(4,5-b)pyridine

To a stirred suspension of NaH (0.006 g, 0.15 mmol) in dry DMF (1.0 ml) under nitrogen at room temperature was added 5,7-dimethyl-2-ethyl-3-(2′-)aminosulfonyl (1,1′-biphen-4-yl)methyl)imidazo-(4,5-b)pyridine (0.05 g, 0.12 mmol). After stirring for 30 minutes at room temperature, isopropylsulfonylchloride (0.04 ml, 0.36 mmol) was added, and the resulting mixture was stirred at room temperature for 18h. The reaction mixture was poured into ice water (50 ml), acidified with 5% citric acid solution and extracted with chloroform (15 ml X 3). The combined organic phase was washed with water and brine, and then dried over $MgSO_4$. Removal of the solvent gave the crude product as a foam which was purified by flash-chromatography starting with 5% MeOH-$CH_2Cl_2$ and then with $CH_2Cl_2$-MeOH-$NH_4OH$ (40:10:1) to give the desired product as a cream colored solid. $^1$H NMR (300 MHz, $CD_3OD$): δ 8.25 (dd, J = 7.7, 1.7 Hz, 1H), 7.60-7.40 (m, 3H), 7.29-7.15 (m, 3H), 7.08 (s, 1H), 5.63 (s, 2H), 3.25 (m, 1H), 2.99 (q, J=7.5 Hz, 2H), 2.66 (s, 3H), 2.64 (s, 3H), 1.37 (t, J = 7.5 Hz, 3H), 1.26 (d, J= 6.83 Hz, 6H); FAB-MS: 527 (M+H).

Example 4

Preparation of 5,7-dimethyl-2-ethyl-3-(2′-((2-bromophenylsulfonylamino)sulfonyl)(1,1′-biphen-4-yl)-methyl)-imidazo(4,5-b)pyridine

The titled compound was prepared by using a similar procedure to that described in Example 2. $^1$H NMR (300 MHz, $CD_3OD$): δ 7.88 (dd, J = 7.7, 1.7 Hz, 1H), 7.72 (d, J=7.7 Hz, 1H), 7.55 (d, 1H), 7.46 (d, 1H), 7.30-6.85 (m, 12H), 6.82 (s, 1H), 5.35 (s, 2H), 2.68 (q, J=7.5 Hz, 2H), 2.47 (s, 3H), 2.42 (s, 3H), 1.15 (t, J = 7.5 Hz, 3H); FAB-MS: 677,679 (M+H).

Example 5

Preparation of 5,7-dimethyl-2-ethyl-3-(2′-((phenylsulfonylamino)sulfonyl)(1,1′-biphen-4-yl)methyl)-imidazo(4,5-b)pyridine

The titled compound was prepared by using a similar procedure to that described in Example 2. $^1$H NMR (300 MHz, $CDCl_3$): δ 7.88 (d, J = 7.7 Hz, 1H), 7.50 (d, J=7.7 Hz, 2H), 7.29-6.87 (m, 11H), 6.79 (s, 1H), 5.35 (s, 2H), 2.73 (q, J=7.5 Hz, 2H), 2.58 (s, 3H), 2.44 (s, 3H), 1.22 (t, J = 7.5 Hz, 3H); FAB-MS: 583 (M+Na).

Example 6

Preparation of 5,7-dimethyl-2-ethyl-3-(2′-((2-thienylsulfonylamino)sulfonyl)(1,1′-biphen-4-yl)methyl)-imidazo(4,5-b)pyridine

The titled compound was prepared by using a similar procedure to that described in Example 2. $^1$H NMR (300 MHz, $CDCl_3$): δ 7.88 (d, J = 7.7 Hz, 1H), 7.30-6.9 (m, 10H), 6.79 (s, 1H), 6.56 (m, 1H), 5.38 (s 2H), 2.75 (q, J=7.5 Hz, 2H), 2.58 (s, 3H), 2.44 (s, 3H), 1.22 (t, J = 7.5 Hz, 3H); FAB-MS: 567 (M+H).

Example 7

Preparation of 5,7-dimethyl-2-ethyl-3-(2′-((dibenzylphosphonylamino)sulfonyl)(1,1′-biphen-4-yl)methyl)-imidazo(4,5-b)pyridine

To a stirred solution of 5,7-dimethyl-2-ethyl-3-(2′-(aminosulfonyl)(1,1′-biphen-4-yl)methyl)-imidazo(4,5-b)pyridine (0.076 g, 0.18 mmol) in dry THF (1.5 ml) was added n-BuLi (1.6 M solution in hexane) (0.23 ml, 0.36 mmol) at 0°C. After stirring fo 15 minutes at that temperature, a solution of dibenzylphosphorylchloride (0.097 g, 0.36 mmol) in THF (0.5 ml) was added, and the resulting mixture was stirred at room temperature for 18h. The reaction mixture was concentrated under reduced pressure, and the residue was treated with 5% citric acid solution (5 ml) and extracted with methylene chloride (15 ml X 3). The combined organic phase was washed with water and brine, and then dried over $MgSO_4$. The crude product obtained after removal of the solvent was purified on silica-gel by flash-chromatography using chloroform-MeOH-$NH_4OH$ (90:10:0.5) to give the title product. $^1$H NMR (300 MHz, $CD_3OD$): δ 8.25 (dd, J = 7.7, 1.7 Hz, 1H), 7.40-6.90 (m, 18H), 6.78 (s, 1H), 5.28 (s, 2H), 4.6 (s, 4H), 2.78 (q, J=7.5 Hz, 2H), 2.54 (s, 3H), 2.44 (s, 3H), 1.24 (t, J = 7.5 Hz, 3H), 1.26 (d, J= 6.83 Hz, 6H); FAB-MS: 703 (M+Na).

Example 8

Preparation of 5,7-dimethyl-2-ethyl-3-(2′-((diethylphosphonylamino)sulfonyl)(1,1′-biphen-4-yl)methyl)-imidazo(4,5-b)pyridine

The titled compound was prepared by using a similar procedure to that described in Example 7. $^1$H NMR (300 MHz, $CDCl_3$): δ 8.15 (d, J = 7.7 Hz, 1H), 7.40-6.95 (m, 7H), 6.85 (s, 1H), 5.45 (s, 2H), 3.75 (m, 4H), 2.82 (q, J=7.5 Hz, 2H), 2.61 (s, 3H), 2.47 (s, 3H), 1.32 (t, J = 7Hz.5 , 3H), 1.03 (t, 6H); FAB-MS: 557 (M+H).

Example 9

Preparation of methyl 2-ethyl-7-methylimidazo(4,5-b)-pyridine-5-carboxylate

Step 1: Preparation of 2-ethyl-7-methylimidazo-(4,5-b)pyridine-4-oxide

A solution of 2-ethyl-7-methylimidazo(4,5-b)-pyridine (28 g, 174 mmol) and m-chloroperbenzoic acid (80-90%, 44.6 g) in $CHCl_3$ (300 mL) was heated at reflux for 0.5 h. The mixture was concentrated and purified ($SiO_2$, 100% $CH_2Cl_2$ gradient to 30% $CH_2Cl_2$/MeOH) to afford 2-ethyl-7-methylimidazo (4,5-b)pyridine-4-oxide as a solid. $^1H$ NMR (300 MHz, $CD_3OD$) $\delta$ 8.13 (d, 1H, J = 6 Hz), 7.13 (d, 1H, J = 6 Hz), 3.01 (q, 2H, J =7.5 Hz), 2.60 (s, 3H), 1.46 (t, 3H, J = 7.5 Hz).

Step 2: Preparation of 5-chloro-2-ethyl-7-methyl-imidazo(4,5-b)pyridine

A mixture of 2-ethyl-7-methylimidazo(4,5-b)-pyridine-4-oxide (29.75 g, 0.168 mol), $CHCl_3$ (25 mL) and $POCl_3$ (160 mL) was heated to 80°C for 1 h. After pouring over ice, the mixture was neutralized by careful addition of $NH_4OH$ and extracted with EtOAc. Concentration gave 5-chloro-2-ethyl-7-methylimidazo-(4,5-b)pyridine as a solid. $^1H$ NMR (250 MHz, $CDCl_3$) $\delta$ 7.07 (s, 1 H) 3.10 (q, 2H, J =7.5 Hz), 2.67 (s, 3H), 1.48 (t, 3H, J = 7.5 Hz).

Step 3: Preparation of 5-bromo-2-ethyl-7-methylimidazo(4,5-b)pyridine

A mixture of 5-chloro-2-ethyl-7-methyl-imidazo(4,5-b)pyridine (22.2 g, 0.113 mol) 30% HBr-HOAc was heated to 100°C for 19 h. The mixture was poured onto ice, neutralized with $NH_4OH$, extracted (5 x EtOAc), and the organic layers were concentrated to afford 5-bromo-2-ethyl-7-methylimidazo(4,5-b)-pyridine as a solid, after crystallization from EtOAc. $^1H$ NMR (300 MHz, $CDCl_3$) $\delta$ 7.22 (s, 1 H) 3.13 (q, 2H, J =7.5 Hz), 2.66 (s, 3H), 1.47 (t, 3H, J = 7.5 Hz).

Step 4: Preparation of 3-benzyl-5-bromo-2-ethyl-7-methylimidazo(4,5-b)pyridine

To a solution of 5-bromo-2-ethyl-7-methyl-imidazo(4,5-b)pyridine (10 g, 39 mmol) in DMF (70 mL) at room temperature was added NaH (1.3 g of an 80 % dispersion, 43 mmol). After 20 min benzyl bromide (5.15 mL, 43 mmol) was added and the reaction was stirred for 16 h. The mixture was poured onto 500 g of ice and the solid residue was filtered, washed with water and air dried to afford 3-benzyl-5-bromo-2-ethyl-7-methylimidazo(4,5-b)pyridine. $^1H$ NMR (300 MHz, $CDCl_3$) $\delta$ 7.33-7.22 (m, 3H), 7.19 (s, 1 H), 7.11-7.07 (m, 2H), 5.42 (s, 2H), 2.76 (q, 2H, J =7.5 Hz), 2.63 (s, 3H), 1.29 (t, 3H, J = 7.5 Hz).

Step 5: Preparation of 3-benzyl-5-cyano-2-ethyl-7-methylimidazo(4,5-b)pyridine

A mixture of 3-benzyl-5-bromo-2-ethyl-7-methylimidazo(4,5-b)pyridine (0.62 g, 1.8 mmol) and CuCN (0.806 g, 9.0 mmol) was heated in pyridine (4 mL) at reflux for 10 h under nitrogen. The reaction was cooled, then water (50 mL), KCN (1.17 g), and EtOAc (20 mL) were added and the mixture was heated to 50°C for 5 min. Cooling and extraction with EtOAc (2 x 50 mL) gave 3-benzyl-5-cyano-2-ethyl-7-methylimidazo(4,5-b)pyridine as a tan solid. $^1H$ NMR (400 MHz, $CDCl_3$) $\delta$ 7.40 (s, 1 H) 7.35-7.20 (m, 3H), 7.18-7.07 (m, 2H), 5.44 (s, 2H), 2.83 (q, 2H, J =7.5 Hz), 2.67 (s, 3H), 1.32 (t, 3H, J = 7.5 Hz).

Step 6: Preparation of methyl 3-benzyl-2-ethyl-7-methylimidazo(4,5-b)pyridine-5-carboxylate

A solution of 3-benzyl-5-cyano-2-ethyl-7-methylimidazo(4,5-b)pyridine (0.44 g, 1.59 mmol) in $H_2SO_4$ (4 mL) and $H_2O$ (4 mL) was heated to 80°C for 8 h. The reaction was cooled, MeOH (150 mL) was added, then conc. $NH_4OH$ was added until the mixture turned basic. The white solid $(NH_4)_2SO_4$ was filtered and washed with MeOH. The water and MeOH were removed in vacuo and and the residue was taken up in MeOH and then filtered to remove any remaining $(NH_4)_2SO_4$. After concentrating, and removal of water from the residue by evaporation from toluene, anhydrous 3% HCl-MeOH (50 mL) was added and the mixture was stirred overnight at rt. Filtration, concentration, and extraction from 5 % aqueous $Na_2CO_3$ with $CH_2Cl_2$ gave methyl 3-benzyl-2-ethyl-7-methylimidazo-(4,5-b)-pyridine-5-carboxylate as a solid. $^1H$ NMR (300 MHz, $CDCl_3$) $\delta$ 7.93 (s, 1 H) 7.38-7.29 (m, 3H), 7.12-7.03 (m, 2H), 5.53 (s, 2H), 3.96 (s, 3H), 2.78 (q, 2H, J =7.5 Hz), 2.70 (s, 3H), 1.29 (t, 3H, J = 7.5 Hz)

Step 7: Preparation of methyl 2-ethyl-7-methylimidazo (4,5-b)pyridine-5-carboxylate.

A mixture of crude methyl 3-benzyl-2-ethyl-7-methylimidazo(4,5-b)pyridine-5-carboxylate (0.75 g) in MeOH (30mL) and conc. aqueous HCl (1 mL) and 100 mg of moist Pearlman's catalyst were shaken under 1 atm. $H_2$ for 24 h. Filtration, concentration, and extraction from dilute $NH_4OH$ with EtOAc followed by drying ($Na_2SO_4$), concentration, and purification ($SiO_2$, 5% MeOH/EtOAc) gave methyl 2-ethyl-7-methylimidazo(4,5-b)pyridine-5-carboxylate as a solid. $^1H$ NMR (400 MHz, $CDCl_3$) δ 7.90 (s, 1 H) 4.00 (s, 3H), 3.10 (q, 2H, J =7.5 Hz), 2.71 (s, 3H), 1.38 (t, 3H, J = 7.5 Hz.).

Example 10

Preparation of 4′-bromomethylbiphenyl-2-(O-tert-butyl)-N-hydroxysulfonamide

Step 1: Preparation of 2-bromobenzene(O-tert-butyl)-N-hydroxysulfonamide

To a stirred solution of 2-bromobenzenesulfonyl chloride (Lancaster Synthesis) (1.0 g, 4.0 mmol) in chloroform (10 ml) under nitrogen at 0°C was added O-tert-butylhydroxylamine hydrochloride (Fluka) (0.6g, 4.77 mmol) in three portions. The solution was stirred at room temperature for 18 h and then diluted with methylene chloride (20 ml). The organic phase was washed successively with 5% citric acid, water and then dried over $MgSO_4$. Removal of the solvent in vacuo gave the crude product as white solid, which was then purified by flash chromatography (silica gel, 10% ethyl acetate-hexane) to afford 2-bromobenzene(O-tert-butyl)N-hydroxysulfonamide (1.12 g, 89%) as a white solid;
$^1H$ NMR (300 MHz, $CDCl_3$) δ 8.15 (dd, J = 7.5, 2.1 Hz, 1H), 7.75 (d, J = 7.6, 1.8 Hz, 1H), 7.55-7.35 (m, 3H), 5.11 (s, 1H), 1.21 (s, 9H). FAB-MS: 309 (M+H).

Step 2: Preparation of 4′-methylbiphenyl-2-(O-tert-butyl)-N-hydroxy sulfonamide

A solution of 2-bromobenzene(O-tert-butyl)-N-hydroxysulfonamide (0.31 g, 1.0 mmol), p-tolyltrimethyltin (0.3 g, 1.18 mmol) and bis(triphenylphosphine)palladium(II) chloride (Aldrich) (0.036 g) in dry dimethylformamide (6 ml) was stirred under nitrogen at 90°C for 6 h. The black suspension was cooled to room temperature, then filtered through a pad of celite which was washed with tetrahydrofuran. The colorless filtrate was evaporated to dryness then purified by flash chromatography (silica gel, 8% ethyl acetate-hexane) to give the titled compound as a semi-solid mass. $^1H$ NMR (300 MHz, $CDCl_3$) δ 8.15 (d, J = 7.8, 1.6 Hz, 1H), 7.67-7.50 (m, 2H), 7.36-7.24 (m, 5H), 5.78 (s, 1H), 2.42 (s, 3H), 1.08 (s, 9H). FAB-MS: 320 (M+H).

Step 3: Preparation of 4′-bromomethylbiphenyl-2-(O-tert-butyl)-N-hydroxysulfonamide

A mixture of N-Bromosuccinimide (0.14 g, 0.78 mmol), a,a′-azoisobutyronitrile (10 mg) and 4′-methylbiphenyl-2-(O-tert-butyl)-N-hydroxy sulfonamide (0.25 g, 0.78 mmol) in carbon tetrachloride (10 ml) was refluxed for 7 h. After cooling to room temperature the mixture was filtered and the filtrate evaporated to dryness. Flash chromatography (silica gel, 10% ethyl acetate-hexane) afforded 4′-methylbiphenyl-2-(O-tert-butyl)-N-hydroxy sulfonamide as a white solid. $^1H$ NMR (300 MHz, $CDCl_3$) δ 8.15 (d, J = 7.8 Hz, 1H), 7.70-7.30 (m, 7H), 5.72 (s, 1H), 4.55 (s, 2H), 1.08 (s, 9H). FAB-MS: 398, 400 (M+H).

Example 11

Preparation of 5,7-dimethyl-2-ethyl-3-(2′-((N-hydroxy-amino)sulfonyl)(1,1′-biphen-4-yl)methyl)-imidazo-(4,5-b)pyridine

Step 1: Preparation of 5,7-dimethyl-2-ethyl-3-(2′-((O-tert-butyl-N-hydroxyamino)sulfonyl)(1,1′biphen-4-yl)methyl)-imidazo(4,5-b)pyridine

5,7-Dimethyl-2-ethyl-imidazo(4,5-b)pyridine (0.079 g, 0.45 mmol) was added to a stirred suspension of sodium hydride (60% dispersion) (12 mg) in dimethylformamide (1 ml) at room temperature under nitrogen. The mixture was heated at 50°C for 45 min then cooled to room temperature. A solution of 4′-bromomethylbiphenyl-2-(O-tert-butyl)-N-hydroxysulfonamide (0.18 g, 0.45 mmol) in dimethylformamide (1 ml) was added dropwise and the solution heated at 50°C for 3 h. After cooling to room temperature the solvent was removed in vacuo. Flash chromatography (silica gel, 40, 60% ethyl acetate-hexane) afforded 5,7-dimethyl-2-ethyl-3-(2′-

((O-tert-butyl-N-hydroxyamino)sulfonyl)(1,1′-biphen-4-yl)methyl)imidazo-(4,5-b)pyridine as a glass-like solid. $^1$H NMR (300 MHz, $CDCl_3$): δ 8.13 (d, 1H), 7.63-7.48 (m, 2H), 7.42-7.25 (m, 5H), 6.90 (s, 1H), 5.72 (broad s, 1H), 5.51 (s, 2H), 2.83 (q, 2H), 2.64 (s, 3H), 2.59 (s, 3H), 1.36 (t, 3H), 1.02 (s, 9H); FAB-MS: 493 (M+H)($C_{27}H_{32}NO_3S$).

Step 2: Preparation of 5,7-Dimethyl-2-ethyl-3-(2′-((N-hydroxyamino)sulfonyl)(1,1′-biphen-4-yl)-methyl)-imidazo(4,5-b)pyridine

Anisole (2 drops) was added to a stirred solution of 5,7-dimethyl-2-ethyl-3-(2′-((O-tert-butyl-N-hydroxyamino)sulfonyl)(1,1′-biphen-4-yl)methyl)-imidazo(4,5-b)pyridine (0.05 g) in trifluoroacetic acid (1.5 ml) under nitrogen at room temperature. The solution was stirred at room temperature for 18 h, and then the solvent was removed in vacuo. The residue was tritureted with dry ether and the resulting solid was collected by filteration. The solid was finally crystallized from methanol (by dissolving in minimum amount of hot methanol) to give the crystalline product. $^1$H NMR (300 MHz, $CD_3OD$): δ 8.13 (d, 1H), 7.63-7.48 (m, 2H), 7.42-7.25 (m, 5H), 6.90 (s, 1H), 5.51 (s, 2H), 2.83 (q, 2H), 2.64 (s, 3H), 2.59 (s, 3H), 1.36 (t, 3H); FAB-MS: 437 (M+H).

Example 12

Preparation of 3-[2′-(3H-1,2,3-5-oxathiadiazole,2-oxide-4-yl)1,1′-biphenyl-4-yl]methyl-5,7-dimethyl-2-ethyl-3H-imidazo(4,5-b)pyridine

Step 1: 3-(2′-cyano-1,1′-biphenyl-4-yl)methyl-5,7-dimethyl-2-ethyl-3H-imidazo(4,5-b)pyridine

To a suspension of 60 % NaH (400 mg) in DMF (10 mL) was added the solution of 5,7-dimethyl-2-ethyl-3H-imidazo(4,5-b)pyridine (1.75 g, 10 mmol) in DMF (10 mL) at 0°C. After 5 min, 4-bromomethyl-2′-cyano-1,1′-biphenyl (2.72 g, 10 mmol; Eur. Pat. Appl 324,377, 1989) in DMF (10 mL) was added at 0oC and the mixture was stirred at rt for 15 hrs. Extractive workup (2 x EtOAc) from water followed by purification of the concentrated organic phases ($SiO_2$,) gave 3-(2′-cyano-1,1′-biphenyl-4-yl)methyl-5,7-dimethyl-2-ethyl-3H-imidazo(4,5-b)pyridine as a solid.

Step 2: 3-(2′-(N-Hydroxymethanimidamide)-1,1′-biphenyl-4-yl)methyl-5,7-dimethyl-2-ethyl-3H-imidazo(4,5-b)pyridine

To a mixture of the 3-(2′-cyano-1,1′-biphenyl-4-yl)methyl-5,7-dimethyl-2-ethyl-3H-imidazo-(4,5-b)pyridine (366 mg, 1 mmol) and $NH_2OH{\cdot}HCl$ (695 mg, 10 mmol) in EtOH (3 mL) was added 25 % NaOMe solution in MeOH (2.2 mL, 10 mmol). The solution was refluxed for 48 h. After cooling to rt $CHCl_3$ (50 mL) was added to the solution. The solution was washed with water (3X) and brine (1X), dried over anhydrous $MgSO_4$. Concentration afforded the title compound (388 mg) as a white powder.
$^1$H NMR ($CDCl_3$, 400 MHz): δ 7.52 (d, 1H, J=7.4 Hz), 7.45~7.25 (m, 5H), 7.13 (d, 2H, J=7.8 Hz), 6.88 (s, 1H), 5.46 (s, 2H), 4.37 (s, 2H), 2.78 (q, 2H, J=7.4 Hz), 2.61 (s, 3H), 2.57 (s, 3H), 1.28 (t, 3H, J=7,7 Hz).

Step 3: 3-[2′-(3H-1,2,3,5-oxathiadiazole-2-oxide-4-yl)-1,1′-biphenyl-4-yl]methyl-5,7-dimethyl,2-ethyl-3H-imidazo(4,5-b)pyridine

To a solution of the 3-(2′-(N-Hydroxy-methanimidamide)-1,1′-biphenyl-4-yl-)methyl-5,7-dimethyl-2-ethyl-3H-imidazo(4,5-b)pyridine (61.4 mg, 0.15 mmol) in anhydrous pyridine (1 mL) was added the 2N $SOCl_2$ solution (92 mL, 0.13 mmol) in $CH_2Cl_2$ dropwise at 0°C. The solution was stirred at 0°C for 1 h and the reaction was quenched with water. The product was extracted with EtOAc (3X). The organic layer was washed with water (3X) and brine (1X), and dried over anhydrous $MgSO_4$. After concentration the product was purified by flash chromatography (H:E = 1:1, 100% EtOAc) to afford the title compound (31.3 mg) as a solid.
$^1$H NMR ($CDCl_3$): δ 7.78 (d, 1H, J=7.78 Hz), 7.3 (t, 1H, J=7.1 Hz), 7.44 (t, 1H, J=7.5 Hz), 7.36 (d, 1H, J=7.4 Hz), 7.20 (d, 2H, J=8.0 Hz), 7.11 (d, 2H, J=8.0 Hz), 6.84 (s, 1H), 5.40 (ABq, 2H), 2.70 (q, 2H, J=7.5 Hz), 2.53 (s, 3H), 2.52 (s, 3H), 1.27 (t, 3H, J=7.6 Hz).

Example 13

Preparation of 5,7-dimethyl-2-ethyl-3-[2′-(2-(2-bromophenyl)-2,5-dihydro-1,2,3,5-thiatriazole-1-oxide-4-yl)-[1,1′]-biphenyl-4-yl]-methyl-3H-imidazo [4,5-b]pyridine

Step 1: Preparation of 2-Bromo-N-(2-cyanoethyl)-benzamide

2-Bromobenzoyl chloride (4.389 g, 20 mmol) was dissolved in THF (20 mL) and dripped in 20 equal portions alternating with 20 equal portions of 1 N-NaOH (20 mL) into a solution of 2-amino-propionitrile fumarate (2.563 g, 20 mL) at )°C. The solution was stirred at rt for 18 h. After the addition of water the product was extracted with EtOAc (3X). The combined organic layer was washed brine and dried over anhydrous $MgSO_4$. Concentration afforded the carboxamide (4.5 g) as a white powder. $^1H$ NMR ($CDCl_3$, 200 MHz): δ 7.62~7.20 (m, 4H), 6.74 (broad m, 1H), 3.66 (q, 2H, J=7.7 Hz), 2.72 (t, 2H, J=7Hz)

Step 2: Preparation of N-(2-cyanoethyl)-4′-methyl [1,1′]biphenyl-2-carboxamide

To a mixture of the above amide (233 mg, 0.92 mmol), trimethyl-p-toluyltin (230 mg, 0.92 mmol) and tetrakis(triphenylphosphine)palladium(0) (53 mg, 0.046 mmol) was added anhydrous toluene (5 mL). The mixture was stirred at 110 °C for 18 h. The solution was diluted with EtOAc and washed with brine. After drying over anhydrous $MgSO_4$ followed by concentration, the product was purified by flash chromatography (H:E=5:1, 1:1) to give the title compound (167 mg) as a solid.
$^1H$ NMR ($CDCl_3$, 400 MHz): δ 7.68 (d, 1H, J=7 Hz), 7.55/7.20 (m, 7H), 5.65 (broad m, 1H), 3.43 (q, 2H, J=7.7 Hz), 2.39 (t 2H, J=7Hz).

Step 3: Preparation of 2-Phenyl-5-(2′-cyanoethyl)-4-(4′-methyl-[1,1′]biphenyl-2-yl)-2,5-dihydro-1,2,3,5-thiatriazole-1-oxide

The mixture of the above amide (400 mg, 1.52 mmol) and PC15 (347 mg, 1.67 mmol) was heated with a heat gun under aspirator vacuum over 10 min. After gas evolution ceased the resultant oil was cooled to rt. The oil was dissolved in anhyrous 1,4-dioxane (3 mL) and phenylhydrazine (745 mL, 7.58 mmol) was added at rt dropwise. The solution was stirred at rt over 18 h. The solution was diluted with EtOAc and washed with brine. After drying over anhydrous $MgSO_4$ followed by concentration, the product was purified by flash chromatography (H:E=10:1, 5:1, 1:1) to give the amidrazone (180 mg) as a foamy glass. The above amidrazone (161 mg, 0.455 mmol) was dissolved in $CH_2Cl_2$ (3 mL). To the solution were added pyridine (79 mg, 1 mmol) and 2 N-$SOCl_2$ in $CH_2Cl_2$ (250 μL, 0.5 mmol) at 0°C. The solution was stirred at 0°C for 30 min and at rt for 1.5 h. The solution was diluted with EtOAc and washed with brine. After drying over anhydrous $MgSO_4$ followed by concentration, the product was purified by flash chromatography (H:E=5:1, 1:1) to give the title compound (180 mg) as a foamy glass.
$^1H$ NMR ($CDCl_3$ 400 MHz): 7.70/7.14 (m, 13H), 3.50 (m, 1H), 3.33 (m, 1H), 2.36 (s, 3H), 2.10/1.86 (m, 2H)

Step 4: Preparation of 2-(2-Bromophenyl)-5-(2′-cyanoethyl)-4-[4′-(bromomethyl)[1,1′]biphenyl-2-yl]-2,5-dihydro-1,2,3,5-thiatriazole-1-oxide

To a solution of the above thiatriazole (116 mg, 0.29 mmol) in $CCl_4$ (5 mL) were added NBS (56.8 mg, 0.32 mmol) and AIBN (10 mg). The solution was refluxed for 2h. The additional NBS (56.8 mg, 0.32 mmol) and AIBN (10 mg) were added and the solution was refluxed for 4h. The NBS (103 mg, 0.58 mmol) and AIBN (20 mg) were added again and the solution was refluxed for additional 4h. After cooling the solid was filtered off. The solution was diluted with EtOAc and washed with brine. After drying over anhydrous $MgSO_4$ followed by concentration, the product was purified by flash chromatography (H:E=10:1, 5:1, 1:1) to give the title compound (85.8 mg) as a foamy glass.
$^1H$ NMR ($CDCl_3$, 400 MHz): δ 7.70/7.35 (m, 12H), 4.48 (s, 2H), 3.56/3.27 (m, 2H)

Step 5: Prepration of 5,7-dimethyl-2-ethyl-3-[2′-(2-(2-bromophenyl)-2,5-dihydro-1,2,3,5-thiatriazole-1-oxide-4-yl)-[1,1′]-biphenyl-4-yl]methyl-3H-imidazo-[4,5-b]pyridine

To a suspension of 60 % NaH (7.3 mg, 0.184 mmol) in DMF (2 mL) was added a DMF (1 mL) solution of the 5,7-dimethyl-2-ethyl-3H-imidazo(4,5-b)pyridine (32.2 mg, 0.184 mmol) at 0°C. The solution was stirred at rt for 15 min. To the solution was added the above bromide (80 mg, 0.143 mmol) in DMF (1 mL) at 0°C. The

solution was stirred at rt for 18h. The solution was diluted with EtOAc and washed with brine. After drying over anhydrous $MgSO_4$ followed by concentration, the product was purified by flash chromatography (H:E=10:1, 5:1, 1:3, 100 % EtOAc) to give the title compound (28.4 mg) as a solid. $^1H$ NMR ($CDCl_3$ 400 MHz): δ 7.90 (d, 1H, J=7.7 Hz), 7.55~7.24 (m, 9H), 7.15 (d, 2H, J=8.1Hz), 6.87 (s, 1H), 5.45 (ABq, 2H), 2.75 (q 2H J=7.5 Hz), 2.59 (s, 3H), 2.56 (s, 3H), 1.28 (t, 3H, 7.6 Hz).

Example 14

Preparation of 5,7-dimethyl-2-ethyl-3-[2′-(2-phenyl-2,5-dihydro-1,2,3,5-thiatriazole-1-oxide-4-yl)-[1,1′]-biphenyl-4-yl] methyl-3H-imidazo[4,5-b]pyridine

Step 1: Preparation of 4-(2-Bromophenyl)-5-(2-cyanoethyl)-2-phenyl-2,5-dihydro-1,2,3,5-thiatriazole-1-oxide

The mixture of 2-bromo-N-(2′-cyanoethyl)-benzamide (938 mg, 3.71 mmol) and $PCl_5$ (849 mg, 4.08 mmol) was heated with a heat gun under aspirator vacuum over 10 min. After gas evolution ceased the resultant oil was cooled to rt. The oil was dissolved in anhyrous 1,4-dioxane (5 mL) and phenylhydrazine (1.82 mL, 18.54 mmol) was added at rt dropwise. The solution was stirred at rt over 18 h. The solution was diluted with EtOAc and washed with brine. After drying over anhydrous $MgSO_4$ followed by concentration, the product was purified by flash chromatography (H:E=10:1, 5:1, 1:1) to give the amidrazone (260 mg) as a foamy glass. The above amidrazone (250 mg, 0.729 mmol) was dissolved in $CH_2Cl_22$ (5 mL). To the solution were added pyridine (118 μL, 1.458 mmol) and 2 N-$SOCl_2$ in $CH_2Cl_2$ (365 mL, 0.729 mmol) at 0°C. The solution was stirred at 0°C for 30 min and at rt for 1.5 h. The solution was diluted with EtOAc and washed with brine. After drying over anhydrous $MgSO_4$ followed by concentration, the product was purified by flash chromatography (H:E=5:1, 1:1) to give the title compound (166 mg) as a foamy glass. $^1H$ NMR ($CDCl_3$, 400 MHz): δ 7.72 (d, 1H, J=7.8 Hz), 7.60-7.35 (m, 7H), 7.17 (t, 1H, 7.3 Hz), 4.11 (m, 1H), 3.72 (m, 1H), 2.79 (m, 1H), 2.66 (m, 1H).

Step 2: Preparation of 2-Ethyl-5,7-dimethyl-3-(4-(trimethylstannylphenyl)methyl)-3H-imidazo(4,5-b)pyridine

To a suspension of 60 % NaH (223 mg, 5.564 mmol) in DMF (5 mL) was added a DMF (5 mL) solution of the 5,7-dimethyl-2-ethyl-3H-imidazo(4,5-b)pyridine (885 mg, 5.058 mmol) at 0°C. The solution was stirred at rt for 10 min. To the solution was added a-bromo-4-trimethylstannyltoluene (1.688 g, 5.058 mmol) in DMF (5 mL) at 0°C. The solution was stirred at rt for 18h. The solution was diluted with EtOAc and washed with water. The aqueous layer was extracted with EtOAc (3X). The combined organic layer was washed with brine. After drying over anhydrous $MgSO_4$ followed by concentration, the product was purified by flash chromatography (H:E=10:1, 5:1, 1:3, 100 % EtOAc) to give the title compound (28.4 mg) as a solid.
$^1H$ NMR ($CDCl_3$, 400 MHz): δ 7.37 (d, 2H, J=7.8 Hz), 7.05 (d, 2H, J=7.3 Hz), 6.86 (s, 1H), 5.42 (s, 2H), 2.77 (q, 2H, J=7.5 Hz), 2.61 (s, 3H), 2.56 (s, 3H), 1.29 (t, 3H J=7.8 Hz).

Step 3: Preparation of 5,7-dimethyl-2-ethyl-3-[2′-(5-(cyanoethyl)2-phenyl-2,5-dihydro-1,2,3,5-thiatriazole-1-oxide-4-yl)-[1,1′]-biphenyl-4-yl] methyl-3H-imidazo[4,5-b]pyridine

A mixture of the above stannyl compound (100 mg, 0.234 mmol), the thiatriazole (80 mg, 0.206 mmol) and bis(triphenylphosphine)Pd(II) chloride (7.2 mg, 0.01) was dissolved in anhydrous DMF (3 mL). The solution was stirred at 120°C for 4h. After the reaction was quenched with water the product was extracted with EtOAc (3X). The combined organic layer was washed with brine. After drying over anhydrous $MgSO_4$ followed by concentration, the product was purified by flash chromatography (H:E=5:1, 1:1) to give the title compound (28.4 mg) as a solid.
$^1H$ NMR ($CDCl_3$, 400 MHz): δ 7.70~7.35 (m, 11H), 7.16 (d, 2H, J=8.0 Hz), 6.90 (s, 1H), 5.46 (ABq, 2H), 3.45 (m, 1H), 3.30 (m, 1H), 2.80 (q, 2H, J=7.5 Hz); 2.62 (s, 3H), 2.56 (s, 3H), 2.12 (m, 2H), 1.30 (t, 3H, J=7.8 Hz).

Step 4: Preparation of 5,7-dimethyl-2-ethyl-3-[2′-(2-phenyl-2,5-dihydro-1,2,3,5-thiatriazole-1-oxide-4-yl)-[1,1′]-biphenyl-4-yl]methyl-3H-imidazo[4,5-b]pyridine

To a solution of the above thiatriazole (28 mg, 0.049 mmol) in THF (1 mL) was added 5 drops of 5 N-NaOH and MeOH (1 mL) at rt. The solution was stirred at rt fot 48h. After concentration the residue was dissolved in water (2 mL). The pH of the solution was adjusted to ~4 by the addition of 1N-HCl. The product was extracted with EtOAc (3X). The combined organic layer was washed with brine. After drying over anhydrous $MgSO_4$ followed by concentration, the product was purified by flash chromatography (H:E=5:1, 1:1) to give the title compound (10.2 mg) as a solid.
$^1$H NMR ($CDCl_3$, 400 MHz): δ 7.92 (d, 1H, J=7.6 Hz), 7.51~7.10 (m, 12H), 6.91 (s, 1H), 5.48 (ABq, 2H), 2.81 (q, 2H, J=7.7), 2.62 (s, 3H), 2.58 (s, 3H), 1.30 (t, 3H, J=7.5 Hz).

Example 15

Preparation of 5,7-dimethyl-2--ethyl-3-[2′-(N-cyanoaminosulfonyl)[1,1′]-biphenyl-4-yl]methyl-3H-imidazo[4,5-b]pyridine

To a stirred solution of 5,7-dimethyl-2-ethyl-3-[2′-(aminosulfonyl)[1,1′]-biphenyl-4-yl] methyl-3H-imidazo[4,5-b]pyridine (122 mg, 0.29mmol) in THF (1.5 mL) at 0 °C was added sodium hexamethyldisilazine (0.35 mL of a 1 M solution in THF). After 10 min, a solution of BrCN (37 mg, 0.35 mmol) in THF (1 ml) was added dropwise via a double tipped needle. The reaction was warmed to r.t. and stirred for 3 h at which time 0.3 mL of HOAc was added. Extraction with $CH_2Cl_2$ (5 x 30 mL) from $H_2O$ (30 mL), evaporation of the organic extracts and purification (SiO2, 80:19:1 $CH_2Cl_2/CH_3OH/NH_4OH$) gave 5,7-dimethyl-2-ethyl-3-[2′-(N-cyanoaminosulfonyl) [1,1′]biphenyl4-yl]methyl-3H-imidazo[4,5-b]pyridine as a glass.
$^1$H NMR (200 MHz, $CD_3OD$) δ 8.08 (d, 1 H, J = 7.2 Hz), 7.58-7.44 (m, 2 H), 7.39 (d, 2 H, J = 8.2 Hz), 7.40 (d, 1 H, J = 7.2 Hz), 7.0 (d, 2 H J = 8.2 Hz), 7.00 (s, 1H), 5.59 (s, 2 H), 2.89 (q, 2 H, J=7.5 Hz), 2.60 (s, 3 H), 2.58 (s, 3 H), 1.29 (t, 3 H, J=7.5 Hz).

Example 16

Preparation of 2-butyl-3-[2′-[(N-butoxycarbonyl) aminosulfonyl)[1,1′]biphenyl-4-yl]methyl-6-[N-(n-pentanoyl)]-3H-imidazo-[4,5-b]pyridine

Step 1: Preparation of 2-amino-3,5-dinitropyridine

To a stirred solution of 2-amino-3-nitropyridine in conc. $H_2SO_4$ (50 mL) at 0 °C was added $HNO_3$ (3.10 mL, d=1.49) dropwise over 10 min. The mixture was warmed to r.t. for 20 min then heated to 50 °C for 90 min. The reaction mixture was cooled and poured into 400 g of ice. The resulting percipitate was filtered and air dried to give 10.3 g of 2-amino-3,5-dinitropyridine as a yellow solid.

Step 2: Preparation of 6-[N-(n-pentanoyl)]-2-butyl-imidazo[4,5-b]pyridine

A mixture of 2-amino-3,5-dinitropyridine (5.32 g, 28.9 mmol), THF (100 mL), methanol (250 mL) and Raney-nickel (3 mL of a 1:1 suspension in $H_2O$) was stirred under $H_2$ (1 atm.) was stirred for 5 h. The reaction mxture was quickly filtered into a receiving flask containing 5 mL of conc. HCl and the solvent was removed in vacuo at r. t. To this crude 2,3,5-triaminopyridine.HCl was added valeric acid (9.43 mL, 86.7 mmol) and polyphosphoric acid (100 mL) and this mixture was heated to 95 °C for 6 h. The warmed mixture was poured into stirred ice-$H_2O$ (200 mL) and this mixture was cooled and neutralized (to pH 4) by the addition of conc. $NH_4OH$. Extraction with EtOAc(3 x 75 mL), concentration, and purification (SiO2, 5 % MeOH/EtOAc) gave 6-[N-(n-pentanoyl)]-2-butylimidazo[4,5-b]pyridine as a solid.

Step 3: Preparation of 2-butyl-3-[2′-(N-tert-butylaminosulfonyl[1,1′]biphenyl-4-yl-]methyl-6-[N-(n-pentanoyl)]-3H-imidazo[4,5-b]pyridine

A solution of 6-[N-(n-pentanoyl)]-2-butyl-imidazo[4,5-b]pyridine, $K_2CO_3$ (956 mg, 6.92 mmol), and 4′-bromomethylbiphenyl-2-tert-butylsulfonamide 1.65 g, 3.46 mmol) in DMF (15 mL) was stirred for 12 h at r.t. The reaction mixture was poured into $H_2O$ (50 mL), extracted with EtOAc (2 x 50 mL), concentrated, and purified (SiO2, 4:1 EtOAc/hexanes) to give 740 mg of 2-butyl-3-[2′-(N-tert-butylaminosulfonyl)[1,1′]biphenyl-4-yl]methyl-6-[N-(n-pentanoyl)]-3H-imidazo[4,5-b]pyridine as a foam.

Step 4: Preparation of 2-butyl-3-[2′-(aminosulfonyl) [1,1′]biphenyl-4-yl]methyl-6-[N-(n-pentanoyl)-3H-imidazo[4,5-b]pyridine

A solution of 2-butyl-3-[2′-(N-tert-butylaminosulfonyl)[1,1′]biphenyl-4-yl]methyl-6-[N-(n-pentanoyl)]-3H-imidazo[4,5-b]pyridine (710 mg, 1.23 mmol) in trifluoroacetic acid (50 mL) was stirred at r. t. for 12 h. The mixture was concentrated, dissolved in EtOAc (40 mL) and washed with saturated aqueous $Na_2CO_3$. The organic ext-

racts were dried ($K_2CO_3$) and concentrated to give 2-butyl-3-[2′-(aminosulfonyl)[1,1′]biphenyl-4-yl] methyl-6-[N-(n-pentanoyl)]-3H-imidazo[4,5-b]pyridine as a solid.

Step 5: Preparation of 2-butyl-3-[2′-(N-butoxycarbonylaminosulfonyl)[1,1′]-biphenyl-4-yl] methyl-6-[N-(n-pentanoyl)]-3H-imidazo[4,5-b] pyridine

To a mixture of 2-butyl-3-[2′-(aminosulfonyl) [1,1′]-biphenyl-4-yl]methyl-6-[N-(n-pentanoyl)]-3H-imidazo[4,5-b]pyridine (65 mg, 0.124 mmol) and 4-dimethyaminopyridine (45 mg, 0.373 mmol) in pyridine (1.5 mL) was added n-butylchloroformate (0.078 mL, 0.62 mmol). After 48 h at r. t., MeOH (2 mL) was added and the mixture was concentrated, dissolved in EtOAc (40 mL), washed with $H_2O$ (15 mL), concentrated, and purified (SiO2, (97:3 $CH_2Cl_2$/MeOH) to give 2-butyl-3-[2′-(N-butoxycarbonyl aminosulfonyl)[1,1′]-biphenyl-4-yl]methyl-6-[N-(n-pentanoyl)]-3H-imidazo[4,5-b]pyridine as a solid. $^1$H NMR (200 MHz, $CD_3OD$) δ 8.48 (d, 1 H, J = 2 Hz), 8.30 (d, 1 H, J = 2 Hz) 8.15 (dd, 1 H, J = 1.4 and 7.8 Hz), 7.68-7.48 (m, 2 H), 7.35-7.20 (m, 3 H), 7.10 (d, 2 H J = 8.2 Hz), 5.53 (s, 2 H), 3.89 (t, 2H, J = 6.4 Hz) 2.87 (t, 2 H, J=7.4 Hz), 2.39 (t, 2 H, J=7.2 Hz), 1.85-1.56 (m, 4 H), 1.55-0.70 (m, 17 H).

Example 17

Preparation of 5,7-Dimethyl-2-ethyl-3-[2′-[(N-ethoxycarbonyl)aminosulfonyl)[1,1′]-biphenyl-4-yl] methyl--3H-imidazo-[4,5-b]pyridine

To a stirred suspension of NaH (0.014g, 0.36 mMol) in dry DMF (1.5ml) was added 5,7-dimethyl-2-ethyl-3-[2′-(aminosulfonyl[1,1′]-biphenyl-4-yl] methyl-3H-imidazo[4,5-b]pyridine (Example 2)(0.075g, 0.18mMol) at 25oC. After 15min. of stirring, ethylchloroformate (0.034ml, 0.36mMol) was added to the above mixture and stirring continued at that temperature for15h. The reaction mixture was then diluted with ice-water and acidified with 10% citric acid. The precipitate obtained was extracted into ethylacetate (30ml), and the organic phase was washed with water and then dried over anhydrous $MgSO_4$. Remoyal of the solvent in vacuo gave the crude product which was purified by flash-chromatography using CH2C12-MeOH-NH4OH (90:10:1) to give the desired compound (0.024g) as an amorphous solid.
$^1$H NMR (400 MHz, $CD_3OD$) δ 8.1 (d, 1 H, J = 7.2 Hz), 7.50-7.40 (m, 2 H), 7.35 (d, 2 H, J = 8.2 Hz), 7.15 (d,1 H, J = 7.2 Hz), 7.10 (d, 2 H J = 8.2 Hz), 7.00 (s, 1H), 5.58 (s, 2 H), 3.72 (t, 2H, J = 8 Hz) 2.92 (t, 2 H,J=7.4 Hz),2.60 (s, 3H), 2.57 (s, 3H), 1.31 (t, 3 H), 1.02 (t, 3 H). FAB-MS: m/e 493 (M+1)

EXAMPLES A3 TO A12

The compounds of the Formula (II) exemplified in Table A are prepared from the appropriate substituted starting materials utilizing the general procedures outlined in the noted schemes.

TABLE A

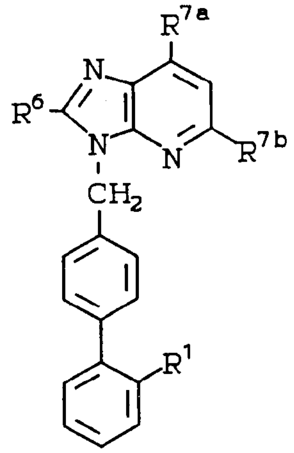

(II)

| Compound No. # | $R^1$ | $R^6$ | $R^{7a}$ | $R^{7b}$ | Scheme |
|---|---|---|---|---|---|
| A3 | $-SO_2NHSO_2Me$ | Et | Me | Me | 8 |
| A4 | $-SO_2NHSO_2iPr$ | Pr | $CO_2H$ | Me | 8 |
| A5 | 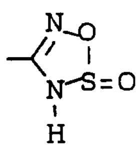 | Et | Me | Me | 16, 17 |
| A6 | 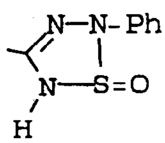 | Et | Me | Me | 18-20 |
| | | Et | $CO_2H$ | Me | 25 |

TABLE A (CON'T)

| Compound No. # | $R^1$ | $R^6$ | $R^{7a}$ | $R^{7b}$ | Scheme |
|---|---|---|---|---|---|
| A7 | 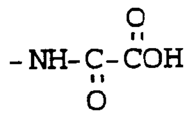 | Et | $CO_2H$ | Me | 25 |
| A8 | $-SO_2NHSO_2iPr$ | Et | Me | Me | 8 |
| A9 | 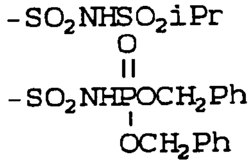 | Et | Me | Me | 13 |
| A10 | 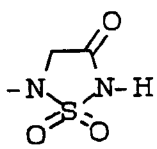 | Et | Me | Me | 21 |
| A11 | 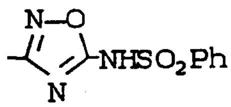 | Et | Me | Me | 11 |
| A12 | 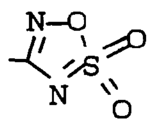 | Et | Me | Me | 15 |

EXAMPLES B1 TO B11

The compounds of the formula (III) exemplified in Table B are prepared from the appropriately substituted starting material utilizing the general procedures outlined in the noted schemes.

TABLE B

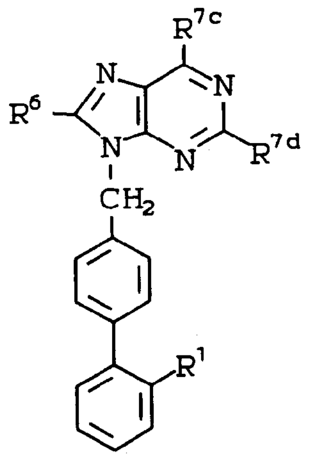

(III)

| Compound No. | $R^1$ | $R^6$ | $R^{7c}$ | $R^{7d}$ | Scheme |
|---|---|---|---|---|---|
| B1 | $-SO_2NHOH$ | Pr | Me | $-NHCH_3$ | 26 |
| B2 | $-SO_2NHOH$ | Pr | Me | 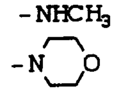 | 26 |
| B3 | $-SO_2NHSO_2iPr$ | Pr | Me | 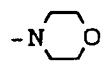 | 8 |
| B4 | $-SO_2NHSO_2Ph$ | Et | Me | $-NHCH_3$ | 8, 9 |
| B5 | 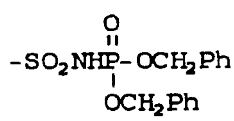 | Pr | Me | 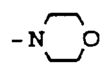 | 13 |
| B6 | 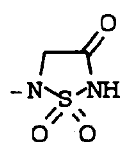 | Et | Me | 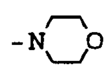 | 21 |

## TABLE B (CON'T)

| Compound No. | $R^1$ | $R^6$ | $R^{7c}$ | $R^{7d}$ | Scheme |
|---|---|---|---|---|---|
| B7 | 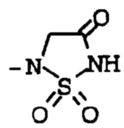 | Pr | Me | $-NHCH_3$ | 21 |
| B8 | 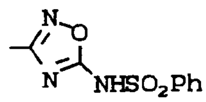 | Pr | Me | 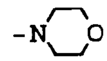 | 21 |
| B9 | 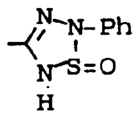 | Pr | Me | $-NHCH_3$ | 18-20 |
| B10 | 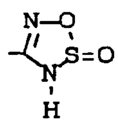 | Et | Me | 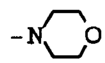 | 16, 17 |
| B11 | 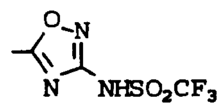 | Pr | Me | 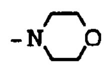 | 23 |

EXAMPLES C1 TO C11

The compounds of the formula (IV) exemplified in Table C are prepared from the appropriate substituted starting material utilizing the general procedures outlined in the noted schemes.

## TABLE C

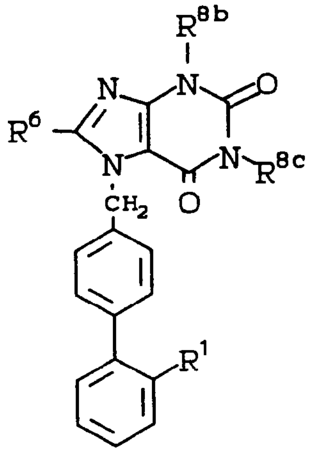

(IV)

| Compound No. | $R^1$ | $R^6$ | $R^{8b}$ | $R^{8c}$ | Scheme |
|---|---|---|---|---|---|
| C1 | $-SO_2NHOH$ | Pr | Me | Me | 26 |
| C2 | $-SO_2NHOH$ | nBu | Me | Me | 26 |
| C3 | $-SO_2NHSO_2Ph$ | Pr | Me | Me | 8, 9 |
| C4 | 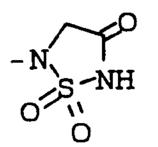 | Pr | Me | Me | 21 |
| C5 | 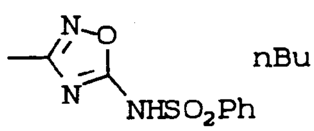 | nBu | Me | Me | 23 |

FORMULATIONS EXAMPLES

Typical Pharmaceutical Compositions Containing a Compound of the Invention

A: Dry Filled Capsules Containing 50 mg of Active Ingredient Per Capsule

| Ingredient | Amount per capsule (mg) |
|---|---|
| Compound A-1 | 50 |
| Lactose | 149 |
| Magnesium stearate | 1 |
| Capsule (size No. 1) | 200 |

The Compound A-1 (title compound of Example 11) can be reduced to a No. 60 powder and the lactose

and magnesium stearate can then be passed through a No. 60 blotting cloth onto the powder. The combined ingredients can then be mixed for about 10 minutes and filled into a No. 1 dry gelatin capsule.

B: Tablet

A typical tablet would contain the Compound A-1 (25 mg), pregelatinized starch USP (82 mg), microcrystaline cellulose (82 mg) and magnesium stearate (1 mg).

C: Combination Tablet

A typical combination tablet would contain, for example, a diuretic such as hydrochlorothiazide and consist of the Compound A-1 (7.5 mg), hydrochlorothiazide (50 mg) pregelatinized starch USP (82 mg), microcrystalline cellulose (82 mg) and magnesium stearate (1 mg).

D: Suppository

Typical suppository formulations for rectal administration can contain the Compound A-1 (1-25 mg), butylated hydroxyanisole (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or a diuretic and/or an angiotensin converting enzyme and/or a calcium channel blocker in pharmaceutically effective amounts as described, for example, in C above.

E: Injection

A typical injectable formulation would contain the Compound A-1 (5.42 mg), sodium phosphate dibasic anhydrous (11.4 mg) benzyl alcohol (0.01 ml) and water for injection (1.0 ml). Such an injectable formulation can also include a pharmaceutically effective amount of another active ingredient such as another antihypertensive and/or a diuretic and/or an angiotensin converting enzyme inhibitor and/or a calcium channel blocker.

## Claims

1. A compound of structural formula:

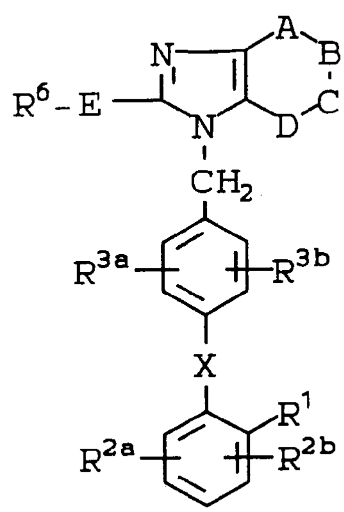

(I)

wherein:

$R^1$ is

(a) $-SO_2N(R^{24})-OR^{24}$,

(b) $-SO_2NHSO_2R^{23}$,

(c)

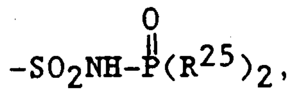

,

(d)

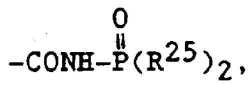

,

(e) $-SO_2NHCN$,

(f) $-SO_2NHCO_2R^{23}$,

(g)

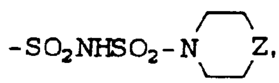

,

(h) $-NHSO_2NHSO_2R^{23}$,

(i)

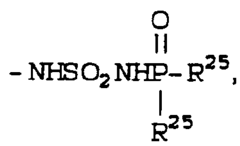

,

(j)

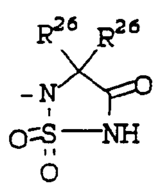

,

(k)

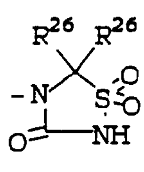

,

(l)

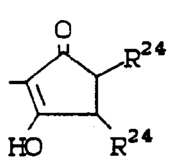

,

(m)

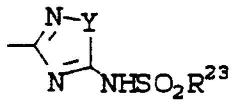
,

(n)

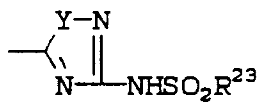
,

(o)

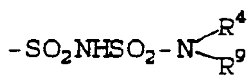
,

(p)

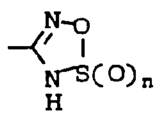
,

(q)

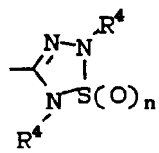
,

(r)

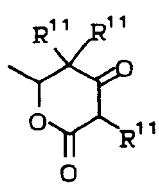
,

(s)

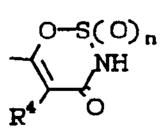
,

(t)

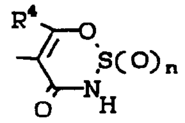 ,

(u)

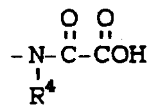 , or

(v) $-NHSO_2R^{23}$; wherein Y is O or S;

$R^{2a}$ and $R^{2b}$ are independently H, Cl, Br, I, F, $-NO_2$, $-NH_2$, $C_1$-$C_4$-alkylamino, di($C_1$-$C_4$ alkyl)amino, $-SO_2NHR^9$, $CF_3$, $C_1$-$C_6$alkyl, $C_1$-$C_6$-alkoxy, $CH_2$-$C_1$-$C_6$-alkoxy, $CH_2$-S-$C_1$-$C_6$-alkyl, $CH_2NR^9R^9$, $CH_2$-aryl, or aryl;

$R^{3a}$ is

(a) H,
(b) Cl, Br, I, or F,
(c) $C_1$-$C_6$-alkyl,
(d) $C_1$-$C_6$-alkoxy, or
(e) $C_1$-$C_6$-alkoxyalkyl;

$R^{3b}$ is

(a) H,
(b) Cl, Br, I, or F,
(c) $NO_2$,
(d) $C_1$-$C_6$-alkyl
(e) $C_1$-$C_6$-acyloxy,
(f) $C_1$-$C_6$-cycloalkyl
(g) $C_1$-$C_6$-alkoxy
(h) $-NHSO_2R^4$,
(i) hydroxy $C_1$-$C_4$-alkyl,
(j) aryl $C_1$-$C_4$-alkyl,
(k) $C_1$-$C_4$-alkylthio,
(l) $C_1$-$C_4$-alkyl sulfinyl,
(m) $C_1$-$C_4$-alkyl sulfonyl,
(n) $NH_2$,
(o) $C_1$-$C_4$-alkylamino,
(p) $C_1$-$C_4$-dialkylamino,
(q) fluoro $C_1$-$C_4$-alkyl,
(r) $-SO_2$-$NHR^9$,
(s) aryl, wherein aryl is phenyl or naphthyl or substituted phenyl or naphthyl with one or two substituents selected from the group consisting of Cl, Br, I, F, $C_1$-$C_4$-alkyl, $C_1$-$C_4$-alkoxy, $NO_2$, $CF_3$, $C_1$-$C_4$-alkylthio, OH, $NH_2$, NH($C_1$-$C_4$-alkyl), N($C_1$-$C_4$-alkyl)$_2$, $CO_2H$, and $CO_2$-$C_1$-$C_4$-alkyl; or
(t) furyl;

$R^4$ is H, $C_1$-$C_6$-alkyl, aryl, $-CH_2$-aryl, -CO-$C_1$-$C_6$alkyl, -CO-$C_3$-$C_6$-cycloalkyl, -CO-aryl, $-CO_2$-$C_1$-$C_6$-alkyl, $-CO_2$-$C_3$-$C_6$-cycloalkyl, $-CO_2$-aryl, -CONH-$C_1$-$C_6$-alkyl, $-SO_2$-aryl, or $-SO_2$-$C_1$-$C_6$-alkyl;

$R^{4a}$ is $C_1$-$C_6$-alkyl, aryl or $-CH_2$-aryl;

$R^5$ is

H, 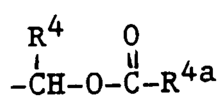;

E is a single bond, $-NR^{13}(CH_2)_s-$,$-S(O)_n-$, $(CH_2)_5-$ where n is 0 to 2 and s is 0 to 5, -CH(OH)-, -O-, -CO-;

$R^6$ is

(a) aryl or substituted aryl with 1 or 2 substituents selected from the group consisting of Cl, Br, I, F, -O-$C_1$-$C_4$-alkyl, $C_1$-$C_4$-alkyl, $-NO_2$, $-CF_3$, $-SO_2NR^9R^{10}$ -S-$C_1$-$C_4$-alkyl, -OH, $-NH_2$, $C_3$-$C_7$-cycloalkyl, $C_3$-$C_{10}$-alkenyl;

(b) $C_1$-$C_9$-alkyl, $C_2$-$C_6$-alkenyl or $C_2$-$C_6$-alkynyl or substituted $C_1$-$C_9$ alkyl, $C_2$-$C_6$ alkenyl, or $C_2$-$C_6$ alkynyl with a substituent selected from the group consisting of aryl as defined above, $C_3$-$C_7$-cycloalkyl, Cl, Br, I, F, -OH, $-NH_2$, -NH($C_1$-$C_4$-alkyl), $-CF_2CF_3$, -N($C_1$-$C_4$-alkyl)$_2$, $-NH-SO_2R^4$, $-COOR^4$, $-CF_3$, $-CF_2CH_3$, -$SO_2NHR^9$; or

(c) an unsubstituted, monosubstituted or disubstituted aromatic 5 or 6 membered cyclic ring which contains one or two members selected from the group consisting of N, O, S, and wherein the substituents are members selected from the group consisting of: -OH, -SH, $C_1$-$C_4$-alkyl, $C_1$-$C_4$-alkyloxy $-CF_3$, Cl, Br, I, F, or $NO_2$,

(d) perfluoro-$C_1$-$C_4$-alkyl, or

(e) $C_3$-$C_7$-cycloalkyl- or mono- or disubstituted $C_3$-$C_7$-cycloalkyl substituted with $C_1$-$C_4$-alkyl or $-CF_3$ substituents;

$R^9$ is H, $C_1$-$C_5$-alkyl, aryl or $-CH_2$-aryl;

$R^{10}$ is H, $C_1$-$C_4$-alkyl;

$R^{11}$ is H, $C_1$-$C_6$-alkyl, $C_2$-$C_4$-alkenyl, $C_1$-$C_4$-alkoxy-$C_1$-$C_4$-alkyl, or

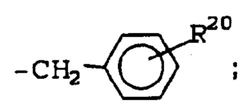 ;

$R^{12}$ is -CN, $-NO_2$ or $-CO_2R^4$;

$R^{13}$ is H, -CO($C_1$-$C_4$-alkyl), $C_1$-$C_6$-alkyl, allyl, $C_3$-$C_6$-cycloalkyl, phenyl or benzyl;

$R^{14}$ is H, $C_1$-$C_8$-alkyl, -$C_1$-$C_8$-perfluoroalkyl, $C_3$-$C_6$-cycloalkyl, phenyl or benzyl;

$R^{15}$ is H or $C_1$-$C_6$-alkyl;

$R^{16}$ is H, $C_1$-$C_6$-alkyl, $C_3$-$C_6$-cycloalkyl, phenyl or benzyl;

$R^{17}$ is $-NR^9R^{10}$, $-OR^{10}$, $-NHCONH_2$, $-NHCSNH_2$,

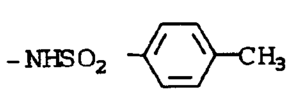 or 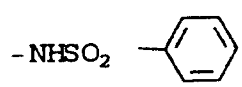 ;

$R^{18}$ and $R^{19}$ are independently $C_1$-$C_4$-alkyl or taken together are $-(CH_2)_q-$where q is 2 or 3;

$R^{20}$ is H, $-NO_2$, $-NH_2$, -OH or $-OCH_3$;

$R^{23}$ is

(a) aryl,

(b) heteroaryl, wherein heteroaryl is an unsubstituted, monosubstituted or disubstituted five- or six-membered aromatic ring which contains 1 to 3 heteroatoms selected from the group consisting of O, N or S and wherein the substituents are members selected from the group consisting of: -OH, -SH, -$C_1$-$C_4$-alkyl, -$C_1$-$C_4$-alkoxy, Cl, Br, F, I, $-NO_2$, $-CO_2H$, $-CO_2$-$C_1$-$C_4$-alkyl, $-NH_2$, -NH($C_1$-$C_4$-alkyl) and -N($C_1$-$C_4$-alkyl)$_2$;

(c) $C_3$-$C_4$-cycloalkyl,

(d) $C_1$-$C_4$-alkyl or substituted $C_1$-$C_4$ alkyl with a substituent that is a member selected from the group consisting of aryl, heteroaryl, -OH, -SH, -$C_1$-$C_4$-alkyl, -$C_3$-$C_7$-cycloalkyl, -O($C_1$-$C_4$-alkyl), $-SO_n$($C_1$-$C_4$-alkyl), $-CF_3$, Cl, Br, F, I, $-NO_2$, $-CO_2H$, $-CO_2$-$C_1$-$C_4$-alkyl, $-NH_2$, NH($C_1$-$C_4$-alkyl), $-NHCOR^{4a}$ -N($C_1$-$C_4$-alkyl)$_2$, -PO(OH)($C_1$-$C_4$-alkyl), -PO(OH)(aryl) or -PO(OH)(O-$C_1$-$C_4$-alkyl); where n is 0 to 2, or

(e) perfluoro-$C_1$-$C_4$-alkyl;

$R^{24}$ is

(a) H,

(b) aryl as defined above,

(c) $C_1$-$C_{10}$-alkyl optionally substituted with aryl, F, Cl, Br, -OH, $-NH_2$, -NH($C_1$-$C_4$-alkyl), -N($C_1$-$C_4$-alkyl)$_2$, $CF_3$, O-$C_1$-$C_4$-alkyl, $O(CH_2)_{n+1}$-O-$C_1$-$C_4$-alkyl, or

(d) $C_3$-$C_7$-cycloalkyl;

$R^{25}$ is

(a) aryl and substituted aryl as defined above,

(b) $C_1$-$C_6$-alkyl optionally substituted with aryl, F, Cl, Br, -OH, $-NH_2$, -NH($C_1$-$C_4$-alkyl), -N($C_1$-$C_4$-alkyl)$_2$, $CF_3$, $-COOR^4$, or CN,

(c) $-CH(R^4)-O-CO-R^{4a}$;or

(d) -OH, -O-$C_1$-$C_6$-alkyl wherein alkyl is as defined in (b);

$R^{26}$ is

(a) H,

(b) $C_1$-$C_6$-alkyl optionally substituted with aryl, F, Cl, Br, -OH, $-NH_2$, -NH($C_1$-$C_4$-alkyl), -N($C_1$-$C_4$-alkyl)$_2$, $CF_3$, $-COOR^4$, or CN,

(c) F, Cl, Br, or

(d) -O-$C_1$-$C_4$-alkyl wherein alkyl is defined as in (b);

X is

(a) a carbon-carbon single bond,

(b) -CO-,

(c) -O-,

(d) -S-,

(e)

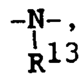

(f)

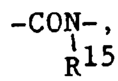

(g)

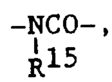

(h) $-OCH_2-$,

(i) $-CH_2O-$,

(j) $-SCH_2-$,

(k) $-CH_2S-$,

(l) $-NHC(R^9)(R^{10})$,

(m) $-NR^9SO_2-$,

(n) $-SO_2NR^9-$,

(o) $-C(R^9)(R^{10})NH-$,

(p) -CH=CH-,

(q) -CF=CF-,

(r) -CH=CF-,

(s) -CF=CH-,

(t) $-CH_2CH_2-$,

(u) $-CF_2CF_2-$,

(v) 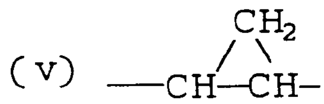 and 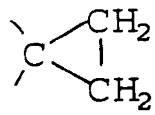 ,

(w)

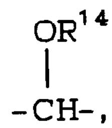

(x)

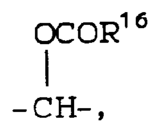

(y)

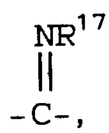 or

(z)

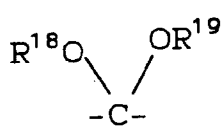 ;

Z is $CH_2$, O, $NR^{13}$ or S;

-A-B-C-D- represents the constituent atoms of a 6-member saturated or unsaturated heterocyclic ring with the imidazole to which they are attached containing 1 to 3 nitrogen atoms and includes the following:

1) 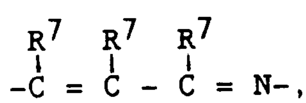

2) 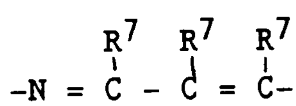

3) 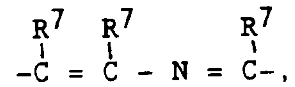

4) 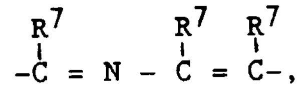

5) 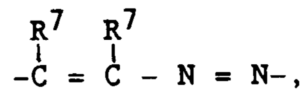

6) 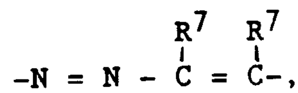

7) 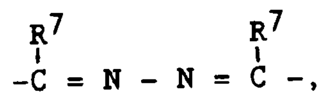

8) 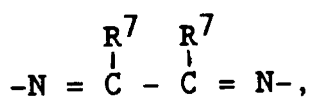

9) 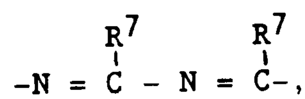

10) 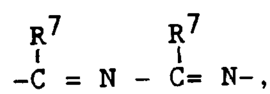

11) 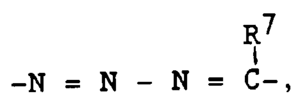

12) 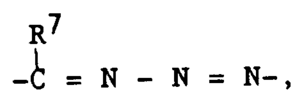

13) 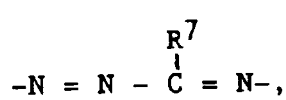

14) 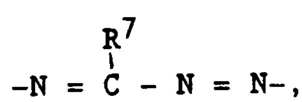

15) 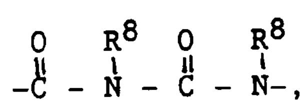

16) 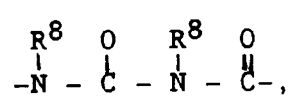

17) 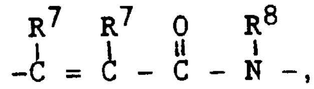

18) 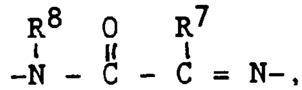

19) 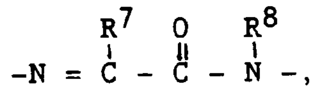

20) 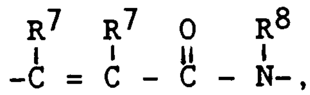

21) 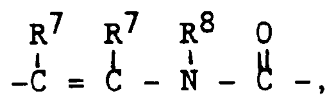

22) 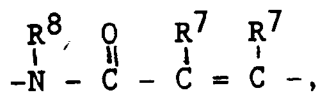

23) 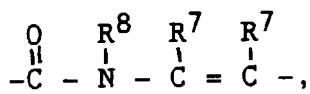

24) 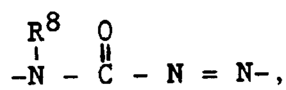

25) 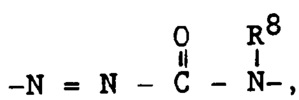

26) 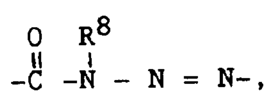

27) 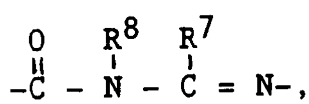

28) 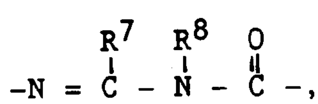

29) 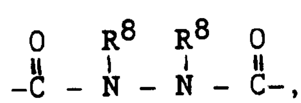

30) 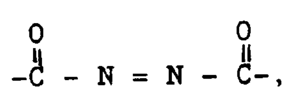

31) 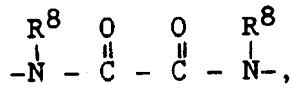

32) 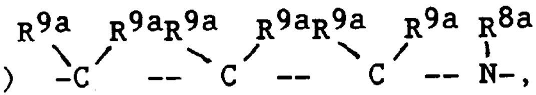

33) 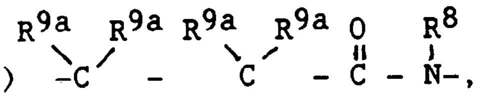

34) 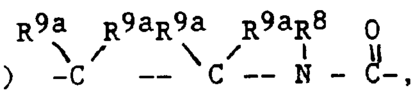

35) 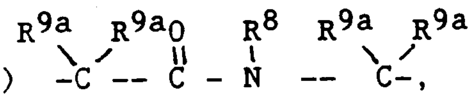

36) 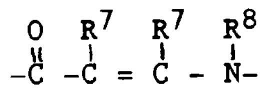

37) 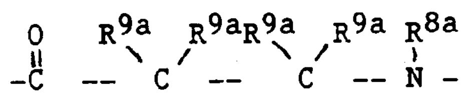

38) 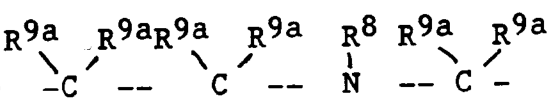

$R^7$ groups can be the same or different and represent:

a) hydrogen,

b) $C_1$-$C_6$ alkyl, or $C_2$-$C_6$ alkenyl, or $C_2$-$C_6$ alkynyl each of which is unsubstituted or substituted with:

i) -OH

ii) $C_1$-$C_4$-alkoxy,

iii) $-CO_2R^4$ or $-CO_2R^5$,

iv) $-OCOR^4$,

v)

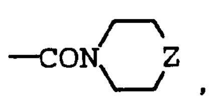

vi) $-CON(R^4)_2$

$R^4$ O

vii) -N - $CR^4$

viii) $-N(R^4)_2$,

ix) aryl,

x) heterocyclic as defined in (o) below,

xi) $-S(O)_nR^{23}$,

xii) tetrazo-5-yl,

xiii) $-CONHSO_2R^{23}$,

xiv) $-SO_2NHR^{23}$,

xv) $-SO_2NHCOR^{23}$,
xvi)

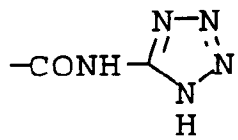,

xvii)

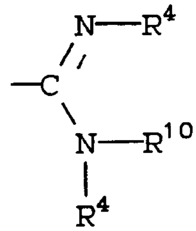,

xviii)

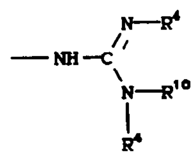,

xix) $-PO(OR^4)_2$,
xx) $-PO(OR^4)R^9$,
c) fluoro, chloro, bromo or iodo,
d) perfluoro-$C_1$-$C_4$-alkyl,
e) -OH,
f) $-NH_2$,
g)

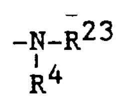,

h)

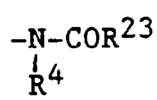,

i) $-OR^{23}$,
j) $-CO_2R^4$ or $-CO_2R^5$,
k) $-CON(R^4)_2$,
l) -NH-$C_3$-$C_7$-cycloalkyl,
m) $C_3$-$C_7$-cycloalkyl,
n) aryl,
o) heteroaryl which is a five- or six-membered saturated or unsaturated ring containing up to three heteroatoms selected from the group consisting of O, N or S wherein S may in the form of sulfoxide or sulfone and which may be substituted with one or two substituents which are members selected from the group consisting of Cl, Br, F, I, $C_1$-$C_4$alkyl, $C_1$-$C_4$-alkoxy, $C_1$-$C_4$-$S(O)_n$-, $CF_3$, $NO_2$, OH, $CO_2H$, $CO_2$-$C_1$-$C_4$-alkyl, $NH_2$, NH($C_1$-$C_4$-alkyl) or $N(R^4)_2$;
p) -CN,

q)

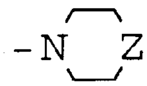 ,

r) $-SO_2N(R^4)_2$;
s) tetrazol-5-yl,
t) $-CONHSO_2R^{23}$,
u) $-PO(OR^4)_2$,
v) $-SO_2NHR^{23}$,
w) $-SO_2NHCOR^{23}$,
x) $-S(O)_n-R^{23}$,
y)

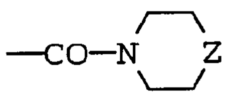 ,

z) $-PO(OR^4)R^9$ or $-PO(OR^5)R^9$,
aa) $-SO_2NHCON(R^{23})_2$,
bb) $-NHSO_2NHR^{23}$,
cc) $-NHSO_2NHCOR^{23}$,
dd) $NHCONHSO_2R^{23}$,
ee) $-N(R^4)CO_2R^{23}$,
ff)

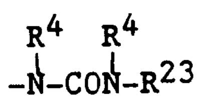 ,

gg) -CO-aryl,
hh)

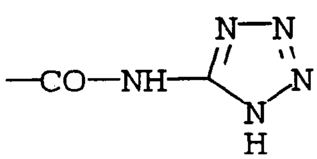 ,

ii) $-CO-C_1-C_4$-alkyl,
jj) $-SO_2NH-CN$,
kk) $-NHSO_2R^{23}$,
ll)

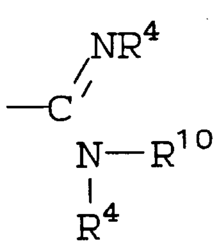 ,

mm)

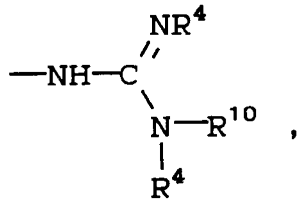

$R^8$ groups can be the same or different and represent:

a) hydrogen,

b) $C_1$-$C_6$-alkyl or $C_2$-$C_6$ alkenyl either unsubstituted or substituted with hydroxy, $C_1$-$C_4$-alkoxy, $-N(R^4)_2$, $-CO_2R^4$ or $C_3$-$C_5$-cycloalkyl,

c) $C_3$-$C_5$-cycloalkyl,

$R^{8a}$ is $R^8$ or $C_1$-$C_4$-acyl;

$R^{9a}$ groups can be the same or different and represent:

a) hydrogen,

b) $C_1$-$C_6$ alkyl either unsubstituted or. substituted with

i) hydroxy,

ii) $-CO_2R^4$,

iii) $-CONHR^4$, or

iv) $-CON(R^4)_2$; and, the pharmaceutically acceptable salts thereof

**2.** The compound of Claim 1, wherein:

$R^1$ is

(a) $-SO_2N(R^{24})-OR^{24}$,

(b) $-SO_2NHSO_2R^{23}$,

(c)

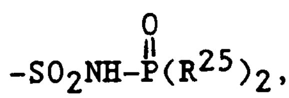

(d) $-SO_2NHCN$,

(e) $-SO_2NHCO_2R^{23}$,

(f)

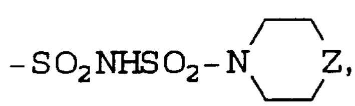

(g)

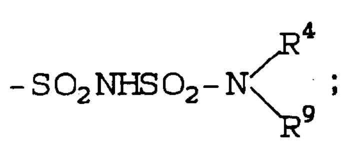

(h) $-NHSO_2NHSO_2R^{23}$,

(i)

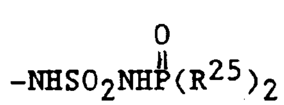

(j)

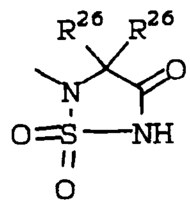

,

(k)

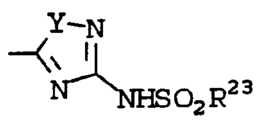

,

(l)

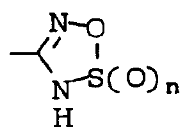

,

(m)

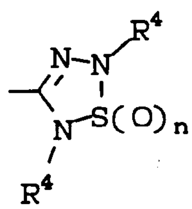

,

(n)

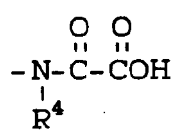

, or

(o) $-NHSO_2R^{23}$;

X is a single bond;

$R^{2a}$ and $R^{2b}$ are independently:

a) $C_1$-$C_6$-alkyl,
b) halogen,
c) hydrogen,
d) $CH_2$-$C_1$-$C_6$-alkoxy, or
e) $C_1$-$C_6$-alkoxy;

$R^{3a}$ and $R^{3b}$ are independently:

a) $C_1$-$C_6$-alkyl,
b) halogen,
c) $C_1$-$C_6$-alkoxy, or
d) hydrogen;

$R^4$ is H, or $C_1$-$C_4$-alkyl;

E is a single bond or -S-;

$R^6$ is $C_1$-$C_6$-alkyl, $C_3$-$C_7$-cycloalkyl, $C_2$-$C_6$-alkenyl or $C_2$-$C_6$- alkynyl each of which is either unsubstituted or substituted with $C_1$-$C_4$- alkylthio, $C_1$-$C_4$-alkoxy, $CF_3$, $CF_2CF_3$ or $-CF_2CH_3$;

A-B-C-D- represents:

1) 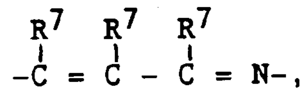

2) 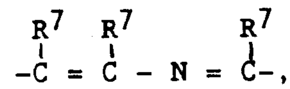

3) 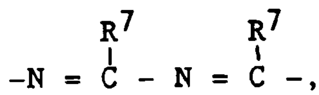

4) 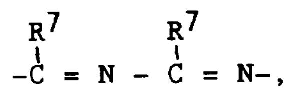

5) 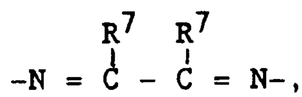

6) 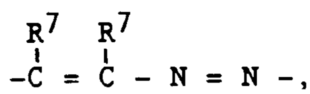

7) 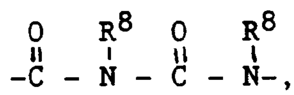

8) 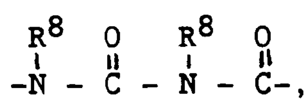

9) 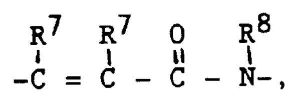

10) 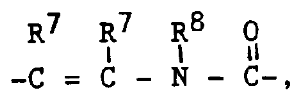

11) 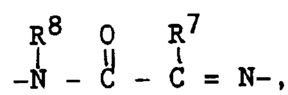

12) 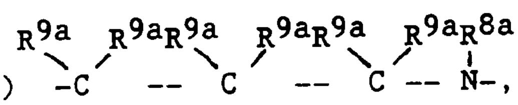

13) 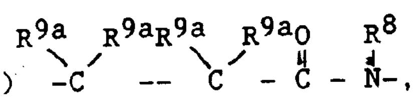 or

14) 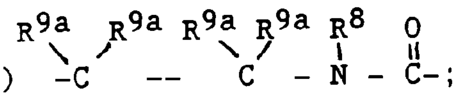

$R^7$ groups are the same or different and represent:

a) hydrogen,
b) $-C_1-C_4$-alkyl, either unsubstituted or substituted with:
   i) OH,
   ii) $-CO_2R^4$ or $-CO_2R^5$,
   iii) $-NH_2$,
   iv) ($C_1$-$C_4$ alkyl)amino,
   v) di($C_1$-$C_4$ alkyl)amino,
c) halo,
d) $-CF_3$,
e) -OH,
f) $-N(R^4)_2$,
g) $-C_1-C_4$-alkoxy,
h) $-CO_2R^4$ or $-CO_2R^5$,
i) $-CONH_2$,
j) $-C_3-C_7$-cycloalkyl,
k) aryl,
l) heteroaryl,
m) $-CF_3$,
n) tetrazol-5-yl,
o) $-CONHSO_2R^{23}$, or
p) $-NHSO_2R^{23}$;

$R^8$ groups are the same or different and represent,

a) hydrogen,
b) $C_1-C_4$-alkyl either unsubstituted or substituted with -OH or $-CO_2R^4$;

$R^{8a}$ represents

a) hydrogen,
b) $C_1$-$C_4$ alkyl, or
c) ($C_1-C_4$-alkyl)CO-; and

$R^{9a}$ groups are the same or different and represent:

a) hydrogen,
b) $C_1-C_4$-alkyl.

**3.** The compound of Claim 2 wherein:

$R^1$ is:

(a) $-SO_2N(R^{24})-OR^{24}$,
(b) $-SO_2NHSO_2R^{23}$,
(c)

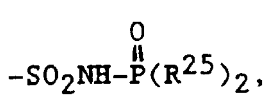

(d) $-SO_2NHCN$,
(e) $-SO_2NHCO_2R^{23}$,
(f)

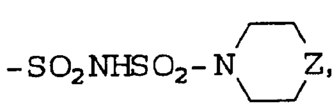

(g)

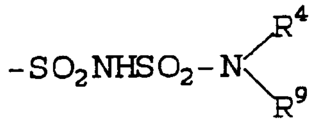 ;

(h) $-NHSO_2NHSO_2R^{23}$,

(i)

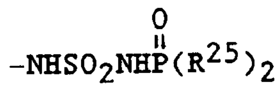 ,

(j)

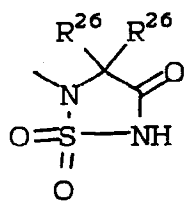 ,

(k)

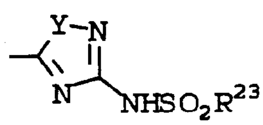 ,

(l)

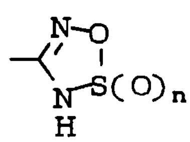 ,

(m)

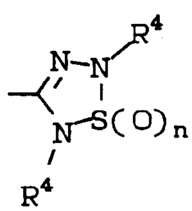 ,

(n)

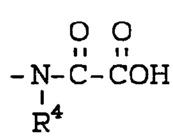 , or

(o) $-NHSO_2R^{23}$;

E is a single bond; and,

A-B-C-D represents:

1) 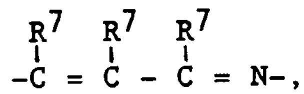

2) 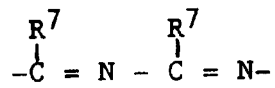 or

3) 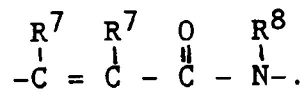

**4.** The compound of Claim 2 of the Formula (II)

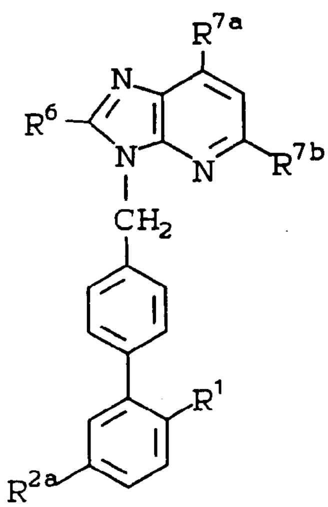

(II)

wherein:

$R^1$ is:

(a) $-SO_2N(R^{24})-OR^{24}$,

(b) $-SO_2NHSO_2R^{23}$,

(c)

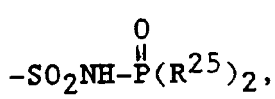

(d) $-SO_2NHCN$,

(e) $-SO_2NHCO_2R^{23}$,

(f)

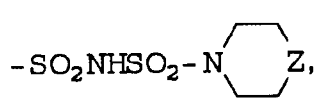

(g)

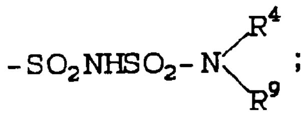

(h) -$NHSO_2NHSO_2R^{23}$,

(i)

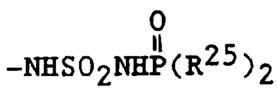

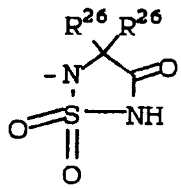
,

(k)

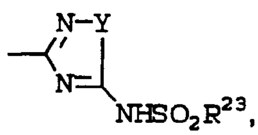
,

(l)

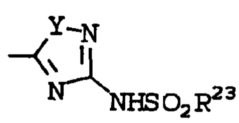
,

(m)

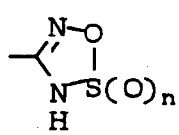
,

(n)

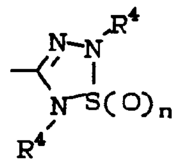 ,

(o) $-NHSO_2R^{23}$;

$R^6$ is $C_1$-$C_6$-alkyl or $C_2$-$C_6$ alkenyl or $C_3$-$C_7$-cycloalkyl; and

$R^{7a}$ and $R^{7b}$ independently are:

(a) hydrogen,

(b) $C_1$-$C_6$ alkyl or $C_2$-$C_6$ alkenyl,

(c) $-CO_2R^4$ in which $R^4$ is hydrogen, $C_1$-$C_6$-alkyl phenyl or benzyl; or

(d) $-CO(R^4)_2$.

**5.** The compound of Claim 4 wherein:

$R^1$ is:

(a) $-SO_2N(R^{24})-OR^{24}$,

(b) $-SO_2NHSO_2R^{23}$,

(c)

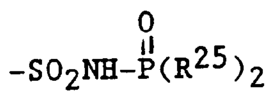 ,

(d) $-SO_2NHCN$,

(e) $-SO_2NHCO_2R^{23}$,

(f)

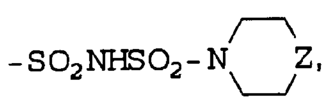 or

(g)

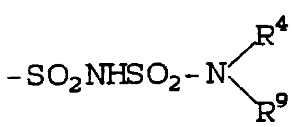 ;

$R^{2a}$ is:

(a) $C_1$-$C_6$-alkyl, or

(b) $CH_2$-$C_1$-$C_6$-alkoxy;

$R^6$ is $C_1$-$C_6$-alkyl; and

$R^{7a}$ and $R^{7b}$ independently are hydrogen, $C_1$-$C_6$ alkyl or $CO_2R^4$.

**6.** The compound of Claim 2 of the Formula (III)

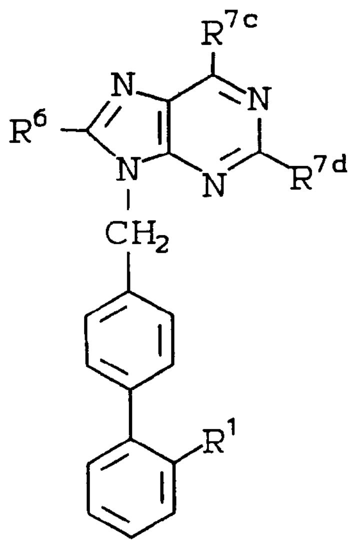

(III)

wherein:

$R^1$ is:

(a) $-SO_2N(R^{24})-OR^{24}$,

(b) $-SO_2NHSO_2R^{23}$,

(c)

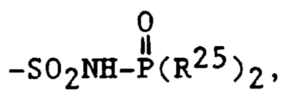

(d) $-SO_2NHCN$,

(e) $-SO_2NHCO_2R^{23}$,

(f)

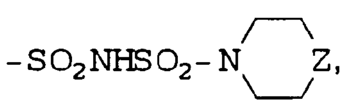

(g)

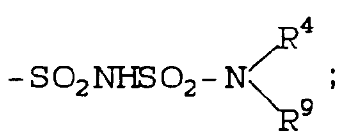

(h) $-NHSO_2NHSO_2R^{23}$, or

(i)

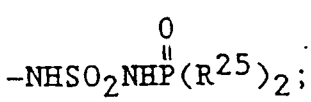

(j)

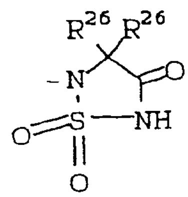

,

(k)

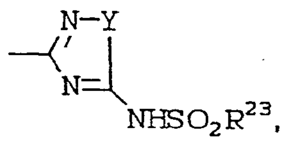

,

(l)

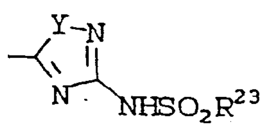

,

(m)

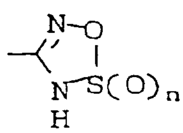

,

(n)

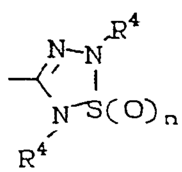

,

(o) $-NHSO_2R^{23}$;

$R^6$ is $C_1$-$C_6$-alkyl or $C_2$-$C_6$ alkenyl or $C_3$-$C_7$-cycloalkyl; and

$R^{7c}$ and $R^{7d}$ independently are:

(a) hydrogen,

(b) $C_1$-$C_6$ alkyl or $C_2$-$C_6$ alkenyl,

(c) $-CO_2R^4$ in which $R^4$ is hydrogen, $C_{1\text{-}6}$- phenyl or benzyl;

(d) $-CON(R^4)_2$,

(e) $-NH_2$,

(f)

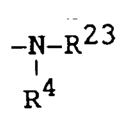

in which $R^{23}$ is $C_1$-$C_4$-alkyl, phenyl or perfluoro $C_1$-$C_4$-alkyl, or
(g)

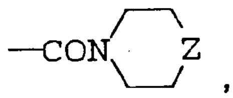

in which Z is $CH_2$, O or S.

**7.** The compound of Claim 2 of the Formula (IV)

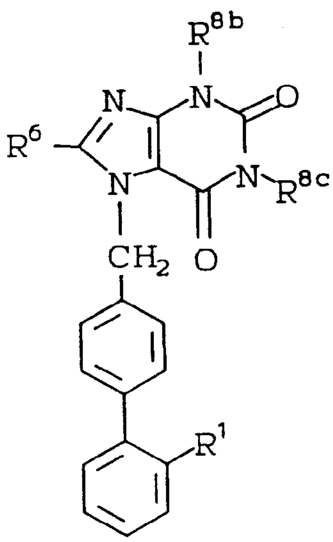
(IV)

wherein:

$R^1$ is:

(a) $-SO_2N(R^{24})-OR^{24}$,
(b) $-SO_2NHSO_2R^{23}$,
(c)

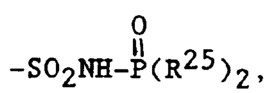

(d) $-SO_2NHCN$,
(e) $-SO_2NHCO_2R^{23}$,
(f)

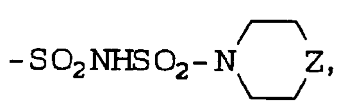

(g)

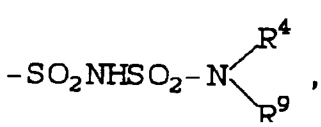

(h) $-NHSO_2NHSO_2R^{23}$,

(i)

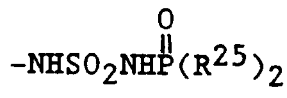,

(j)

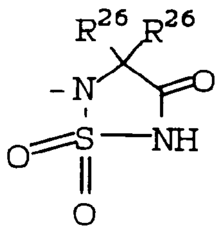,

(k)

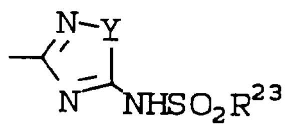, or

(l) $-NHSO_2R^{53}$;

$R^6$ is $C_1$-$C_6$-alkyl or $C_2$-$C_6$ alkenyl or $C_3$-$C_7$-cycloalkyl; and

$R^{8b}$ and $R^{8c}$ independently are:

(a) hydrogen, or

(b) $C_1$-$C_6$ alkyl or $C_2$-$C_6$ alkenyl

**8.** A pharmaceutical formulation for the treatment of hypertension and congestive heart failure comprising a pharmaceutically acceptable carrier and an effective antihypertensive amount of the compound of Claim 1.

**9.** The use of a compound of Claim 1 for the manufacture of a medicament for treating hypertension and congestive heart failure.

**10.** An ophthalmological formulation for the treatment of ocular hypertension comprising an ophthalmologically acceptable carrier and an effective ocular antihypertensive amount of a compound of Claim 1.